(19) Europäisches Patentamt
European Patent Office
Office européen des brevets

(11) Veröffentlichungsnummer: **0 292 806 B1**

(12) # EUROPÄISCHE PATENTSCHRIFT

(45) Veröffentlichungstag der Patentschrift: **02.11.94**

(51) Int. Cl.$^5$: **C07D 501/20**, A61K 31/545, A23K 1/17

(21) Anmeldenummer: **88107679.8**

(22) Anmeldetag: **13.05.88**

Die Akte enthält technische Angaben, die nach dem Eingang der Anmeldung eingereicht wurden und die nicht in dieser Patentschrift enthalten sind.

(54) **Substituierte Vinylcephalosporine, Verfahren zu ihrer Herstellung und ihre Verwendung als Arzneimittel.**

(30) Priorität: **26.05.87 DE 3717663**
**08.10.87 DE 3734005**

(43) Veröffentlichungstag der Anmeldung:
**30.11.88 Patentblatt 88/48**

(45) Bekanntmachung des Hinweises auf die Patenterteilung:
**02.11.94 Patentblatt 94/44**

(84) Benannte Vertragsstaaten:
**AT BE CH DE ES FR GB GR IT LI NL SE**

(56) Entgegenhaltungen:
**EP-A- 0 195 947**
**FR-A- 2 081 451**
**FR-A- 2 540 117**
**US-A- 4 255 423**
**US-A- 4 619 925**

(73) Patentinhaber: **BAYER AG**

**D-51368 Leverkusen (DE)**

(72) Erfinder: **Schmidt, Gunter, Dr.**
**Pahlkestrasse 63**
**D-5600 Wuppertal 1 (DE)**
Erfinder: **Metzger, Karl Georg, Dr.**
**Pahlkestrasse 75**
**D-5600 Wuppertal 1 (DE)**
Erfinder: **Zeiler, Hans-Joachim, Dr.**
**Am Rohm 86**
**D-5600 Wuppertal 1 (DE)**
Erfinder: **Endermann, Rainer, Dr.**
**In den Birken 152 a**
**D-5600 Wuppertal 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Es ist bekannt, daß verschiedene Vertreter der 7-α-Aminoacylcephalosporine mit unterschiedlichen Substituenten in der 3-Stellung des Moleküls, so z.B. Cephalexin [7-(D-α-Phenylglycyclamido)-3-methyl-3-cephem-4-carbonsäure, vgl. DE-OS 24 32 485], Cefaclor [7-(D-α-Phenylglycylamido)-3-chlor-3-cephem-4-carbonsäure, vgl. DE-OS 24 08 698 und 27 28 578] und Cefadroxil [7-(D-α-p-Hydroxyphenylglycylamido)-3-methyl-3-cephem-4-carbonsäure, vgl. DE-OS 27 18 741] gute antibiotische Wirksamkeit haben.

Weiterhin sind 3-Alkenyl-substituierte Cephalosporine als oral wirksame Verbindungen in DE-OS 34 02 642 und US-Patent 4 619 925 beschrieben.

Benzothiazolylglycylamido substituierte Vinylcephalosporine sind aus DE-OS 35.08.258 bekannt. Die vorliegende Erfindung betrifft eine Auswahl der in DE-OS 35.08.258 beschriebenen Stoffe.

Die Erfindung betrifft nun β-Lactam-Verbindungen der allgemeinen Formel (I)

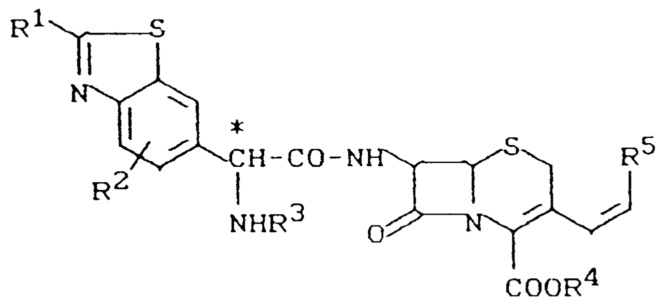

(I),

in welcher

$R^1$

- für Wasserstoff steht, oder
- für ein geradkettiges, verzweigtes oder cyclisches Alkyl mit bis zu 8 Kohlenstoffatomen steht, das durch Halogen, Hydroxy, Alkoxy mit bis zu 4 Kohlenstoffatomen, Carboxy, Phenyl, Sulfo oder durch eine Gruppe der Formel

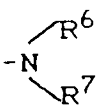

substituiert sein kann,

wobei

$R^6$,$R^7$

- gleich oder verschieden sind und
- Wasserstoff oder Alkyl mit bis zu 6 Kohlenstoffatomen, oder
- für Phenyl, Halogen, Alkoxy, Alkylthio, Alkylsulfonyl mit jeweils bis zu 6 Kohlenstoffatomen, Sulfo, Sulfamoyl, Mercapto, Hydroxy, Phenylthio, Phenyloxy, Guanidino, Amidino, oder
- für eine Gruppe der Formel

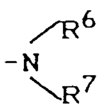

steht,

wobei

$R^6$, $R^7$ die oben angegebene Bedeutung haben,

$R^2$

- für Wasserstoff, Alkyl, Alkoxy, Alkylthio mit jeweils bis zu 6 Kohlenstoffatomen, Trifluormethyl, Trifluormethoxy, Hydroxy, Mercapto, Nitro, Cyano, oder Halogen steht,

$R^3$

- für Wasserstoff oder
- für eine in der $\beta$-Lactamchemie übliche Aminoschutzgruppe steht,

$R^4$

- für Wasserstoff,
- für eine in der $\beta$-Lactamchemie übliche Carboxyschutzgruppe oder
- für einen in vivo spaltbaren Ester steht,

und

$R^5$

- für Wasserstoff steht,
- für geradkettiges oder verzweigtes Alkyl mit bis zu 6 Kohlenstoffatomen steht, das substituiert sein kann durch Halogen, Alkoxy, Alkylthio mit jeweils bis zu 6 Kohlenstoffatomen, Hydroxy, Amino oder durch einen Rest der Formel

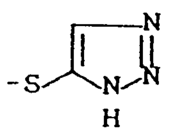 , 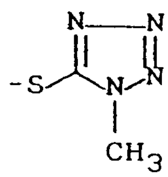 , 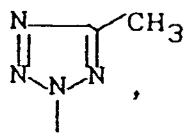 ,

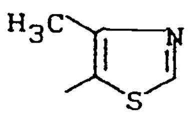 , 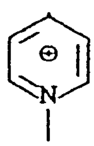 , 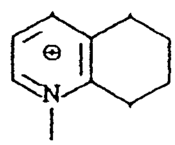

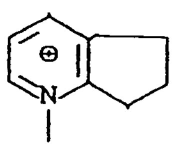 , 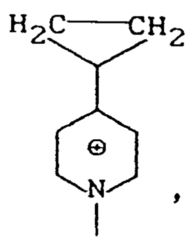 , 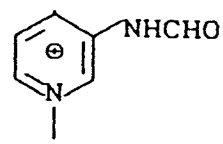

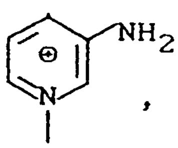 , 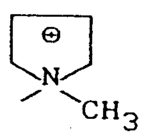 , 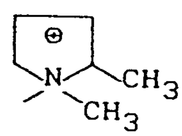 ,

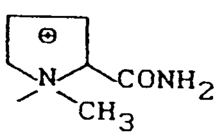 , 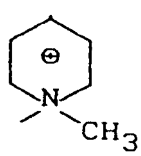 , 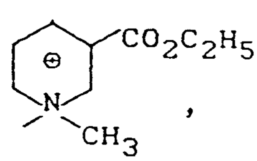 ,

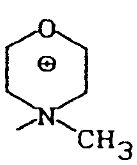 , 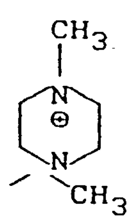 oder 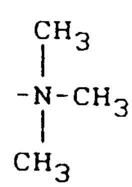

und deren Salze.

Aminoschutzgruppe im Rahmen der oben angegebenen Definition steht im allgemeinen für eine in der β-Lactam-Chemie üblichen Schutzgruppe aus der Reihe: Benzyl, tert-Butoxycarbonyl, Benzyloxycarbonyl, 2-Nitrobenzyloxycarbonyl, 4-Nitrobenzyloxycarbonyl, 2,2,2-Trichlorethoxycarbonyl, Fluorenyl-(9)-methoxycarbonyl, N-Diphenyl-methoxycarbonyl, Acetoacetyl, 2-Nitrobenzoyl, 2-Nitrophenylsulfenyl, Phthaloyl, Trityl, Vinyloxycarbonyl, Formyl, Benzoyl, Allyloxycarbonyl, 2,4-Dimethoxybenzyloxycarbonyl, 2-Methylthioethoxycarbonyl, 1,3-Dithian-2-ylmethoxycarbonyl (Dmoc), Trimethyl-, Triethyl-, Triphenylsilyl, tert-Butyl-dimethylsilyl, tert-Butyl-diphenylsilyl, 1-Methyl-2-benzoyl-vinyl, 1-Methyl-2-ethoxycarbonyl-vinyl, 1-Methyl-2-methoxycarbonyl-vinyl, 1-Methyl-2-(2,6-dimethoxybenzoyl)-vinyl, 4-Methoxybenzyloxycarbonyl (siehe E. Wünsch, Methoden der Organischen Chemie, Houben-Weyl, Bd. 15/I (1974)).

Carboxyschutzgruppe im Rahmen der oben angegebenen Definition steht für die in der β-Lactam-Chemie üblichen Carboxyschutzgruppen. Bevorzugt sind leicht abspaltbare Gruppen zu nennen wie zum Beispiel: Methyl, Ethyl, tert.Butyl, Decyl, 2-Chlorethyl, 2,2,2-Trichlorethyl, Cyanoethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl. (Siehe a) E. Wünsch, Methoden der Organischen Chemie, Houben-Weyl, Bd. 15/I (1974) und b) Th. W. Greene, Protective Groupsin Organic Synthesis, J. Wiley & Sons (1981)).

Steht $R^4$ für einen in vivo leicht abspaltbaren Esterrest, so sind hiermit pharmazeutisch verträgliche Esterreste gemeint, die in vivo leicht zu freien Carboxylgruppen ($R^4$ = H) hydrolysiert werden.

Solche Esterreste sind auf dem β-Lactam-Gebiet gut bekannt. In den meisten Fällen bessern sie die Absorptionseigenschaften der β-Lactam-Verbindungen. Außerdem sollte der Rest $R^4$ von solcher Art sein, daß er einer Verbindung der Formel (I) pharmazeutisch annehmbare Eigenschaften verleiht und bei Spaltung in vivo pharmazeutisch annehmbare Fragmente freisetzt.

Beispiele für solche Gruppen befinden sich in der DE-OS 2 228 255 und DE-OS 2 350 230. Bevorzugte in vivo abspaltbare Estergruppen sind die der folgenden Formeln:

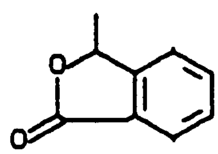 , 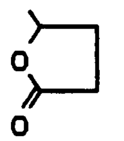 , 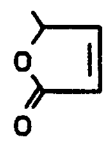 ,

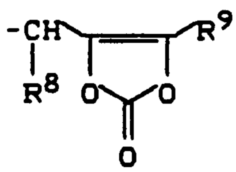 , 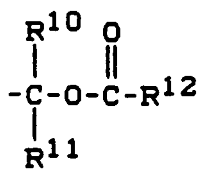 oder

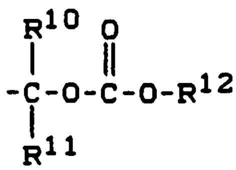

worin

$R^8$ und $R^9$ gleich oder verschieden sind und

- für Wasserstoff, Phenyl oder
- für $C_1$-$C_4$-Alkyl, bevorzugt für Methyl stehen,

$R^{10}$ und $R^{11}$ gleich oder verschieden sind und

- für Wasserstoff oder
- für $C_1$-$C_4$-Alkyl, bevorzugt Methyl stehen

und

$R^{12}$

- für $C_1$-$C_6$-Alkyl, bevorzugt für $C_1$-$C_4$-Alkyl steht.

Die erfindungsgemäßen Verbindungen der allgemeinen Formel (I) können als freie Säuren, als Ester, als innere Salze

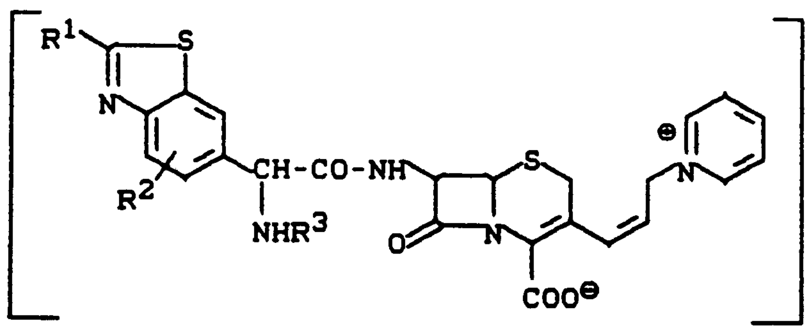

**Beispiel für inneres Salz**

oder als nicht-toxische, physiologisch verträgliche Salze mit einem Gegenkation

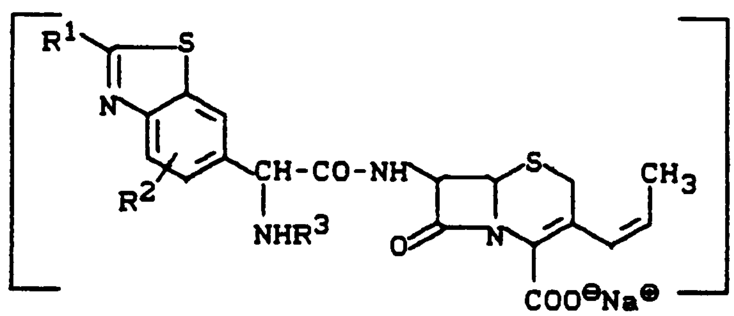

**Beispiel für Salz mit Gegenkation**

oder wenn $R^4$ ein positiv geladener Rest ist als nichttoxisch, physiologisch verträgliche Salze mit einem Gegenanion

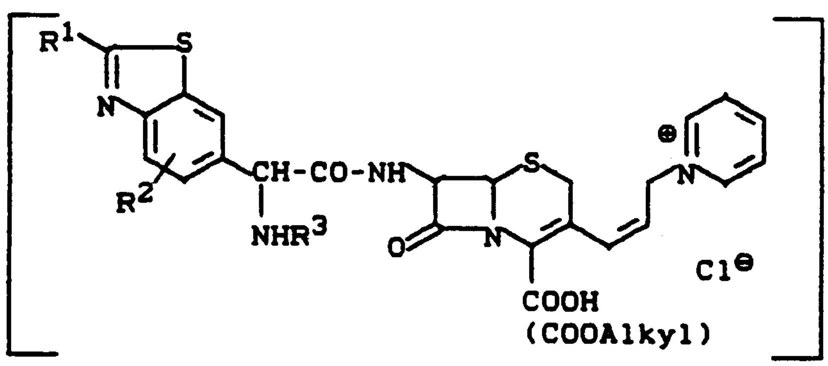

**Beispiel für Salz mit Gegenanion**

vorliegen.

Als Gegenkationen sind bevorzugt Alkali- oder Erdalkalikationen wie beispielsweise Natrium-, Kalium-, Magnesium- oder Calciumionen zu nennen, oder Aluminium-oder Ammoniumionen, sowie nicht-toxische substituierte Ammoniumionen aus Aminen wie Diniedrigalkylamine, Triniedrigalkylamine, Prokain, Dibenzylamin, N,N'-Dibenzylethylendiamin, N-Benzyl-β-phenyl-ethylamin, N-Methylmorpholin, 1-Ephenamin, Dihydroabietylamin, N,N'-Bisdihydroabietylethylendiamin,N-Niedrigalkylpiperidine oder andere Amine, die zur Bildung von Salzen von β-Lactamverbindungen verwendet werden können.

Als Gegenanionen sind bevorzugt anorganische oder organische Säurereste zu nennen wie beispielsweise Chlorid, Bromid, Iodid, Sulfat, Hydrogensulfat, Phosphat, Hydrogenphosphat Carbonat, Hydrogencarbonat, oder Sulfonate wie Methansulfonat, Ethansulfonat, Toluolsulfonat, Benzolsulfonat, Naphthalindisulfonat, oder Carboxylate wie Acetat, Formiat, Oxalat, Tartrat, Citrat, Maleat, Fumarat, Benzoat, Succinat, Lactat.

Die Verbindungen der allgemeinen Formel (I) existieren (bzgl. der Doppelbindung) in der Z-(cis)- sowie in der E-(trans)-Konfiguration. Bevorzugt sind die Verbindungen mit Z-(cis)-Konfiguration. Wegen der Anwesenheit des mit * bezeichneten asymmetrischen Kohlenstoffatoms (siehe Formel I) schließen die erfindungsgemäßen $\beta$-Lactamantibiotika der allgemeinen Formel (I) die D-, L- und D,L-Formen ein. Sowohl die Diastereomerengemische als auch die D-Form und L-Form der erfindungsgemäßen Verbindungen können zur Behandlung bakterieller Infektionskrankheiten eingesetzt werden. Besonders bevorzugt sind die D-Formen der erfindungsgemäßen Verbindungen.

Bevorzugt seinen Verbindungen der allgemeinen Formel (I) genannt,
in welchen

$R^1$

- für Wasserstoff oder
- für geradkettiges, verzweigtes oder cyclisches Alkyl mit bis zu 6 Kohlenstoffatomen steht, oder
- für Phenyl steht, oder
- für eine Gruppe der Formel $-NHR^6$ steht,

worin

$R^6$

- Wasserstoff, Phenyl, Benzyl, Acetyl, Benzoyl oder Alkyl mit bis zu 6 Kohlenstoffatomen bedeutet,

$R^2$

- für Wasserstoff, Methyl, Methoxy, Trifluormethyl, Trifluormethoxy, Nitro, Cyano, Fluor, Chlor, Brom oder Hydroxy steht,

$R^3$

- für Wasserstoff oder
- für eine Aminoschutzgruppe aus der Reihe: tert-Butoxycarbonyl, Benzyloxycarbonyl, 2,2,2-Trichlorethoxycarbonyl, Trityl, Vinyloxycarbonyl, Allyloxycarbonyl, 2,4-Dimethoxybenzyloxycarbonyl, 1-Methyl-2-benzoyl-vinyl, 1-Methyl-2-ethoxy-carbonyl-vinyl, 1-Methyl-2-methoxy-carbonyl-vinyl, 4-Methoxy-benzyloxycarbonyl steht,

$R^4$

- für Wasserstoff steht, oder
- für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxybenzyl, 2,4-Dimethoxybenzyl, 1-Phenoxyethyl, 2-Methyl-2-propenyl, 4-Nitrobenzyl, 2-Nitrobenzyl, Trimethylsilylethyl oder tert.Butyl-dimethylsilylethyl steht, oder
- für einen Rest der Formel

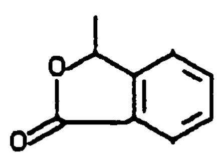 , 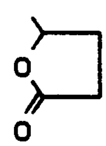 , 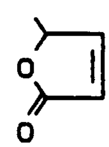 ,

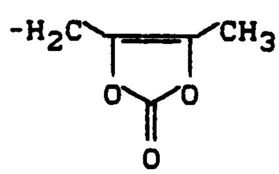 ,

$-CH_2-OCO-C(CH_3)_3$,
$-CH(CH_3)-OCOOC_2H_5$ oder
$-CH_2-OCOCH_3$ steht,

und

$R^5$

- für Wasserstoff steht, oder
- für geradkettiges oder verzweigtes Alkyl mit bis zu 4 Kohlenstoffatomen steht, das substituiert sein kann durch Fluor, Chlor, Brom, Iod, Alkoxy mit bis zu 3 Kohlenstoffatomen, Hydroxy, Amino oder durch einen Rest der Formel

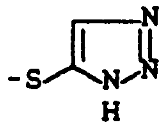, 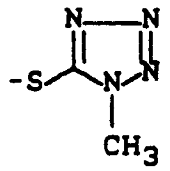, 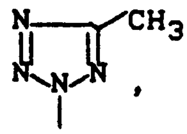,

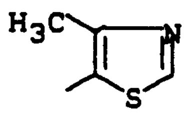, 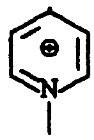, 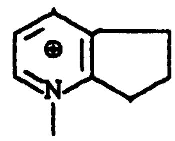,

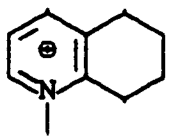, 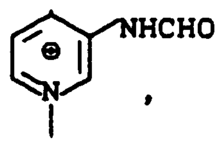, 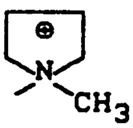,

oder 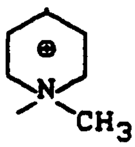

und deren Salze.

Besonders bevorzugt seien Verbindungen der allgemeinen Formel (I) genannt, in welcher

$R^1$
- für Wasserstoff oder
- für geradkettiges oder verzweigtes Alkyl mit bis zu 4 Kohlenstoffatomen steht, oder
- für eine Gruppe der Formel $-NHR^6$ steht,

  worin

$R^6$
- Wasserstoff, Methyl, Ethyl, Propyl oder Isopropyl bedeutet,

$R^2$
- für Wasserstoff oder Hydroxy steht,

$R^3$
- für Wasserstoff oder
- für Benzyloxycarbonyl, 1-Methyl-2-benzoylvinyl-, 4-Methoxybenzyloxycarbonyl, 2-Nitrophenylsulfenyl (NPS), Trityl, Allyloxycarbonyl, tert-Butyl-dimethylsilyl, 1-Methyl-2-methoxycarbonylvinyl (MMV) oder tert-Butoxycarbonyl(Boc) steht, oder

$R^4$
- für Wasserstoff steht, oder
- für Methyl, Ethyl, tert.Butyl, Diphenylmethyl, 2,2,2-Trichlorethyl, Allyl, Acetoxymethyl, 4-Nitrobenzyl, 2-Nitrobenzyl, 4-Methoxybenzyl, Benzyl oder Trimethylsilylethyl steht, oder
- für einen Rest der Formel

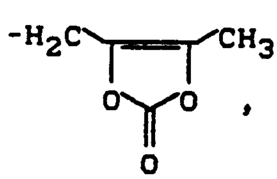,

$-CH(CH_3)-OCOOC_2H_5$

oder $-CH_2-OCO-C(CH_3)_3$ steht,

und

$R^5$

- für Wasserstoff, Methyl, Chlormethyl, Dihydroxyethyl, Iodmethyl, oder
- für einen Rest der Formel

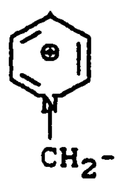 , 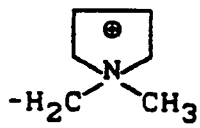 , 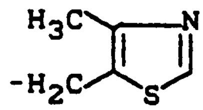 , 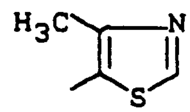

oder 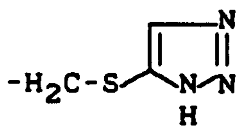

steht,

und deren Salze.

Ganz besonders bevorzugt sind darüberhinaus die in der folgenden Tabelle aufgeführten Verbindungen:

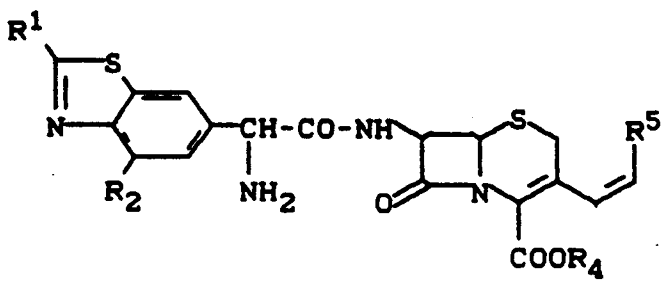

| $R^1$ | $R^2$ | $R^4$ | $R^5$ |
|---|---|---|---|
| $H_3C-$ | H | H | $-CH_3$ |
| $H_2N-$ | H | H | $-C_2H_5$ |
| $H_2N-$ | H | H | $-CH_2Cl$ |
| $H_2N-$ | H | H | $-CH_2-OCH_3$ |
| $H_2N-$ | H | H | $-CH_2I$ |
| H- | H | H | 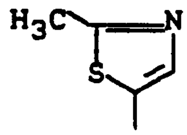 |
| $H_3C-$ | H | H | 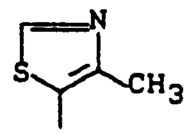 |
| $H_2N-$ | H | H | 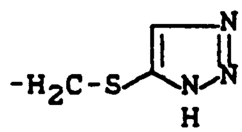 |
| $H_2N$ | H | $CH_2OCOCH_3$ | $CH_3$ |
| $H_2N$ | OH | H | $CH_3$ |
| $H_2N$ | H | $-CH_2OCOCH_3$ | $CH_2-OCH_3$ |
| H | $H_2N$ | H | $CH_3$ |
| $H_2N$ | Cl | H | $CH_3$ |
| $H_2N$ | H | $-CH_2OCOC(CH_3)_3$ | 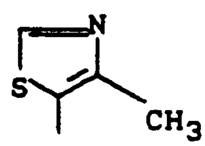 |
| H | OH | H | $CH_3$ |
| $H_2N$ | H | H | 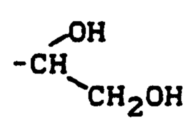 |
| 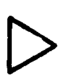 | H | H | $CF_3$ |

| $R^1$ | $R^2$ | $R^4$ | $R^5$ |
|---|---|---|---|
| $CH_3$ | $H_2N$ | H | 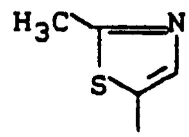 |
| $H_2N$ | OH | $-CH(CH_3)OCOOC_2H_5$ | $CH_3$ |

Darüberhinaus wurde ein Verfahren zur Herstellung der erfindungemäßen substituierten Vinylcephalosporinverbindungen der allgemeinen Formel (I) gefunden, das dadurch gekennzeichnet ist, daß man

[A] substituierte Cephalosporinverbindungen der allgemeinen Formel (II)

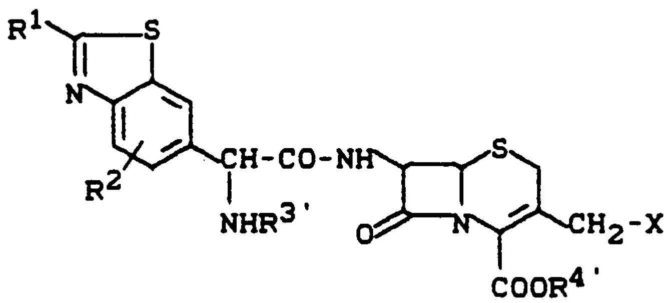

(II)

in welcher

$R^1$ und $R^2$ die oben angegebene Bedeutung hat,

$R^{3'}$ - für eine Aminoschutzgruppe steht,

$R^{4'}$ - für eine Carboxyschutzgruppe steht

und

X - für eine Gruppe der Formel

$-\overset{\oplus}{P}(R^{13})_3Z^{\ominus}$ , 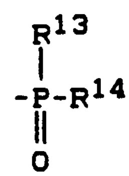 oder 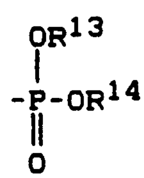

steht,

wobei

$R^{13}$ und $R^{14}$ gleich oder verschieden sind und Alkyl, Phenyl oder Tolyl bedeuten

und

$Z^{\ominus}$ - ein Halogenidanion, bevorzugt Chlorid, Bromid oder Iodid bedeutet,

in inerten Lösemitteln in Anwesenheit von Basen, mit Aldehyden der allgemeinen Formel (III)

$R^5$-CHO (III)

in welcher

$R^5$ die oben angegebene Bedeutung hat

umsetzt,
oder daß man
[B] Phosphoniumverbindungen der allgemeinen Formel (IV)

$R^5$-$CH_2$-X (IV)

in welcher
$R^5$ die oben angegebene Bedeutung hat
und
X
- für eine Gruppe der Formel

$-\overset{\oplus}{P}(R^{13})_3Z^{\ominus}$ , 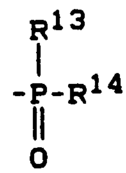 oder 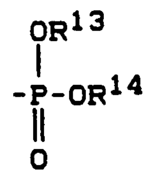 steht,

steht,
wobei
$R^{13}$ und $R^{14}$ gleich oder verschieden sind und Alkyl, Phenyl oder Tolyl bedeuten
und
$Z^{\ominus}$
- ein Halogenidanion, bevorzugt Chlorid, Bromid oder Iodid bedeutet,

in inerten Lösemitteln in Anwesenheit von Basen, mit Cephalosporinaldehyden der allgemeinen Formel (V)

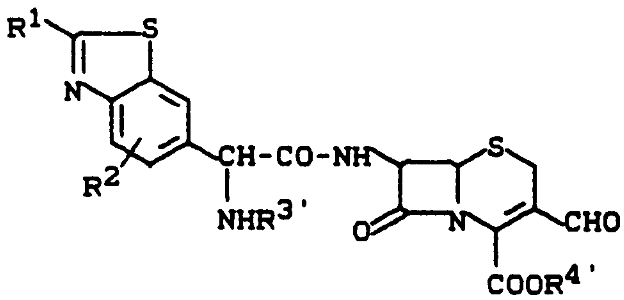
(V)

in welcher
$R^1$ und $R^2$ die oben angegebene Bedeutung haben,
$R^{3'}$ für eine Aminoschutzgruppe steht
und
$R^{4'}$ für eine Carboxyschutzgruppe steht,
umsetzt,
oder, daß man
[C] Carbonsäuren der allgemeinen Formel (VI)

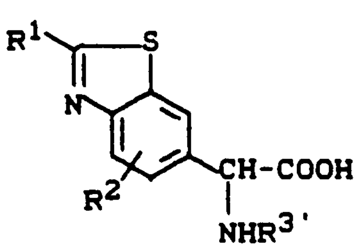
(VI)

in welcher
$R^1$ und $R^2$ die oben angegebene Bedeutung haben,
und
$R^{3'}$ für eine Aminoschutzgruppe steht,
nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester beispielsweise mit N-Hydroxybenztriazol und Dicyclohexylcarbodiimid (DCC),
mit den Vinylcephalosporinaminen der allgemeinen Formel (VII)

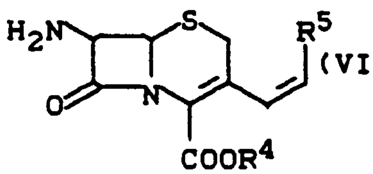
(VII),

in welcher
$R^4$ und $R^5$ die oben angegebene Bedeutung haben,
umsetzt,
dann gegebenenfalls Schutzgruppen abspaltet und die gewünschten Salze oder aus den Salzen die freien Säuren herstellt.

Das erfindungsgemäße Verfahren kann durch das folgende Formelschema erläutert werden:

**Verfahrensvariante A:**

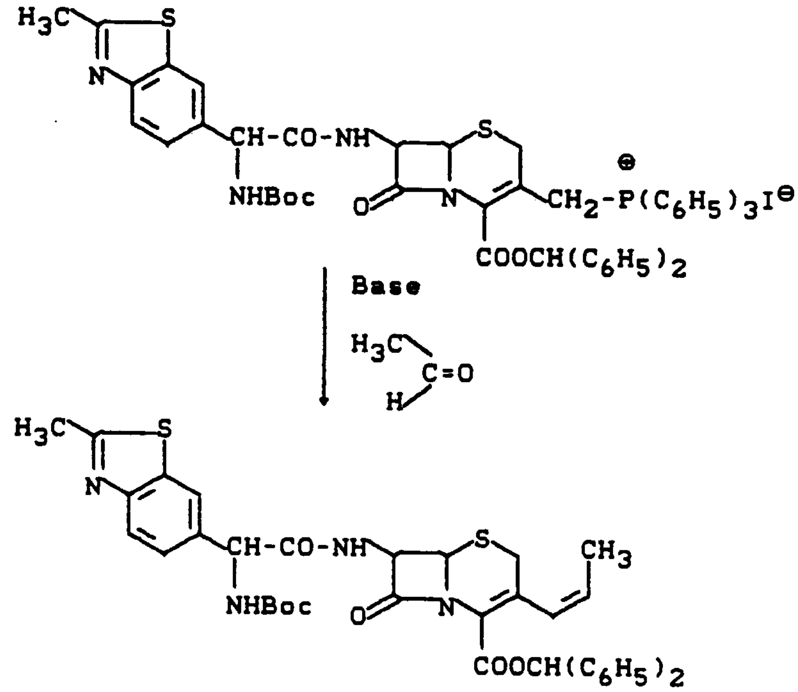


* Boc = $(H_3C)_3C-O-CO-$

**Verfahrensvariante B:**

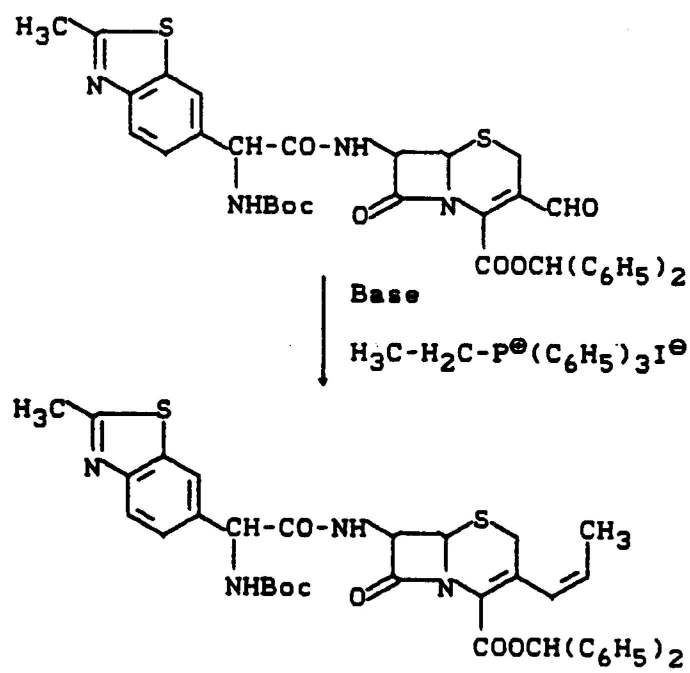


* Boc = $(H_3C)_3C-O-CO-$

**Verfahrensvariante C**

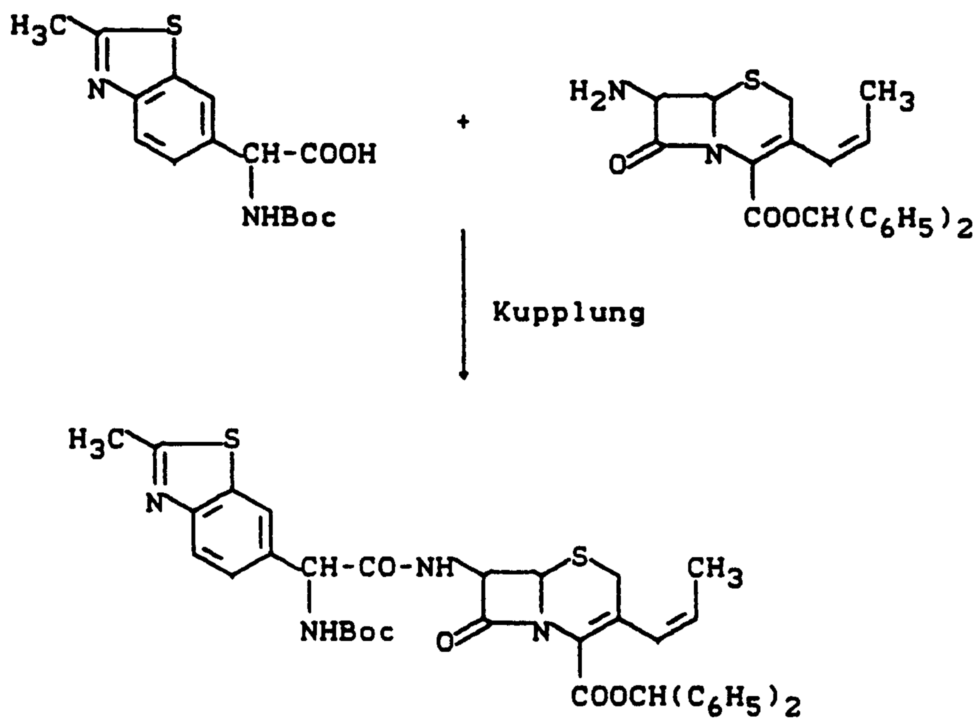


* Boc = $(H_3C)_3C$-O-CO-

Verfahrensvarianten A und B

Als inerte Lösemittel für die Verfahrensvarianten A und B eignen sich die üblichen organischen Lösemittel, die sich unter den Reaktionsbedingungen nicht verändern. Hierzu gehören bevorzugt Ether wie Diethylether, Butylmethylether, Dioxan oder Tetrahydrofuran, oder Kohlenwasserstoffe wie Benzol, Toluol, Xylol oder Cyclohexan, oder Amide wie Dimethylformamid oder Hexamethylphosphorsäuretriamid, oder Alkohole wie Methanol, Ethanol, Propanol oder Isopropanol, oder Chlorkohlenwasserstoffe wie Methylenchlorid, Chloroform oder Tetrachlorkohlenstoff, oder Aceton, Acetonitril oder Essigester. Ebenso ist es möglich, Gemische der genannten Lösemittel zu verwenden.

Als Basen für Verfahrensvarianten A und B eignen sich die üblichen basischen Verbindungen. Hierzu gehören bevorzugt Alkali- oder Erdalkalihydroxide wie beispielsweise Natriumhydroxid, Kaliumhydroxid oder Bariumhydroxid, oder Alkalicarbonate wie Natriumcarbonat, Natriumhydrogencarbonat oder Kaliumcarbonat, oder Alkalialkoholate wie Natriummethanolat, Natriumethanolat, Kaliummethanolat, Kaliumethanolat oder Kalium-tert.butylat.

Die Wahl des Lösemittels bzw. der Base richtet sich nach Stabilität, Hydrolyssempfindlichkeit bzw. CH-Azidität der entsprechenden Phosphorverbindung. Besonders bevorzugt verwendet man als Lösemittel Chlorkohlenwasserstoffe wie beispielsweise Methylenchlorid, Chloroform oder Tetrachlorkohlenstoff in Anwesenheit von Dimethylformamid als Co-Solvens. Als Basen verwendet man besonders bevorzugt Alkalicarbonate wie Natriumcarbonat, Natriumhydrogencarbonat oder Kaliumcarbonat, oder Alkali- oder Erdalkalihydroxide wie beispielsweise Natriumhydroxid, Kaliumhydroxid oder Bariumhydroxid, besonders bevorzugt in Form ihrer wäßrigen Lösungen.

Die Umsetzung wird im allgemeinen in einem Temperaturbereich von -30 °C bis +80 °C, bevorzugt von 0 °C bis +30 °C durchgeführt.

Die Umsetzung kann bei normalem, erhöhtem oder bei erniedrigtem Druck durchgeführt werden (z.B. in einem Bereich von 0,5 bis 5 bar). Im allgemeinen arbeitet man bei Normaldruck.

Bei der Durchführung der Verfahrensvarianten A und B werden im allgemeinen die Phosphorverbindungen (II) bzw. (IV) in einer Menge von 1 bis 3 mol, bevorzugt in molaren Mengen, bezogen auf 1 mol der

Aldehyde (III) bzw. (V) eingesetzt. Die Basen werden im allgemeinen in einer Menge von 1 bis 5 mol, bevorzugt von 1 bis 2 mol, bezogen auf 1 mol der Phosphorverbindungen eingesetzt.

Besonders bevorzugt werden die Verfahrensvarianten A und B als Wittig-Reaktion durchgeführt. Bei der Durchführung des erfindungsgemäßen Verfahrens ist es ebenso möglich, anstelle der Phosphoniumsalze [X = $-P(R^{13})_3^{\oplus}Z^{\ominus}$] die entsprechenden Phosphorane

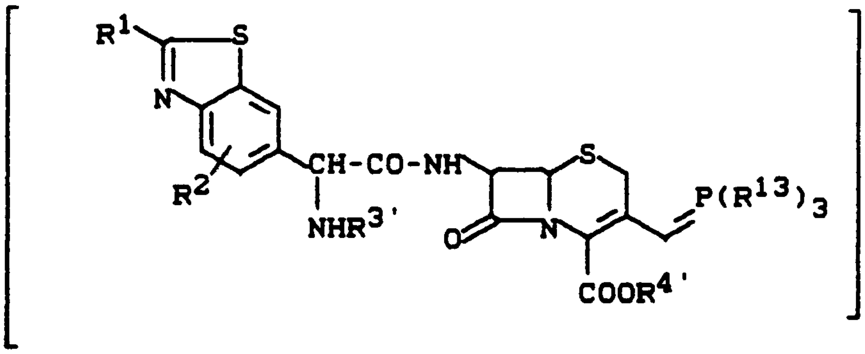

direkt einzusetzten, die man zuvor aus den entsprechenden Phosphoniumsalzen und Base in einer gesonderten Umsetzung hergestellt hat. Es hat sich jedoch als günstig erwiesen, die Umsetzung mit den Triphenylphosphoniumsalzen ( X = $P^{\oplus}(C_6H_5)_3Z^{\ominus}$) in Anwesenheit von Basen als Eintopfverfahren durchzuführen. Als besondere Variante eines Eintopfverfahrens kann die Umsetzung je nach der Stabilität der Phosphorverbindungen auch in Form einer phasentransferkatalysierten Reaktion durchgeführt werden, wobei als Lösemittel Ether, Kohlenwasserstoffe sowie Halogenkohlenwasserstoffe verwendet werden und als Basen wäßrige Natriumhydroxid-oder Kaliumhydroxidlösungen eingesetzt werden.

Wird die Reaktion alternativ derart durchgeführt, daß das entsprechende Phosphoran als Zwischenverbindung isoliert wird und in einem zweiten Schritt mit dem Aldehyd umgesetzt wird, wurde darüberhinaus gefunden, daß durch Zugabe eines geeigneten Lithiumhalogenids wie beispielsweise Lithiumchlorid, Lithiumbromid oder Lithiumiodid die Ausbeute und das Verhältnis von Z-/E-Isomer der Endprodukte der allgemeinen Formel (I) verbessert wird. Die Umsetzung wird hierbei vorzugsweise mit 10 bis 15 Äquivalenten Lithiumhalogenid durchgeführt.

Besonders bevorzugt ist jedoch die Durchführung der Verfahrensvarianten A und B als Eintopfreaktion ohne Isolierung des Zwischenproduktes. Die erfindungsgemäßen Verfahrensvarianten können beispielsweise durchgeführt werden, indem man zu den Phosphoniumverbindungen, in einem geeigneten Lösemittel gelöst oder suspendiert, die Base und dann einen entsprechenden Aldehyd, gegebenfalls in einem geeigneten Lösemittel, zugibt und gegebenenfalls erwärmt. Die Aufarbeitung erfolgt in üblicher Art und Weise durch Extraktion, Chromatographie und/oder Kristallisation.

Weitere spezielle Verfahrenvarianten für die Wittig-Reaktion sind u.a. an den folgenden Stellen beschrieben: J. Fuhrhop und G. Penzlin: Organic Synthesis, Verlag Chemie, 1983, Seite 26 - 35; R.K. Mackie und D.M. Smith: Guidebook to Organic Synthesis, Longman Group Limited, 1982, Seite 93 - 99; H.O. House: Stereochemistry of the Wittig-Reaction with stabilized Ylides: J. Org. Chem. 29, 3327 - 3333 (1964).

Verfahrensvariante C

Es hat sich als vorteilhaft erwiesen, Aminosäuren zu aktivieren und dann mit $\beta$-Lactamen, die als Salze mit einem Amin in Lösung gebracht werden, zu kuppeln.

Besonders vorteilhaft ist die Aktivierung von Carbonsäurederivaten der allgemeinen Formel (VI) mit (a) Sulfonsäurederivaten der allgemeinen Formel (VIII oder mit (b) Chlorameisensäureestern, bevorzugt -ethylester zu Anhydriden der allgemeinen Formel (IXa, b), wie im folgenden Reaktionsschema verdeutlicht wird:

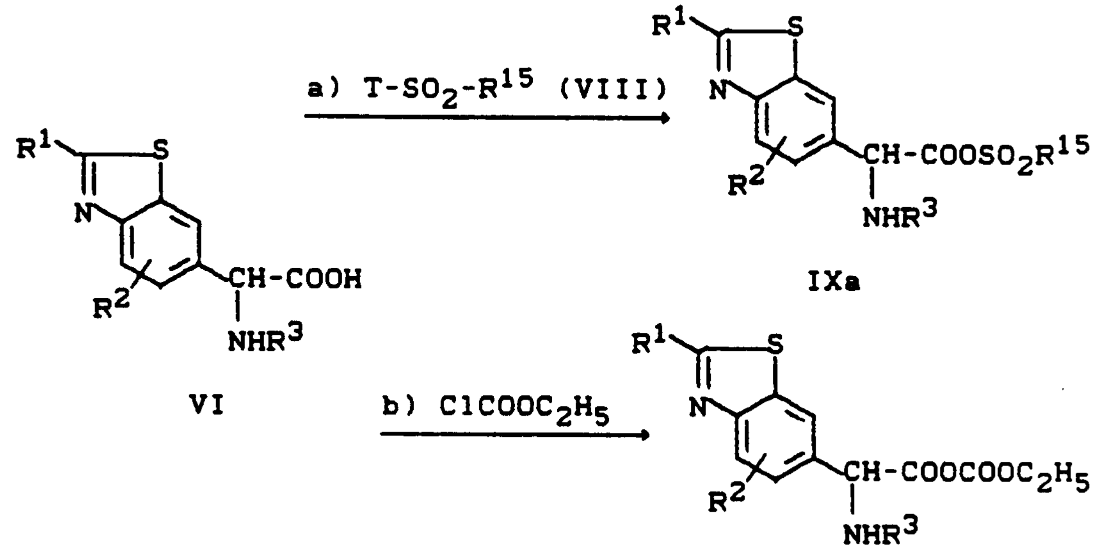


VI

IXa

IXb

Hierbei steht in der Formel (VIII) bzw. (IXa)

T
- für den Rest $R^{15}$-$SO_2$-O- oder Halogen

und

$R^{15}$
- für Alkyl mit bis zu 10 Kohlenstoffatomen, das gegebenenfalls substituiert ist durch Fluor, Chlor, Cyano, Alkyl, Alkoxycarbonyl, Alkoxy oder Alkyl mit jeweils bis zu 4 Kohlenstoffatomen, oder
- für Phenyl, das gegebenenfalls substituiert ist durch Fluor, Chlor, Brom, Cyano, Alkyl, Alkoxy, Alkylthio, Alkoxycarbonyl mit jeweils bis zu 4 Kohlenstoffatomen, Nitro, Trifluormethyl oder Phenyl.

Wenn $R^{15}$ substituiert ist, sind bevorzugt 1 bis 3 Substituenten, besonders bevorzugt die oben genannten vorhanden.

Ganz besonders bevorzugt stellt $R^{15}$ einen Methyl- oder p-Tolylrest dar.

Die gemischten Anhydride der allgemeinen Formel (IXa,b) werden hergestellt, indem man die Carbonsäuren der allgemeinen Formel (VI) und 1 bis 1,4 Äquivalente eines Amins in einem Lösemittel löst und mit 1 bis 1,2 Äquivalenten eines Sulfonsäurederivates der Formel (VIII) oder eines Chlorameisensäuresters reagieren läßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlormethan, oder Amide wie Dimethylformamid oder Hexamethylphosphorsäuretriamid, oder Acetonitril oder Aceton. Ebenso ist es möglich, Gemische der genannten Lösemittel einzusetzen.

Als Amine eignen sich tertiäre Amine, wie beispielsweise Triethylamin, Ethyl-diisopropylamin oder Tributylamin, aber auch sterisch gehinderte sekundäre Amine, wie beispielsweise Diisopropylamin. Ebenso können Gemische der genannten Amine eingesetzt werden.

Die Umsetzungen können bei Temperaturen zwischen -80 °C und Raumtemperatur durchgeführt werden. Vorteilhaft wird die Aktivierung mit Methansulfonsäurechlorid in Dimethylformamid bei -40 °C bis -60 °C innerhalb von 0,2 bis 24 Stunden, vorzugsweise 0,5 bis 5 Stunden durchgeführt.

Zum Lösen der Vinylcephalosporinamine der Formel (VII) für die Umsetzung mit den Verbindungen der Formel (IXa) oder (IXb) :zu den Verbindungen der Formel (I) können die bei der Herstellung der Verbindungen der Formel (IX) genannten Lösemittel oder Wasser, sowie als Base die dort genannten Amine verwendet werden. Besonders vorteilhaft ist auch eine Aktivierung der Carbonsäuren der allgemeinen Formel (VI) durch Überführung in einen aktivierten Ester mit beispielsweise Dicyclohexylcarbodiimid gegebenenfalls in Anwesenheit von N-Hydroxysuccinimid oder 1-Hydroxybenztriazol.

Als Lösemittel eignen sich hierbei alle Solventien, die sich auch für die Herstellung von Anhydriden der allgemeinen Formel (IXa,b) eignen und dort bereits aufgeführt sind.

Die Umsetzungen können bei Temperaturen zwischen -30 °C und +100 °C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit den Vinylcephalosporinaminen der Formel (VII) in Form einer Lösung ihres Aminsalzes innerhalb von 2 bis 24 Stunden umsetzt. Zum Lösen der Vinylcephalosporinamine der Formel (VII) können die bei der Herstellung der Verbindungen der Formel (IX) genannten Lösemittel sowie als Base die dort genannten Amine verwendet werden.

Die als Ausgangsstoffe eingesetzten Aldehyde der allgemeinen Formel (III) sind bekannt oder können nach bekannten Methoden hergestellt werden [Houben-Weyl's "Methoden der organischen Chemie" Bd. VII/1; E2].

Die als Ausgangsverbindungen eingesetzten Cephalosporinaldehyde der allgemeinen Formel (V) sind bekannt oder können nach bekannten Methoden durch Oxidation der entsprechenden 3-Hydroxymethylcephalosporinverbindungen mit Chromtrioxid in Aceton (Jones-Reagenz) hergestellt werden, wie es beispielsweise von J.A. Webber, J. L. Ott and R.T. Vasileff in J. Med. Chemistry 18, 986 (1987) beschrieben wird.

Die als Ausgangsstoffe eingesetzten Phosphoniumverbindungen der allgemeinen Formel (IV) sind bekannt oder können nach bekannten Methoden hergestellt werden [Houben-Weyl's "Methoden der organischen Chemie" Bd. XII/1, 33, 167; Bd. V/1b, 383, 872].

Die als Ausgangsstoffe eingesetzten substituierten Cephalosporinverbindungen der allgemeinen Formel (II) sind teilweise neu und können hergestellt werden,
indem man
Halogenmethylcephalosporinverbindungen der allgemeinen Formel (X)

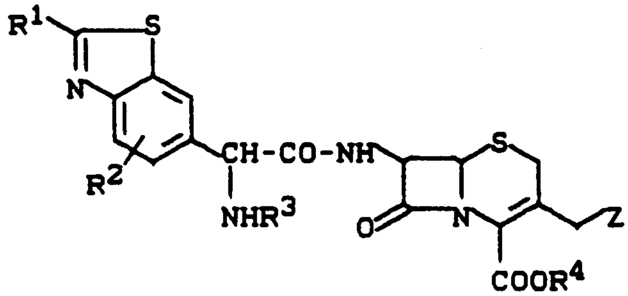

(X)

in welcher
$R^1$, $R^2$, $R^3$ und $R^4$ die oben angegebene Bedeutung haben,
und
Z
- für Halogen, bevorzugt für Chlor, Brom oder Iod steht,

ohne Lösemittel oder in inerten Lösemitteln mit Phosphorverbindungen der allgemeinen Formel (XI)

X| (XI)

wobei
X|
- für eine Phosphorverbindung der Formel XIa, XIb, XIc

$|P(R^{13})_3$ , 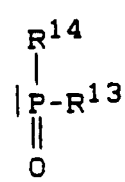 oder 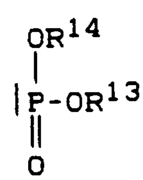

XIa XIb XIc

steht,

wobei

$R^{13}$ und $R^{14}$ gleich oder verschieden sind und

- für Alkyl, Phenyl oder gegebenenfalls substituiertes Phenyl stehen,

umsetzt.

Das erfindungsgemäße Verfahren kann durch folgendes Schema verdeutlicht werden:

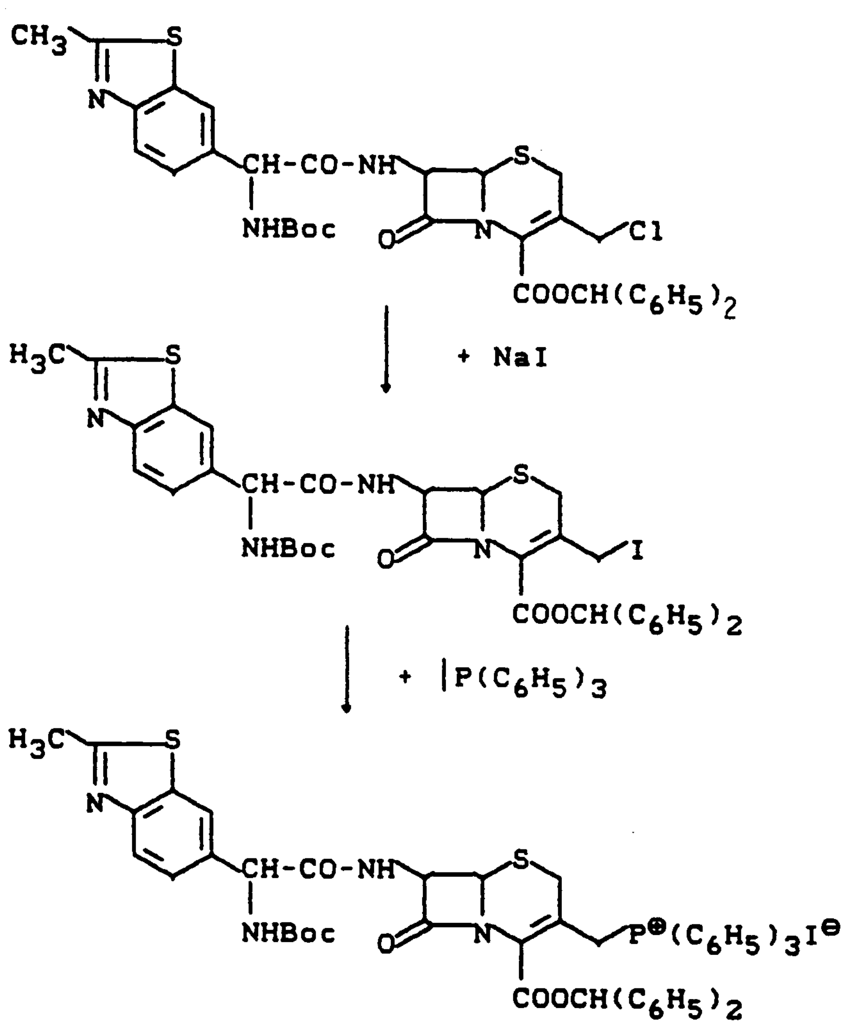


Als inerte Lösemittel eignen sich die üblichen organischen Lösemittel, die unter den Reaktionsbedingungen nicht verändert werden. Hierzu gehören bevorzugt Ether wie Diethylether, Butylmethylether, Dioxan, Tetrahydrofuran oder Glykoldimethylether, oder Kohlenwasserstoffe wie Benzol, Toluol, Xylol, Hexan, Cyclohexan oder Erdölfraktionen, oder Halogenkohlenwasserstoffe wie Methylenchlorid, Chloroform, Tetrachlorkohlenstoff oder Chlorbenzol, oder Essigester, Aceton, Dimethylformamid, Hexamethylphosphorsäuretriamid oder Dimethylacetamid. Ebenso ist es möglich, Gemische der genannten Lösemittel einzusetzen.

Die Umsetzung wird im allgemeinen in einem Temperaturbereich von 0° C bis +150° C, bevorzugt von +20° C bis +180° C, durchgeführt.

Die Umsetzung kann bei normalem, erhöhtem oder erniedrigtem Druck durchgeführt werden. Im allgemeinen arbeitet man bei Normaldruck.

Die Umsetzung erfolgt im allgemeinen derart, daß man die Halogenmethylcephalosporinverbindung und die Phosphorverbindung in einem inerten Lösemittel mischt und gegebenenfalls erwärmt. Hierbei wird die Phosphorverbindung im allgemeinen in einer Menge von 1 bis 5, bevorzugt von 1 bis 2 mol, bezogen auf 1 mol der Chlormethylcephalosporinverbindung eingesetzt.

Bei der Durchführung des erfindungsgemäßen Verfahrens hat es sich als besonders günstig erwiesen, als Halogenmethylcephalosporinverbindungen die entsprechenden Iodverbindungen einzusetzen (X = I),

die man aus den entsprechenden Chlormethyl- bzw. Brommethylverbindungen durch Behandeln mit Natriumiodid in Dimethylformamid oder Aceton erhält. Darüberhinaus ist es möglich, bei der Verwendung der Chlormethyl- bzw. Brommethylverbindungen die Umwandlung zur Iodverbindung und die Umsetzung mit der Phosphorverbindung als Eintopfreaktion durchzuführen. Hierzu werden die entsprechenden Brommethyl- bzw. Chlormethylcephalosporinverbindungen in einem geeigneten Lösemittel wie beispielsweise Ether, Essigester, Kohlenwasserstoffe, Chlorkohlenwasserstoffe, bevorzugt Aceton, mit Natriumiodid und der entsprechenden Phosphorverbindungen umgesetzt.

Die als Ausgangsstoffe eingesetzten Halogenmethylcephalosporinverbindungen der allgemeinen Formel (X) sind neu.

Es wurde ein Verfahren zur Herstellung der Halogenmethylcephalosporine der allgemeinen Formel (X) gefunden, das dadurch gekennzeichnet ist, daß man
Carbonsäuren der allgemeinen Formel (VI)

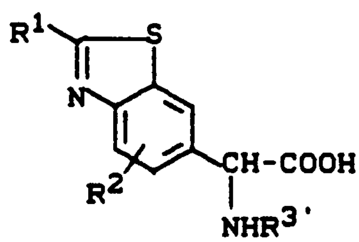

(VI)

in welcher
$R^1$ und $R^2$ die angegebene Bedeutung haben,
und
$R^{3'}$ für eine Aminoschutzgruppe steht,
nach Aktivierung der Carboxylgruppe durch Überführen in ein gemischtes Anhydrid, beispielsweise mit Chlorameisensäureethylester, Chlorameisensäureisobutylester oder Methansulfonsäurechlorid, oder durch Überführen in das Säurehalogenid, oder durch Überführen in einen aktivierten Ester mit beispielsweise N-Hydroxybenztriazol und Dicyclohexylcarbodiimid
mit einer $\beta$-Lactam-Verbindung der allgemeinen Formel (XII)

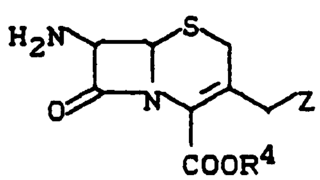

(XII)

in welcher
$R^4$ und Z die oben angegebene Bedeutung haben
umgesetzt werden, dann gegebenenfalls Schutzgruppen abgespaltet werden und die gewünschten Salze oder aus den Salzen die freien Säuren hergestellt werden.

Für die Kupplung von Carbonsäuren (VI) an die $\beta$-Lactam-Verbindung (XII) kann eine große Zahl von aus der Cephalosporin- bzw. Penicillinchemie bekannten Methoden verwendet werden. Es hat sich als vorteilhaft erwiesen, die Carbonsäuren der allgemeinen Formel (VI) mit Aminschutzgruppe ($R_3$) zu aktivieren und dann mit der $\beta$-Lactam-Verbindung der Formel (XII), die als Salze mit einem Amin in Lösung gebracht wurden, zu kuppeln.

Besonders vorteilhaft ist die Aktivierung von Carbonsäurederivaten der allgemeinen Formel (VI) mit (a) Sulfonsäurederivaten der allgemeinen Formel (VIII) oder mit (b) Chlorameisensäureestern, bevorzugt -ethylester zu Anhydriden der allgemeinen Formel (IXa,b), wie im folgenden Reaktionsschema verdeutlicht wird:

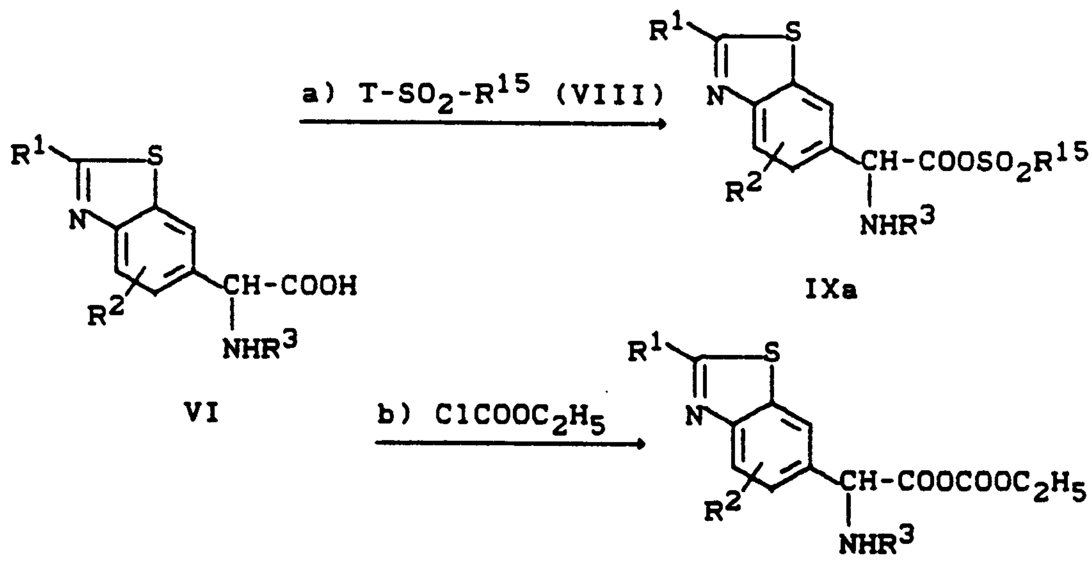


Hierbei steht in der Formel (VIII) bzw. (IXa)

T
- für den Rest $R^{15}$-$SO_2$-O- oder Halogen

und

$R^{15}$
- für Alkyl mit bis zu 10 Kohlenstoffatomen, das gegebenenfalls substituiert ist durch Fluor, Chlor, Cyano, Alkyl, Alkoxycarbonyl, Alkoxy oder Alkyl mit jeweils bis zu 4 Kohlenstoffatomen, oder
- für Phenyl, das gegebenenfalls substituiert ist durch Fluor, Chlor, Brom, Cyano, Alkyl, Alkoxy, Alkylthio, Alkoxycarbonyl mit jeweils bis zu 4 Kohlenstoffatomen, Nitro, Trifluormethyl oder Phenyl.

Wenn $R^{15}$ substituiert ist, sind bevorzugt 1 bis 3 Substituenten, besonders bevorzugt die oben genannten vorhanden.

Ganz besonders bevorzugt stellt $R^{16}$ einen Methyl- oder p-Tolylrest dar.

Die gemischten Anhydride der allgemeinen Formel (IXa,b) werden hergestellt, indem man die Carbonsäuren der allgemeinen Formel (VI) und 1 bis 1,4 Äquivalente eines Amins in einem Lösemittel löst und mit 1 bis 1,2 Äquivalenten eines Sulfonsäurederivates der Formel (VIII) oder Chlorameisensäureester reagieren läßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlormethan, oder Amide wie Dimethylformamid oder Hexamethylphosphorsäuretriamid, oder Acetonitril oder Aceton. Ebenso ist es möglich, Gemische der genannten Lösemittel einzusetzen.

Als Amine eignen sich tertiäre Amine, wie beispielsweise Triethylamin, Ethyl-diisoproplyamin oder Tributylamin, aber auch sterisch gehinderte sekundäre Amine, wie beispielsweise Diisopropylamin. Ebenso können Gemische der genannten Amine eingesetzt werden.

Die Umsetzungen können bei Temperaturen zwischen -80 °C und Raumtemperatur durchgeführt werden. Vorteilhaft wird die Aktivierung mit Methansulfonsäurechlorid in Dimethylformamid bei -40 °C bis -60 °C innerhalb von 0,2 bis 24 Stunden, vorzugsweise 0,5 bis 5 Stunden durchgeführt.

Zum Lösen der $\beta$-Lactam-Verbindung der Formel (VII) für die Umsetzung mit den Verbindungen der Formel (IXa) oder (IXb) zu den Verbindungen der Formel (X) können die bei der Herstellung der Verbindungen der Formel (IX) genannten Lösemittel oder Wasser, sowie als Base die dort genannten Amine verwendet werden.

Besonders vorteilhaft ist auch eine Aktivierung der Carbonsäuren der allgemeinen Formel (VI) durch Überführung in einen aktivierten Ester mit beispielsweise Dicyclohexylcarbodiimid gegebenenfalls in Anwesenheit von N-Hydroxysuccinimid oder 1-Hydroxybenztriazol.

Als Lösemittel eignen sich hierbei alle Solventien, die sich auch für die Herstellung von Anhydriden der allgemeinen Formel (IXa, b) eignen und dort bereits aufgeführt sind.

Die Umsetzungen können bei Temperaturen zwischen -30 °C und +100 °C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit der $\beta$-Lactam-Verbindung der Formel (XII) in Form einer Lösung ihres Aminsalzes innerhalb von 2 bis 24 Stunden umsetzt. Zum Lösen der $\beta$-Lactam-Verbindung der Formel (XII) können die bei der Herstellung der Verbindungen der Formel (IX) genannten Lösemittel, sowie als Base die dort genannten Amine verwendet werden.

Die als Ausgangsstoffe eingesetzten Carbonsäuren der allgemeinen Formel (VI) sind bekannt oder können nach bekannten Methoden hergestellt werden [DE-OS 35.08.258].

Die als Ausgangsstoffe eingesetzten $\beta$-Lactam-Verbindungen der allgemeinen Formel (XII) sind bekannt oder können nach bekannten Methoden hergestellt werden [US-Patentschrift 4 639 448 und DE-OS 3 402 642].

Die als Ausgangsstoffe eingesetzten Amino-$\beta$-Lactame der allgemeinen Formel (VII) sind bekannt oder können nach bekannten Methoden hergestellt werden [DE-OS 34.02.642; US-Patentschrift 4.639.448].

Die erfindungsgemäßen Verbindungen der allgemeinen Formel I weisen bei geringer Toxizität ein breites antibakterielles Spektrum gegen gram-positive und gram-negative Keime und anaerobe Bakterien auf. Diese Eigenschaften ermöglichen ihre Verwendung als chemotherapeutische Wirkstoffe in der Human- und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria genorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis. Insbesondere wirken die erfindungsgemäßen Stoffe gegen Staphylokokken, Streptokokken,Enterokokken und Haemophilus influenzae. Bei parenteraler oder insbesondere oraler Verabreichung sind die neuen Verbindungen gegen Mikroorganismen wie Staphylokokken, Streptokokken, Enterobakteriaceen, Escherichia coli, Klebsiella, Salmonella, Shigella und Proteus sehr gut wirksam.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, post-operative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

In den folgenden Tabellen sind die minimalen Hemmkonzentrationen (MHK-Werte, µg/ml) für das Beispiel 6 im Vergleich mit Cefaclor [M. Gorman et al., Cefaclor, Chronicles of Drug Discovery, Vol. 2, 49, J. Wiley & Sons (1983)] angegeben. Die MHK-Werte werden durch den Agarverdünnungstest mit Hilfe eines Multipoint-Inokulators bestimmt, wobei die Ablesung nach 18 bis 24-stündiger Bebrütung bei 37 °C erfolgt. Als Wuchsmedium wird Isosensitest-Agar verwendet.

| Haemophilus influenzae Nr. | Beispiel 6 | Cefaclor |
|---|---|---|
| 1 | 1 | 4 |
| 2 | 0,5 | 2 |
| 4 | 1 | 8 |
| 5 | 0,5 | 4 |
| 6 | 0,5 | 4 |
| 7 | 0,5 | 2 |
| 8 | 0,5 | 1 |
| 9 | 0,5 | 1 |
| 10 | 0,5 | 2 |

| ICB-Nr. | | Beispiel 6 | Cefaclor |
|---|---|---|---|
| Staph. aureus | 25 412 | 32 | 256 |
| | 25 413 | 0,5 | 4 |
| | 25 414 | 0,5 | 32 |
| | 25 417 | ≤ 0,5 | 4 |
| | 25 418 | ≤ 0,5 | 4 |
| | 25 473 | ≤ 0,5 | 4 |
| | 25 559 | ≤ 0,5 | 8 |
| | 25 560 | ≤ 0,5 | 8 |
| | 25 565 | ≤ 0,5 | 4 |
| | 25 568 | ≤ 0,5 | 4 |
| | 25 569 | ≤ 0,5 | 4 |
| | 25 470 | 0,5 | 8 |
| | 25 508 | 1 | 8 |
| | 25 527 | 2 | 64 |
| | 25 397 | ≤ 0,5 | 8 |
| | 25 523 | ≤ 0,5 | 8 |
| | 25 524 | ≤ 0,5 | 16 |
| | 25 525 | ≤ 0,5 | 4 |
| | 25 583 | 4 | 16 |

| ICB-Nr. | | Beispiel 6 | Cefaclor |
|---|---|---|---|
| Strep. faecalis | 27 261 | 8 | > 256 |
| | 27 249 | 16 | > 256 |
| | 27 250 | 16 | > 256 |
| | 27 251 | 32 | > 256 |
| | 27 252 | 16 | > 256 |
| | 27 253 | 16 | > 256 |
| | 27 254 | 16 | > 256 |
| | 27 255 | 16 | > 256 |
| | 27 256 | 4 | > 256 |
| | 27 257 | 4 | > 256 |

| ICB-Nr. | | Beispiel 6 | Cefaclor |
|---|---|---|---|
| Klebs. pneumoniae | 6310 | ≤ 0,5 | 1 |
| | 6318 | 4 | 2 |
| | 6360 | 64 | 8 |
| | 6362 | ≤ 0,5 | 1 |
| | 6379 | ≤ 0,5 | 1 |
| | 6380 | ≤ 0,5 | 1 |

| ICB-Nr. | | Beispiel 6 | Cefaclor |
|---|---|---|---|
| E. Coli | 4895 | 2 | 1 |
| | 4322 | 2 | 1 |
| | 4800 | 2 | 1 |
| | 4815 | 0,5 | 2 |

| Anaerobe Bakterien Keime | Beispiel 6 | Cefaclor |
|---|---|---|
| 1 Bacteroides fragilis | 1 | 256 |
| 4 Bacteroides fragilis | 0,5 | 128 |
| 6 Bacteroides fragilis | 0,5 | 128 |
| 10 Bacteroides fragilis | 0,5 | 128 |
| 13 Bacteroides thetaiotaomicron | 4 | > 256 |
| 15 Bacteroides distasonis | ≤ 0,5 | 2 |
| 16 Bacteroides ovatus | 8 | > 256 |
| 17 Clostridium perfringens | ≤ 0,5 | 2 |
| 18 Bacteroides vulgatus | ≤ 0,5 | 4 |
| 19 Clostridium perfringens | ≤ 0,5 | ≤ 0,5 |

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder

und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen.

Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können

die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannnten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die erfindungsgemäßen Verbindungen können zum Zwecke der Erweiterung des Wirkungsspektrums und um eine Wirkungssteigerung speziell bei β-lactamasebildenden Bakterien zu erzielen mit anderen antimikrobiellen Wirkstoffen und Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

## Herstellungsbeispiele

### Beispiel 1

7-Amino-3-chlormethyl-3-cephem-4-carbonsäurebenzhydrylester

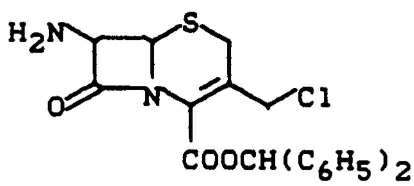

Zu einer Suspension von 50 g (0,0972 mol) 7-Phenylacetamido-3-hydroxymethyl-3-cephem-4-carbonsäurebenzhydrylester in 500 ml Methylenchlorid gibt man bei Raumtemperatur 19,64 ml (0,242 mol) Pyridin. Nach Abkühlung auf -20 °C werden 40,48 g (0,0972 mol) Phosphorpentachlorid zugegeben und 5 Minuten bei -20 °C gerührt. Mit einem Eisbad wird auf 0 ° C erwärmt und 10 Minuten gerührt, anschließend wird mit einem Wasserbad auf 15 °C erwärmt und 1 Stunde gerührt. Danach wird der Ansatz auf -70 °C gekühlt und 720 ml kaltes Methanol schnell zugegeben. Dann wird 5 Minuten bei -70 °C, 10 Minuten bei 0 °C und 25 Minuten bei +15 °C gerührt. Anschließend wird die Lösung im Vakuum stark eingeengt und mit 1400 ml gesättigte Natriumhydrogencarbonat-Lösung versetzt. Man extrahiert diese Lösung dreimal mit Methylenchlorid, trocknet die organische Phase mit Natriumsulfat und engt im Vakuum ein. Das Rohprodukt wird an 500 g Kieselgel 60 (0,04 - 0,063 mm) mit Methylenchlorid chromatographiert.

| | |
|---|---|
| Ausbeute: | 29,0 g (72% der Theorie) |
| | $C_{21}H_{19}ClN_2O_3S$ (414,9) |
| NMR ($CDCl_3$): | δ = 2,06 (s, 2H); 3,45 (d, 1H); 3,62 (d, 1H); 4,25 - 4,41 (q, 2H); 4,75 (d, 1H); 4,93 (d, 1H); 6,97 (s, 1H); 7,25 - 7,46 (m, 10H) ppm. |

Beispiel 2

D-7-[2-(t-Butoxycarbonylamino)-2-(2-aminobenzothiazol-6-yl)-glycylamido]-3-chlormethyl-3-cephem-4-carbonsäurebenzhydrylester

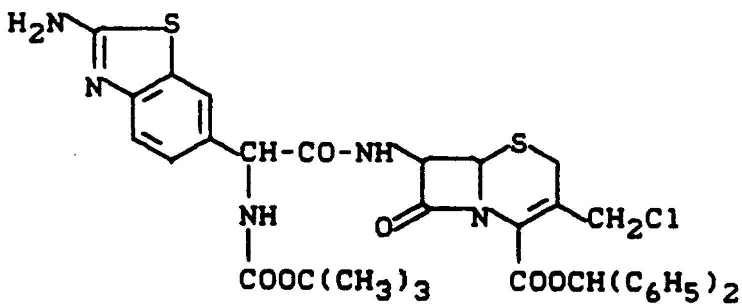

Zu einer Mischung von 28,3 g (0,0875 mol) D-α-t-Butoxycarbonylamino-α-(2-aminobenzothiazol-6-yl)-essigsäure und 24,1 g (0,058 mol) 7-Amino-3-chlormethyl-3-cephem-4-carbonsäurebenzhydrylester (Beispiel 1) in 136 ml Tetrahydrofuran und 77 ml Dimethylformamid gibt man 18,07 g (0,0875 mol) N,N'-Dicyclohexylcarbodiimid (DCC) gelöst in 150 ml Tetrahydrofuran bei 0°C hinzu, rührt anschließend 2 Stunden bei Raumtemperatur und engt zur Trockne ein. Der Rückstand wird in 1200 ml Essigester suspendiert, 10 Minuten gerührt und danach ungelöste Bestandteile durch Absaugen abgetrennt. Nach Abdestillieren von Essigester wird der Rückstand an Kieselgel 60 (0,04 - 0,063 mm) mit Toluol / Essigester (1 : 1) chromatographiert.

| | |
|---|---|
| Ausbeute: | 17,6 g (42% der Theorie) |
| | $C_{35}H_{34}ClN_5S_2O_6$ (720,3) |
| NMR (DMSO): | δ = 1,37 (s, 9H); 3,46 (d, 1H); 3,64 (d, 1H); 4,32 - 4,43 (q, 2H); 5,11 (d, 5 Hz, 1H); 5,31 (d, 1H); 5,8 - 5,86 (q, 1H); 6,98 (s, 1H); 7,15 - 7,5 (mm, 14H); 7,67 (s, 1H) ppm |

Beispiel 3

D-7-[2-(t-Butoxycarbonylamino)-2-(2-aminobenzothiazol-6-yl)glycylamido]-3-iodmethyl-3-cephem-4-carbonsäurebenzhydrylester

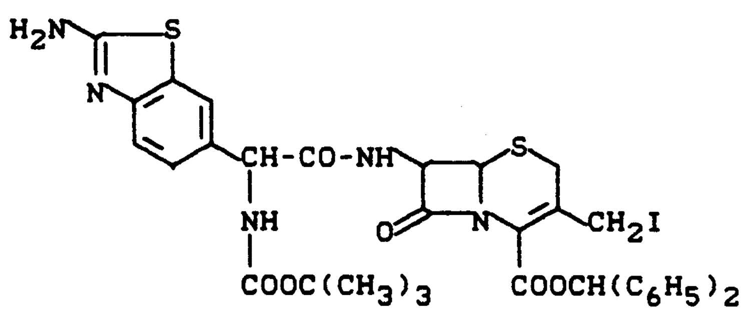

Eine Mischung von 20,3 g (0,0282 mol) D-7-[2-(t-Butoxycarbonylamino)-2-(2-aminobenzothiazol-6-yl)-glycylamido]-3-chlormethyl-3-cephem-4-carbonsäurebenzhydrylester (Beispiel 2) und 12,68 g (0,0846 mol) Natriumiodid in 300 ml Aceton wird 2 Stunden bei Raumtemperatur gerührt und zur Trockne eingedampft. Der Rückstand wird in 500 ml Essigester aufgenommen, mit einer wäßrigen Natriumthiosulfat-Lösung, Wasser und Natriumchlorid-Lösung gewaschen. Nach Trocknen über Natriumsulfat wird vom Lösemittel abdestilliert und der Rückstand in Ether digeriert.

Ausbeute: 22 g

Die Verbindung wird direkt in der nächsten Stufe eingesetzt.

Beispiel 4

D-7-[2-(t-Butoxycarbonylamino)-2-(2-aminobenzothiazol-6-yl)glycylamido]-3-(triphenylphosphonio)methyl-3-cephem-4-carbonsäurebenzhydrylester-iodid

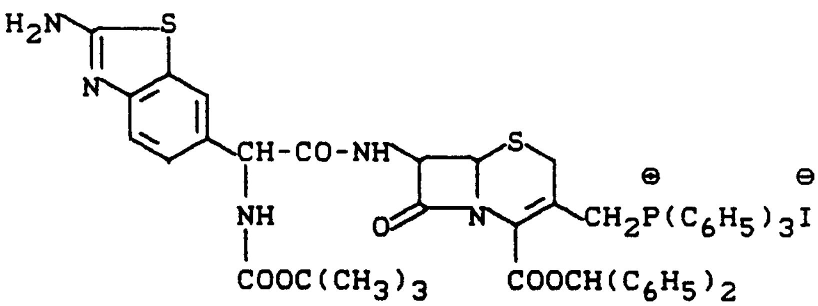

Eine Mischung von 22 g (0,0271 mol) D-7-[2-(t-Butoxycarbonylamino)-2-(2-aminobenzothiazol-6-yl)-glycylamido]-3-iodmethyl-3-cephem-4-carbonsäure-benzhydrylester (Beispiel 3) und 21,32 g (0,0813 mol) Triphenylphosphin in 500 ml Essigester rührt man 1 Stunde bei Raumtemperatur. Das Produkt fällt nach 30 Minuten aus. Man engt die Mischung bei vermindertem Druck auf etwa 150 ml ein und versetzt das Konzentrat mit 500 ml Ether. Der erhaltene Niederschlag wird abgesaugt und mit Ether nachgewaschen.

Ausbeute: 19,6 g (67% der Theorie)
$C_{53}H_{49}IN_5O_6PS_2$ (1074,1)

NMR (DMSO): $\delta$ = 1,35 (s, 9H); 3,32 - 3,42 (dd, 2H); 4,81 - 4,93 (t, 2H); 5,2 (d, 1H); 5,33 (d, 1H); 5,72 - 5,79 (q, 1H); 6,24 (s, 1H); 7,2 - 7,49 (mm, 15H); 7,6 - 7,79 (m, 15H) ppm.

Beispiel 5

D-7-[2-(t-Butoxycarbonylamino)-2-(2-aminobenzothiazol-6-yl)glycylamido]-3-[(Z)-1-propen-1-yl]-3-cephem-4-carbonsäurebenzhydrylester

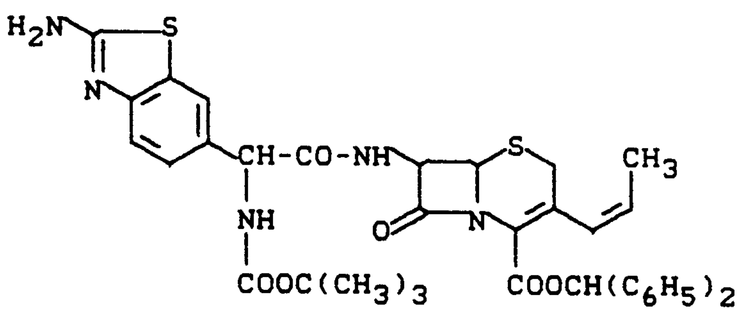

Zu einer kalten Lösung von 6,08 g (0,07 mol) Lithiumbromid in 50 ml Dimethylformamid und 150 ml Methylenchlorid gibt man bei -5°C 7,04 ml (0,126 mol) Acetaldehyd und 7,6 g (0,007 mol) D-7-[2-(t-Butoxycarbonylamino)-2-(2-aminobenzothiazol-6-yl)glycylamido]-3-(triphenylphosphonio)methyl-3-cephem-4-carbonsäurebenzhydrylester-iodid (Beispiel 4). Man rührt die Mischung 20 Stunden bei -5°C und danach 5 Stunden bei Raumtemperatur. Die Lösung wird im Vakuum auf ca. 50 ml eingeengt und in einem Lösemittelgemisch aus 200 ml Essigester und 200 ml Wasser verteilt. Die obere Schicht wird abgetrennt und einmal mit wäßriger Kochsalzlösung gewaschen. Nach Trocknen über Natriumsulfat und Abdestillieren des Lösemittels wird der Rückstand in Toluol aufgenommen und auf eine Säule, die mit Kieselgel (0,04 - 0,063 mm) gepackt ist, gegeben. Zunächst wird mit Toluol und danach mit dem Lösemittelgemisch Toluol / Essigester (5:1) und Toluol / Essigester (1:1) eluiert.

Ausbeute: 2,9 g (58% der Theorie)
$C_{37}H_{37}N_5O_6S_2$ (711,9)

NMR ($CDCl_3$). δ = 1,35 (dd, 3H); 1,43 (s, 9H); 3,15 (d, 1H); 3,31 (d, 1H); 4,97 (d, 1H); 5,3 (s, 1H); 5,46 - 5,55 (m, 1H); 5,71 (breites s, 2H); 5,78 - 5,85 (q, 1H); 6,03 (d, J = 11 Hz, 1H); 6,87 (s, 1H); 7,2 - 7,4 (mm, 12H); 7,5 (s, 1H) ppm.

Beispiel 6

D-7-[(2-Aminobenzothiazol-6-yl)glycylamido]-3-[(Z)-1-propen-1-yl]-3-cephem-4-carbonsäure, cis-Isomer

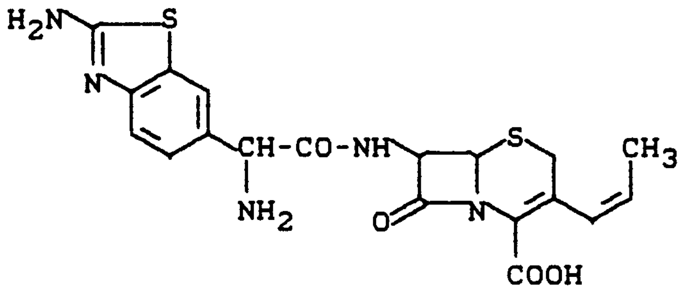

cis

2,9 g (4,1 mmol) D-7-[2-(t-Butoxycarbonylamino)-2-(2-aminobenzothiazol-6-yl)glycylamido]-3-[(Z)-1-propen-1-yl]-3-cephem-4-carbonsäurebenzhydrylester (Beispiel 5) werden in 20 ml Methylenchlorid gelöst, mit 40 ml Trifluoressigsäure (TFA) versetzt und 60 Minuten magnetisch bei Raumtemperatur gerührt. Methylenchlorid und Trifluoressigsäure werden im Vakuum entfernt, das zurückbleibende, halbfeste rote Öl in Ether verrieben, abgesaugt und mit Ether gewaschen. Das hellgelbe Trifluoracetat wird im Vakuum getrocknet, anschließend in 100 ml Wasser suspendiert, von unlöslichen gelben Flocken über Kieselgur abgesaugt und mit 30 ml Wasser nachgewaschen. Die noch leicht trübe Lösung wird nochmals über ein Membranfilter (Millipore, 0,45 µm) filtriert. Das Filtrat wird auf eine RP 18-Säule (Hibar 250-25, Merck) gepumpt. Die Säule wird zunächst mit 200 ml Wasser (Fraktion 1), danach mit 400 ml 5%igem Methanol (Fraktion 2) und schließlich mit 10%igem Methanol eluiert, wobei jeweils 300 ml Fraktionen gesammelt werden (Fraktion 3 bis 12). Die Fraktionen werden mittels analytischer HPLC untersucht und die Fraktionen 6 bis 10, die den gewünschten Peak enthalten, vereinigt, Methanol im Vakuum abdestilliert und lyophilisiert.

| | |
|---|---|
| Ausbeute: | 400 mg |
| | $C_{19}H_{19}N_5O_4S_2$ (445,5) |
| NMR (DCOOD): | δ = 1,67 (dd, 3H); 3,41 (d, 1H); 3,55 (d, 1H); 5,3 (d, 1H); 5,78 (s, 1H); 5,81-5,91 (q und m, 2H); 6,25 (d, J = 11,6 Hz, 1H); 7,81 - 7,9 (q, 2H); 8,18 (s, 1H) ppm. |
| HPLC-analytisch : | Hibar 250-4, RP-8, 10 µm, 254 nm |
| Laufmittel: | 1000 ml $CH_3CN$ - 30 ml Essigsäure - 870 ml Wasser |
| Fluß: | 4 ml/min, Konzentration: 1 mg/ml |
| Retention: | 3,22 (Gehalt: 97,3%) |

Beispiel 7

D-7-[(2-Aminobenzothiazol-6-yl)glycylamido]-3-[(E)-1-propen-1-yl]-3-cephem-4-carbonsäure, trans-Isomer

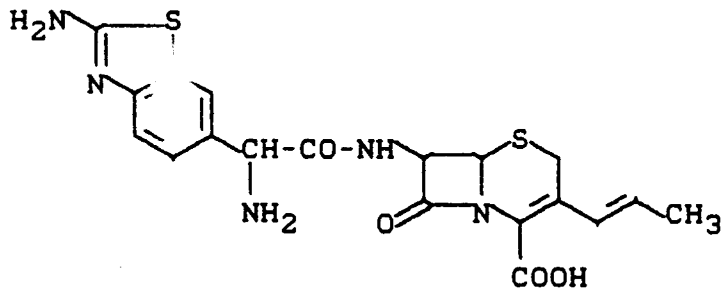

trans

Aus der präparativen Hochdruck-Flüssigkeits-Chromatographie der D-7-[(2-Aminobenzothiazol-6-yl)-glycylamido]-3-[(Z)-1-propen-1-yl]-3-cephem-4-carbonsäure (cis-Isomer; Beispiel 6) gewinnt man durch weitere Elution mit 30%igem Methanol die E-isomere Verbindung.

| | |
|---|---|
| Ausbeute: | 137 mg |
| | $C_{19}H_{19}N_5O_4S_2$ (445,5) |
| NMR (DCOOD): | δ = 1,9 (d, 3H); 3,61 (s, 2H); 5,25 (d, 1H); 5,72 (s, 1H); 5,87 (d, 1H); 6,23-6,46 (m, 1H); 7,04 (d, J = 15,8 Hz, 1H); 7,8 - 7,88 (q, 2H); 8,18 (s, 1H) ppm. |
| HPLC-analytisch: | Hibar 250-4, RP-8 10 µm, 254 nm |
| Laufmittel: | 100 ml $CH_3CN$ - 30 ml Essigsäure - 870 ml Wasser |
| Fluß: | 4 ml/min, Konzentration: 1 mg/ml |
| Retention: | 5,20 (Gehalt: 67,2%) |

Beispiel 8

7-Phenylacetamido-3-(triphenylphosphonio)methyl-3-cephem-4-carbonsäurebenzhydrylester-iodid

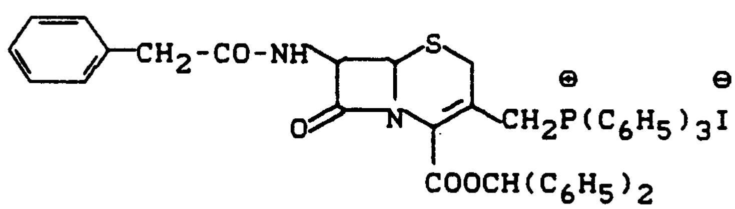

32,5 g (0,0609 mol) 7-Phenylacetamido-3-chlormethyl-3-cephem-4-carbonsäure-benzhydrylester werden in 330 ml Aceton gelöst und unter Rühren mit 10,1 g (0,0674 mol) NaI und 17,6 g (0,0671 mol) Triphenylphosphin nacheinander versetzt. Nach 1,5 Stunden Rühren bei Raumtemperatur wird das unlösliche Material durch Absaugen abgetrennt und die klare Mutterlauge in 1000 ml Ether eingerührt. Das ausfallende weiße flockige Material wird abgesaugt, mit 300 ml Ether gewaschen und im Vakuum getrocknet.

| | |
|---|---|
| Ausbeute: | 51 g (94% der Theorie) |
| | $C_{47}H_{40}IN_2O_4PS$ (886,8) |
| NMR (DMSO): | δ = 3,51 - 3,61 (q, 4H); 4,93 - 5,05 (t, 1H); 5,22 - 5,33 (d und t, 2H); 5,7 - 5,76 (q, 1H); 6,26 (s, 1H); 7,21 - 7,46 (mm, 15H); 7,68 - 7,79 (m, 15H); 9,14 (d, 1H) ppm. |

Beispiel 9

I. 7-Phenylacetamido-3-[(Z)-propen-1-yl]-3-cephem-4-caronsäurebenzhydrylester

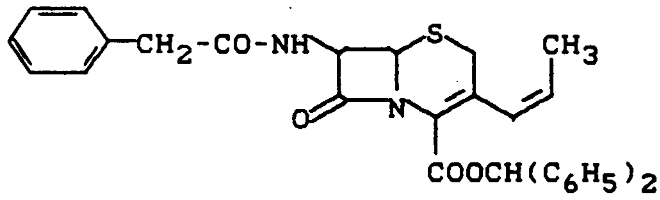

15,9 g (17,9 mmol) 7-Phenylacetamido-3-(triphenylphosphonio)methyl-3-cephem-4-carbonsäurebenzhydrylesteriodid (Beispiel 8) werden in einem 250 ml Dreihalskolben in 100 ml Methylenchlorid und 12,8 ml (229,6 mmol) Acetaldehyd vorgelegt. Man kühlt auf 0 °C und gibt 100 ml Wasser hinzu. Anschließend läßt man unter Konstanthaltung des pH-Wertes bei 8,6 16,3 ml 1N NaOH innerhalb von 4 Stunden zutropfen. Die Reaktionslösung wird mit Methylenchlorid verdünnt, die organische Phase abgetrennt, einmal mit Wasser gewaschen und dann über Natriumsulfat getrocknet. Nach Abtrennen des Trockenmittels wird die Methylenchlorid-Lösung nochmals mit 13 ml (233 mmol) Acetaldehyd versetzt und über Nacht gerührt. Anschließend wird die Reaktionslösung bis zur Trockene eingeengt, erneut in wenig Methylenchlorid gelöst und auf eine Säule gegeben, die mit 500 ml Kieselgel (0,04 -0,063 mm) gefüllt ist. Es werden 400 ml Fraktionen gesammelt und mittels analytischer HPLC alle Fraktionen auf die cis-isomere Verbindung untersucht.

| | |
|---|---|
| HPLC-analytisch: | Hibar 250-4, Lichrosorb Si 60, 5 µm, 254 nm |
| Laufmittel: | 100 ml Methylenchlorid - 3 ml Methanol |
| Fluß: | 2 ml/min, Konzentration: 1 mg/ml |
| Retention: | 5,80 (Gehalt 73,1%) |
| Ausbeute: | 4,75 g (51% der Theorie) |
| | $C_{31}H_{28}N_2O_4S$ (524,6) |
| NMR ($CDCl_3$): | $\delta$ = 1,4 (dd, 3H); 3,22 (d, 1H); 3,41 (d, 1H); 3,65 (q, 2H); 5,0 (d, 1H); 5,48 - 5,6 (m, 1H); 6,07 (d, 1H); 6,92 (s, 1H); 7,21 - 7,4 (m, 15H) ppm. |

II. 7-Phenylacetamido-3-[(Z)-propen-1-yl]-3-cephem-4-carbonsäurebenzhydrylester (anderes Verfahren)

177,2 g (0,2 mol) 7-Phenylacetamido-3-(triphenylphosphonio)methyl-3-cephem-4-carbonsäurebenzhydrylester iodid werden in 800 ml $CH_2Cl_2$ und 80 ml $CH_3OH$ fast vollständig gelöst, auf +5 °C gekühlt und mit 167,8 ml (3,0 mol) Acetaldehyd versetzt; währenddessen die Temperatur nicht über 20 °C ansteigen sollte.

Danach werden langsam 26,4 g (0,25 mol) Natriumcarbonat gelöst in 200 ml Wasser innerhalb von 15 Min. bei 14 °C zugegeben. Das Eisbad wird danach weggenommen und die Lösung 2 1/2 Stunden bei Raumtemperatur gerührt.

Man kontrolliert den Ablauf der Reaktion mit der Dünnschichtchromatographie in Acetonitril: Wasser = 9:1 und Toluol: Essigester = 8:2. Nach Ablauf der Wittig-Reaktion wird die organische Phase abgetrennt und die wäßrige Phase nochmals mit $CH_2Cl_2$ gewaschen. Die vereinigten $CH_2Cl_2$-Phasen werden über 150 g Kieselgel (Merck, 0,04-0,063 mm) filtriert und mit $CH_2Cl_2$(ca. 1000 ml) solange nachgewaschen, bis das Filtrat farblos ist.

Das $CH_2Cl_2$-Filtrat wird über $Na_2SO_4$ getrocknet, danach zu einem öligen Rückstand eingeengt und mit 800 ml Ethanol verrührt. Die ethanolische Lösung läßt man am Rotationsverdampfer 15 Min. rühren, wobei allmählich Produkt auskristallisiert. Ethanol wird weiter abdestilliert und der entstandene Kristallbrei mit ca. 90 ml Ether/100 ml n-Pentan verrührt, abgesaugt und mit 60 ml n-Pentan nachgewaschen. Das Produkt wird im Vakuum über $P_4O_{10}$ über Nacht getrocknet.

| | |
|---|---|
| Ausbeute: | 41,2 g (39,2 % der Theorie) HPLC-analytisch: Hibar 250-4; Lichrosorb Si 60, 5 µm, 254 nm. |
| Laufmittel: | 850 ml Toluol - 150 ml Essigester |
| Fluß: | 2 ml $Min^{-1}$ |
| Retention: | 4,73 (80,2 %; Z-Isomer) |

4,00 (17,4 %; E-Isomer)

Beispiel 10

I. 7-Amino-3-[(Z)-1-propen-1-yl]-3-cephem-4-carbonsäurebenzhydrylester

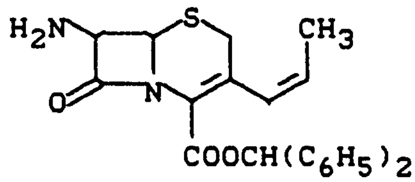

6,4 g (12,2 mmol) 7-Phenylacetamido-3-[(Z)-propen-1-yl]-3-cephem-4-carbonsäurebenzhydrylester (Beispiel 9) werden in 64 ml Methylenchlorid gelöst, mit einem Trockeneisbad auf -40°C gekühlt und nacheinander 2,47 ml (30,5 mmol) Pyridin und 2,54 g (12,2 mmol) Phosphorpentachlorid zugesetzt. Nach 5 Minuten läßt man auf -20°C erwärmen, danach soll die Temperatur innerhalb von 20 Minuten auf -10°C und schließlich auf +10°C ansteigen. Man rührt nunmehr die Lösung 1 Stunde bei +10°C bis +15°C. Anschließend kühlt man den Ansatz auf -40°C, gibt 100 ml Methanol (-30°C) hinzu und rührt noch 30 Minuten bei +10°C. Die Reaktionslösung wird schonend eingeengt, das anfallende Öl in 600 ml Methylenchlorid gelöst, in 800 ml Natriumhydrogencarbonat- Lösung eingerührt und 10 Minuten gerührt. Die Methylenchlorid-Phase wird abgetrennt, einmal mit Wasser gewaschen und über Natriumsulfat getrocknet. Das Methylenchlorid- Filtrat wird an 400 ml Kieselgel (0,04 -0,063 mm) chromatographiert, wobei zunächst mit Methylenchlorid und danach mit Methylenchlorid unter Zusatz von Methanol (Gradient bis 10%) eluiert wird. Das Eluat wird mittels analytischer HPLC und DC (Dünnschichtchromatographie) (Methylenchlorid / Methanol = 100 :1) untersucht.

| | |
|---|---|
| Ausbeute: | 4,2 g |
| | $C_{23}H_{22}N_2O_3S$ (406,5) |
| NMR ($CDCl_3$): | δ = 1,4 (dd, J = 2 Hz und 7 Hz, 3H); 3,3 (d, J = 17 Hz, 1H); 3,48 (d, J = 17 Hz, 1H); 4,75 (d, J = 4,5 Hz, 1H); 4,98 (d, J = 4,5 Hz, 1H); 5,45 - 5,55 (d und q, J = 10 Hz und 7 Hz, 1H); 6,07 (d, J = 11 Hz, 1H); 6,96 (s, 1H); 7,23 - 7,42 (m, 10H); 8,6 (d, 2H) ppm. |
| HPLC-analytisch: | Hibar 250-4, Merck, Lichrosorb Si 60, 5 µm, 254 nm |
| Laufmittel: | 1000 ml Methylenchlorid - 5 ml Methanol |
| Fluß: | 2 ml/min, Konzentration 1 mg/ml |
| Retention: | 12,75 (Gehalt: 70,4%) |

II. 7-Amino-3-[(Z)-1-propen-1-yl)]-3-cephem-4-carbonsäurebenzhydrylester-hydrochlorid (anderes Verfahren)

187,05 g (0,8976 mol; 1,57 equiv.) Phosphorpentachlorid werden bei Raumtemperatur in 3300 ml $CH_2Cl_2$ in einem 4 l Dreihalskolben vorgelegt und innerhalb von 5-10 Min. 66,42 ml (0,821 mol; 1,44 equiv.) Pyridin gelöst in 330 ml $CH_2Cl_2$ hinzugetropft, wobei die Temperatur auf 24-27°C ansteigt und eine klare, farblose Lösung entsteht. Die Lösung wird auf -2°C gekühlt und 300 g (0,572 mol) 7-Phenylacetamido-3-[-(Z)-propen-1-yl]-3-cephem-4-carbonsäurebenzhydrylester hinzugefügt, wobei die Temperatur nicht über +2°C ansteigen darf. Danach entfernt man das Kühlbad und rührt 40 Min., wobei die Temperatur der Reaktionslösung bis auf 10-12°C ansteigt (Iminochlorid-Lösung).

In einen 6 l Dreihalskolben werden 255 ml (2,843 mol; 4,99 equiv.) 1,3-Butandiol gelöst in 1650 ml $CH_2Cl_2$ auf -20°C bis -25°C gekühlt (Aceton/Trockeneis) und die Iminochlorid-Lösung mittels Vakuum innerhalb von 5-10 Min. darin eingeleitet. Die Temperatur darf dabei nicht über -20°C ansteigen. Anschließend wird das Kältebad entfernt und die Reaktionslösung 2 Stunden gerührt, wobei die Temperatur auf 10°C ansteigt.

Nun wird die Reaktionslösung mit 1200 ml Eiswasser, mit 1200 ml 2N HCl und 1200 ml gesättigter Kochsalzlösung gewaschen. Die $CH_2Cl_2$-Phase wird kurz über $Na_2SO_4$ getrocknet, vom Trockenmittel abgetrennt und das Filtrat bis zur Trockene eingeengt. Das ausgefallene kristalline Material wird mit 1200 ml bis 2000 ml Essigester verrührt, anschließend abgesaugt und mit Ether nachgewaschen. Das Produkt

wird über Nacht im Hochvakuum getrocknet:

Ausbeute: 173,9 g (68,7 % der Theorie)
$C_{23}H_{23}ClN_2O_3S$(442,96)

NMR (DMSO): δ = 1,48 (dd,3H); 3,56 (d,1H); 3,79 (d,1H); 5,2 (d,1H); 5,31 (d,1H); 5,56-5,72 (m,1H); 6,21 (schwaches d, 1H); 6,28 (schwaches d,1H); 6,91 (s,1H); 7,25-7,47 (m,10H)ppm.

Das Produkt enthält neben dem Z-Isomer etwas E-Isomer (Z/E = 91:9).

Beispiel 11

D-7-[2-(t-Butyloxycarbonylamino)-2-(2-aminobenzothiazol-6-yl)glycylamido]-3-[(Z)-1-propen-1-yl]-3-cephem-4-carbonsäurebenzhydrylester

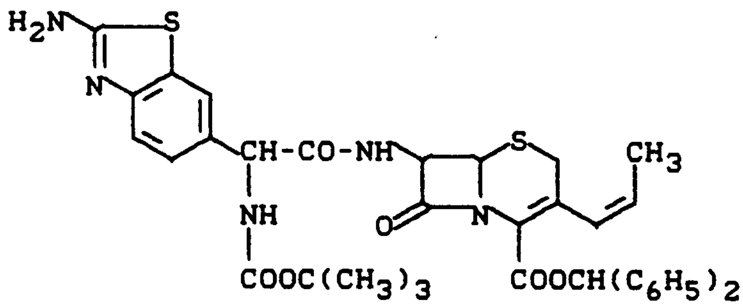

3,4 g (10,5 mmol) D-α-t-Butyloxycarbonylamino-α-(2-aminobenzothiazol-6-yl)essigsäure und 4,0 g (7 mmol) 7-Amino-3-[(Z)-1-propen-1-yl]-3-cephem-4-carbonsäurebenzhydrylester (Beispiel 10) werden in 100 ml Tetrahydrofuran gelöst. Die klare gelbe Lösung wird mit 2,2 g (10,5 mmol) DCC versetzt und dann 2 Stunden bei Raumtemperatur gerührt. Nach zweistündigem Rühren engt man die Mischung zur Trockene ein, der Rückstand wird in 150 ml Essigester suspendiert, 10 Minuten magnetisch gerührt und die unlöslichen Bestandteile durch Absaugen abgetrennt. Das Essigesterfiltrat wird zur Trockne eingeengt, der Rückstand in 50 ml Methylenchlorid gelöst und an 200 ml Kieselgel (0,04 - 0,063 mm) chromatographiert. Die Elution erfolgt mit Methylenchlorid und Methylenchlorid / Methanol -Gemischen in folgender Reihenfolge:

1.) Methylenchlorid (Fraktion 1 bis 6): 200 mg, verworfen
2.) Methylenchlorid - 2% Methanol (Fraktion 7, 8): 200 mg, verworfen
3.) Methylenchlorid - 3% bis 4% Methanol (Fraktion 9, 10)
4.) Methylenchlorid - 4% bis 5% Methanol (Fraktion 11 bis 13) Ausbeute der Fraktion 9 bis 13 : 3,1 g
5.) Methylenchlorid - 5% bis 10% Methanol (Fraktion 14,15): 1,9 g
6.) Methylenchlorid - Methanol (1:1, Fraktion 16): 0,7 g, verworfen

Gemäß analytischer HPLC-Untersuchung enthalten die Fraktionen 9 bis 13 die gewünschte Verbindung.

Ausbeute: 3,1 g (44% der Theorie)
$C_{37}H_{37}N_5O_6S_2$ (711,9)

NMR ($CDCl_3$): δ = 1,35 (dd, 3H); 1,43 (s, 9H); 3,15 (d, 1H); 3,31 (d, 1H); 4,97 (d, 1H); 5,3 (s, 1H); 5,46 - 5,55 (m, 1H); 5,71 (breites s, 2H); 5,78 - 5,85 (q, 1H); 6,03 (breites d, J = 11 Hz, 1H); 6,87 (s, 1H); 7,2 - 7,4 (mm, 12H); 7,5 (s, 1H) ppm.

Beispiel 12

D-7-[(2-Aminobenzothiazol-6-yl)glycylamido]-3-[(Z)-1-propen-1-yl]-3-cephem-4-carbonsäure-trifluoracetat

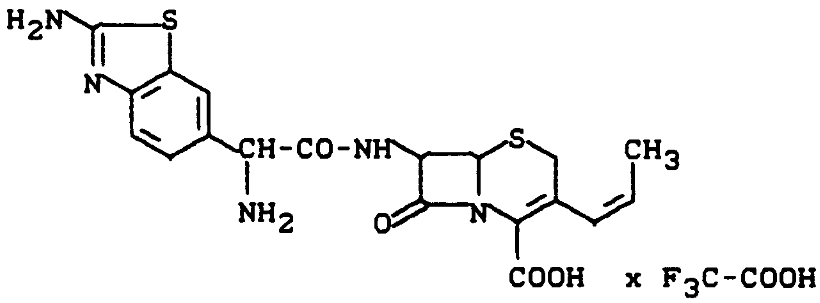

3,1 g (4,35 mmol) D-7-[2-(t-Butyloxycarbonylamino)-2-(2-aminobenzothiazol-6-yl)glycylamido]-3-[(Z) -1-propen-1-yl]-3-cephem-4-carbonsäurediphenylmethylester (Beispiel 11) werden in 20 ml Methylenchlorid gelöst, mit 50 ml Trifluoressigsäure und 5 ml Anisol versetzt und 60 Minuten bei Raumtemperatur gerührt. Man engt die Mischung im Vakuum ein, gibt zu dem Konzentrat 200 ml Ether, filtriert den ausgefallenen Feststoff ab und wäscht mit 100 ml Ether nach. Das hellgelbe Trifluoracetat wird im Vakuum getrocknet.

| | |
|---|---|
| Ausbeute: | 2,7 g |
| | $C_{21}H_{21}F_3N_5O_6S_2$ (560,6) |
| HPLC-analytisch: | Hibar 250-4, Merck RP-8, 10 µm, 254 nm |
| Laufmittel: | 250 ml $CH_3CN$ - 75 ml Eisessig - 2175 ml Wasser |
| Fluß: | 4 ml/min, Konzentration: 1 mg/ml |
| Retention | 3,33 (Gehalt: 76,4%) |

Beispiel 13

D-7-[(2-Aminobenzothiazol-6-yl)glycylamido]-3-[(Z)-1-propen-1-yl]-3-cephem-4-carbonsäure

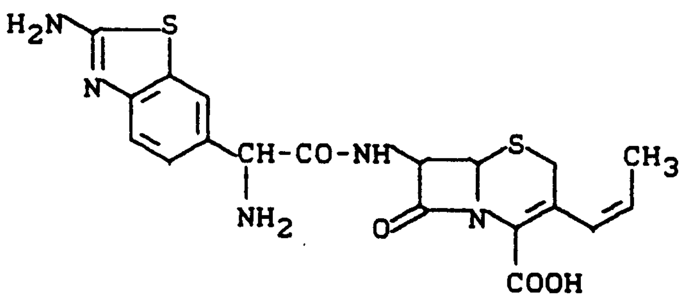

2,6 g (4,64 mmol) D-7-[(2-Aminobenzothiazol-6-yl)glycylamido]-3-[(Z)-1-propen-1-yl]-3-cephem-4-carbonsäure-trifluoracetat (Beispiel 12) werden in 100 ml Wasser suspendiert, von gelben unlöslichen Partikelchen über Kieselgur abgetrennt und mit 30 ml Wasser nachgewaschen.

Die noch leicht getrübte Lösung wird über ein Membranfilter (Millipore, 0,45 µm) filtriert und aus dem Filtrat analog Beispiel 6 mittels präparativer HPLC das 3-Propenylcephalosporin isoliert.

| | |
|---|---|
| Ausbeute: | 500 mg |
| | $C_{19}H_{19}N_5O_4S_2$ (445,5) |
| NMR (DCOOD): | $\delta$ = 1,67 (dd, 3H); 3,41 (d, 1H); 3,55 (d, 1H); 5,3 (d, 1H); 5,78 (s, 1H); 5,81 - 5,91 (q und m, 2H); 6,25 (breites d, J = 11,6 Hz, 1H); 7,81 - 7,9 (q, 2H); 8,18 (s, 1H) ppm. |

Beispiel 14

Natriumsalz der D-α-[(1-Methyl-2-methoxycarbonyl-vinyl)-amino]-(2-aminobenzothiazol-6-yl)essigsäure

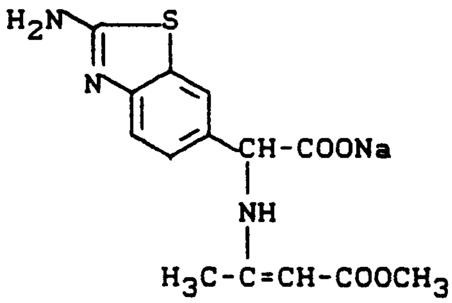

100 g [entspricht 97,2 g (0,435 mol)] D-2-Aminobenzothiazol-6-yl)glycin (Gehalt = 97,2%, ee = 89,7%) werden in methanolische Natronlauge, hergestellt aus 18,6 g (0,465 mol, d.h. 7% Überschuß) NaOH und 1000 ml Methanol, eingetragen. Unter Rückflußkochen und Rühren bildet sich eine klare Lösung, die mit 64 ml (0,59 mol, ca. 40% Überschuß) Acetessigsäuremethylester, gelöst in 100 ml Methanol, innerhalb von 40 min versetzt wird (nach beendetem Zutropfen pH = 10,3; Probe / Wasser = 1:1). Anschließend wird die Lösung 1 h unter Rückfluß erhitzt und danach mehrere Stunden ohne Heizung weiter gerührt. Das auskristallisierte Material wird abgesaugt und mit Toluol (zweimal 200 ml) gewaschen. Der Filtratrückstand wird in 1000 ml Toluol 30 - 40 min zum Sieden erhitzt, anschließend 300 ml Toluol abdestilliert und durch Zutropfen von frischem Toluol gewaschen und über Nacht im Frischluftschrank bei 70 °C getrocknet.

Ausbeute: 81,9 g (52,1% der Theorie)

| $C_{14}H_{14}N_3O_4SNa \cdot H_2O$ (361,4) | | | | | | | |
|---|---|---|---|---|---|---|---|
| Ber. | C 46,53 | H 4,46 | N 11,62 | S 8,87 | Na 6,36 | | |
| Gef. | C 46,2 | H 4,4 | N 11,9 | S 8,4 | Na 5,6 | Br 0,1 | Cl 0,5 |

NMR (DMSO): δ = 1,63 (s, 3H); 3,49 (s, 3H); 4,25 (s, 1H); 4,7 (d, J = 7,5 Hz, 1H); 7,12 (dd, 1H); 7,21 (d, 1H); 7,34 (s, 2H); 7,48 (s, 1H); 9,55 (d, 1H) ppm.

ee = 100%

Beispiel 15

7-Amino-3-[(Z)-1-propen-1-yl]-3-cephem-4-carbonsäure (7-APCA)

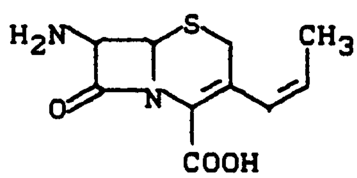

Zu einer gerührten Lösung von 500 ml Trifluoressigsäure (TFA) und 31 ml Anisol, die auf 0 °C gekühlt wird, gibt man 54,5 g (0,123 mol) 7-Amino-3-[(Z)-1-propen-1-yl]-3-cephem-4-carbonsäurebenzhydrylester-hydrochlorid (Beispiel 10). Die Mischung wird 1 Stunde bei Raumtemperatur gerührt, anschließend bei 30 °C im Vakuum eingeengt und der ölige Rückstand mit 600 ml Ether 1 Stunde verrührt. Der Niederschlag wird abgesaugt, mit 300 bis 400 ml Ether gewaschen und der Filterrückstand 3 Stunden im Vakuum getrocknet. Das Trifluoracetat wird in 300 ml Wasser suspendiert, die Aufschlämmung auf +5 °C gekühlt und mit 12 N HCl der pH-Wert auf 0,2 - 0,4 gestellt. Die entstandene klare Lösung wird auf +5 °C gekühlt und mit 4 g Aktivkohle 10 min gerührt. Man saugt über Kieselgur ab und wäscht mit ca. 50 ml 0,1 N HCl nach. Das Filtrat wird bei +5 °C mit 20%iger NaOH bis pH 2,1 eingestellt und das ausgefallene Produkt 1 Stunde im

Kühlschrank stehen gelassen, um die Kristallisation zu vervollständigen. Der Kristallbrei wird abgesaugt, mit 100 ml Wasser und 300 ml Aceton gewaschen und im Vakuum getrocknet.

| | |
|---|---|
| Ausbeute: | 16,4 g (55,4% der Theorie) |
| | $C_{10}H_{12}N_2O_3S$ (240,3) |
| NMR (DCOOD): | δ = 1,8 (dd, 3H); 3,61 (d, 1H); 3,77 (d, 1H); 5,39 (d, 1H); 5,52 (d, 1H); 5,91 - 6,06 (q, 1H); 6,5 (d, 1H) ppm. |
| HPLC-analytisch: | Hibar 250-4, Merck RP-8, 10 µm, 254 nm |
| Laufmittel: | 100 ml $CH_3CN$ - 30 ml Eisessig - 870 ml Wasser |
| Fluß: | 2 ml/min, Konzentration: 0,25 mg/ml |
| Retention: | 2,39 (85,7%; Z-Isomer) |
| | 3,16 (11,3%; E-Isomer) |

Beispiel 16

I. D-7-[(2-Aminobenzothiazol-6-yl)glycyl-amido]-3-[(Z)-1 propen-1-yl]-3-cephem-4-carbonsäure

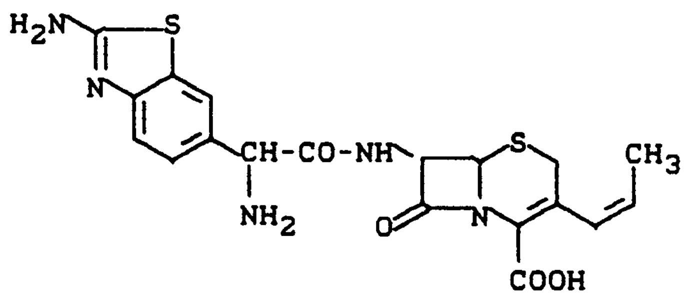

a) Aktivierung der Precursorsäure:

12,09 g [entspricht 11,48 g (33,4 mmol)] Natrium-D-α-[(1-Methyl-2-methoxycarbonyl-vinyl)-amino]-(2-aminobenzothiazol-6-yl)acetat(Gehalt = 95%, Beispiel 14) werden in 57 ml Dimethylformamid gelöst und danach 23 ml Acetonitril hinzugegeben. Man kühlt die Lösung auf -70 °C und gibt hintereinander 115 µl 3-Dimethylaminopropanol und 3,30 ml (34,4 mmol) Chlorameisensäureethylester hinzu und rührt 20 min bei -70 °C.

b) Zubereitung der Cephalosporinkomponente (7-APCA)

9,61 g (40 mmol) 7-Amino-3-[(Z)-1-propen-1-yl]-3-cephem-4-carbonsäure (Beispiel 15) werden in 57 ml Dimethylformamid und 23 ml Acetonitril suspendiert und durch Zugabe von 1 N Natronlauge (36,8 ml) bei Raumtemperatur bis pH 8,5 in eine klare Lösung verwandelt. Man kühlt die Lösung auf -20 °C bis -30 °C ab.

c) Kupplung, Deblockierung und Isolierung des Rohbetains

Die gekühlte 7-APCA-Lösung (-20 °C bis -30 °C) nach b) läßt man langsam zur Lösung des gemischten Anhydrids der Precursorsäure nach a) bei -70 °C zutropfen und rührt 10 min bei -70 °C nach. Anschließend läßt man die Temperatur der Lösung innerhalb von 45 min auf 0 °C kommen (ohne Kühlung) und rührt mit 1,2 g Aktivkohle und 1,2 g Kieselgur noch weitere 10 min. Man filtriert die Reaktionsmischung über ein Seitz-Filter, wäscht mit wenig Dimethylformamid nach und versetzt das Filtrat mit 6,9 ml konzentrierter Salzsäure. Das Volumen der Lösung wird bis auf 115 ml konzentriert, wobei ausgefallene Salze abgetrennt werden. Das Filtrat wird über magnetischem Rühren mit 25% $NH_3$-Lösung auf pH 4,0 gestellt und mit 800 ml Aceton versetzt, wobei das Rohbetain ausfällt. Man rührt die Ausfällung 10 min, saugt ab und wäscht mit Aceton nach und trocknet das Material im Vakuum.

| | |
|---|---|
| Ausbeute: | 12,65 g |
| HPLC-analytisch: | Hibar 250-4, Merck RP-8, 10 µm, 254 nm |
| Laufmittel: | 100 ml $CH_3CN$ - 30 ml Eisessig - 870 ml Wasser |
| Fluß: | 2 ml/min; Konzentration 1 mg/ml |
| Retention: | 7.06 (73,8%; Z-Isomer) |
| | 11,18 (11,5%; E-Isomer) |

Das Rohbetain wird in Wasser suspendiert, mit halbkonzentrierter Salzsäre bei pH 1,2 in Lösung gebracht und 15 min mit 1,2 g A-Kohle gerührt. Die Mischung wird über ein Kieselgur-Bett abgesaugt, mit 20 ml 0,1 N Salzsäure nachgewaschen und das Filtrat auf eine RP 18-Säule (Hibar 250-25, Merck) gepumpt. Die

Säule wird zunächst mit Wasser, danach mit 5%igem Methanol eluiert. Die Fraktionen werden mittels analytischer HPLC untersucht und die Fraktionen, die das Z-isomere Derivat enthalten, vereinigt, Methanol im Vakuum abdestilliert und die wäßrige Lösung lyophilisiert.

Ausbeute: 5,2 g (32,3% der Theorie)

| $C_{19}H_{19}N_5O_4S_2 \cdot 2H_2O$ (481,56) | | | | |
|---|---|---|---|---|
| Ber. | C 47,39 | H 4,81 | N 14,54 | S 13,22 |
| Gef. | C 47,7 | H 4,8 | N 14,3 | S 13,0 |

II. D-7-[(2-Aminobenzothiazol-6-yl)glycyl-amido]-3-[(Z)-1-propen-1-yl]-3-cephem-4-carbonsäure (anderes Verfahren)

a) Aktivierung der Precursorsäure:

100 g [entspricht 95 g (0,277 mol) Reinmaterial; 1,2 equiv.] Natrium-D-α-[(1-Methyl-2-methoxycarbonylvinyl)-amino]-(2-aminobenzothiazol-6-yl)acetat (Gehalt = 95 %, Beispiel 14) werden in 500 ml Dimethylformamid und 200 ml Acetonitril klar gelöst. Man kühlt die Lösung auf -60 °C und gibt nacheinander 1 ml 3-Dimethylamino-1-propanol und 27,7 ml (0,281 mol) Chlorameisensäureethylester hinzu und rührt 30 Min. bei -60 °C.

b) Zubereitung der Cephalosporinkomponente:

102 g (0,2304 mol) 7-Amino-3-[(Z)-1-propen-1-yl]-3-cephem-4-carbonsäurebenzhydrylesterhydrochlorid (Beispiel 10/II) werden in 500 ml Dimethylformamid und 100 ml Acetonitril klar gelöst, bei Raumtemperatur mit 31 ml Wasser und 32,2 ml (0,2289 mol) Triethylamin versetzt und 5 Min. bei Eiskühlung gerührt.

c) Kupplung:

Die auf 0 °C gekühlte Cephalosporin-Lösung (b) gibt man langsam zur Lösung des gemischten Anhydrids (a) bei -60 °C hinzu, wobei die Temperatur von -60 °C auf -30 °C ansteigt. Man rührt insgesamt 30 Min. nach und läßt die Temperatur der Reaktionslösung auf 0 °C kommen. Anschließend gibt man 57 ml konzentrierte Salzsäure hinzu und rührt die Lösung 15 Min. bei 0 °C.

d) Isolierung des Trifluoracetatsalzes:

Man destilliert aus der Reaktionslösung Acetonitril ab und stellt mit 25 % $NH_3$-Lösung unter Eiskühlung auf pH 7,5. Man schüttet die Lösung in 5 l Essigester und 3 l 10 %ige $NaHCO_3$-Lösung, die Kochsalz enthält. Die Mischung wird 5 Min. intensiv gerührt und anschließend über ein Seitz-Filter abgesaugt. Die Essigester-Phase wird abgetrennt, einmal mit 4 l gesättigter $NaHCO_3$-Lösung und zweimal mit 4 l Wasser gewaschen. Danach wird die Essigesterphase über $Na_2SO_4$ getrocknet, vom Trockenmittel abgesaugt und zum Schluß im Vakuum zur Trockne eingeengt. Der entstandene Hartschaum wird 30 Min. im Hochvakuum getrocknet. Ausbeute: 170 g.

e) Deblockierung:

Der Hartschaum wird in 1600 ml $CH_2Cl_2$ gelöst, auf 0 °C gekühlt und mit einer Mischung von 750 ml Trifluoressigsäure und 4 ml Anisol versetzt. Die Lösung wird danach 45 Min. bei Raumtemperatur gerührt, anschließend zu einem Öl eingeengt und der ölige Rückstand mit 6 l Ether digeriert. Das auskristallisierte Material wird abgesaugt, mit Ether gewaschen und über Nacht im Vakuum getrocknet.

Ausbeute: 125 g (97 % der Theorie)
$C_{19}H_{19}N_5O_4S_2 \cdot CF_3COOH$ (559,55) HPLC-analytisch: Hibar 250-4, Merck RP-8, 254 nm
Laufmittel: 100 ml $CH_3CN$ - 30 ml Eisessig - 870 ml Wasser
Fluß: 4 ml/Min.
Retention: 2,25 (90,1 %; Z-Isomer)
3,77 (8,3 %; E-Isomer).

f) Reindarstellung mittels Adsorberharzchromatographie:

Man suspendiert 141 g D-7-[(2-Aminobenzothiazol-6-yl)glycyl-amido]-3-[(Z)-1-propen-1-yl]-3-cephem-4-carbonsäure-trifluoracetat [feuchtes Material = 128,8 g (100 % der Theorie)] in 1000 ml Wasser, rührt 15 Min. intensiv, saugt danach unlösliches Material ab und wäscht mit Wasser nach. Das Filtrat (pH 1,3) wird auf eine Säule gegeben, die mit 8 l Adsorberharz LGP 4429 (Lewatit OC 1062, BAYER AG) gefüllt ist. Man eluiert die Säule zunächst mit 5 l Wasser und anschließend mit jeweils 2 l-Portionen Wasser, zu denen man Aceton mit einem steigenden Anteil von 2 % bis 10 % hinzufügt. Man sammelt insgesamt 15 Fraktionen mit einem Volumen von jeweils 2000 ml:

| Fraktion (a=2000 ml) | Ausbeute (g) |
|---|---|
| 1 | 3,5 |
| 2 | 2,1 |
| 3/4 | 9,5 |
| 5/6 | 16,7 |
| 7/8 | 13,2 |
| 9/10 | 8,3 |
| 11 | 5,1 |
| 12 | 5,3 |
| 13 | 3,7 |
| 14 | 2,3 |
| 15 | 1,0 |

Die Fraktionen von 3 bis 10, die das gewünschte Produkt in hochreiner Form enthalten, werden im Vakuum von Aceton abdestilliert und lypholisiert.

Ausbeute: 47,7 g (43,0 % der Theorie) $C_{19}H_{19}N_5O_4S_2 \cdot 2H_2O$ (481,56)

g) Methanol-Solbatbildung:

168,3 g D-7-[2-Aminobenzothiazol-6-yl)-glycylamido]-3-cephem-4-carbonsäure als Lyophilisat (f) werden in 1700 ml Methanol 90 Min. gerührt, abgesaugt und mit 500 ml Methanol auf der Nutsche nachgewaschen. Der Nutschenrückstand wird erneut in 1000 ml Methanol 45 Min. verrührt, abgesaugt und mit 500 ml Methanol auf der Nutsche nachgewaschen. Das Produkt wird über Nacht im Hochvakuum getrocknet.

Ausbeute: 110,2 g (65,5 % der Theorie) HPLC-ana
lytisch: Hibar 250-4, Merck RP-8, 10 µm, 254 nm
Laufmittel: 100 ml $CH_3CN$ - 30 ml Eisessig - 870 ml Wasser
Fluß: 4 ml $Min^{-1}$; Konzentration: 1 mg $ml^{-1}$
Retention: 3,58 (98,4 %; Z-Isomer)
6,54 (0,79 %; E-Isomer)

h) Hydratbildung:

109,6 g D-7-[2-Aminobenzothiazol-6-yl)glycyl-amido]-3-[(Z)-1-propen-1-yl-3-cephem-4-carbonsäure-methanolsolvat (g) werden in 1100 ml Wasser (gewonnen aus Milli-Q Watersystem, Millipore GmbH) unter Rühren eingetragen und unter Vakuum 2 Stunden magnetisch gerührt. Das Produkt wird abgesaugt und dreimal mit etwa gleich großen Portionen Wasser (aus Milli-Q-Watersystem) gewaschen. Die Substanz wird 36 Stunden im Hochvakuum ohne Trockenmittel getrocknet.

Ausbeute: 94,7 g (86,4 %)

| $C_{19}H_{19}N_5O_4S_2 \cdot 2H_2O$ (463,541) | | | | |
|---|---|---|---|---|
| Ber. | C 49,23 | H 4,57 | N 15,11 | S 13,83 |
| Gef. | C 48,8 | H 5,1 | N 14,9 | S 13,4 |

HPLC-analytisch: Hibar 250-4, Merck RP-8, 10 µm, 254 nm
Laufmittel: 100 ml $CH_3CN$ - 30 ml Eisessig - 870 ml Wasser
Fluß: 4 ml $Min^{-1}$ ; Konzentration: 1 mg $ml^{-1}$
Retention: 3,56 (98,8 %).

Beispiel 17

Natrium-D-7-[(2-Aminobenzothiazol-6-yl)glycol-amido]-3-[(Z)-1-propen-1-yl]-3-cephem-4-carboxylat

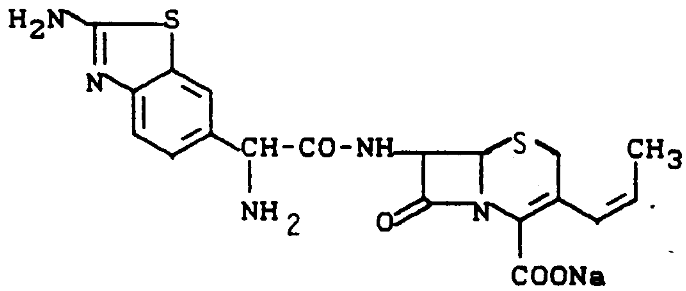

15,0 g (0,0324 mol) D-7-[(2-Aminobenzothiazol-6-yl)glycyl-amido]-3-[(Z)-1-propen-1-yl]-3-cephem-4-carbonsäure (Beispiel 16/II) werden in 300 ml Wasser unter Rühren suspendiert und mit 1 N Natronlauge auf pH = 8,56 unter pH-Stat-Bedingungen gestellt (Memo-Titrator DL 40 RC). Die entstandene hellgelbe Lösung wird über ein Papierfilter abgesaugt und das Filtrat lyophilisiert.

Ausbeute: 15,0 g (92,1 % der Theorie)

| $C_{19}H_{18}N_5NaO_4S_2 \cdot 2H_2O$ (503,54) | | | | | |
|---|---|---|---|---|---|
| Ber. | C 45,32 | H 4,40 | N 13,91 | S 12,74 | Na 4,56 |
| Gef. | C 46,0, | H 4,9 | N 14,0 | S 12,9 | Na 4,3 |

NMR (DCOOD): δ = 1,64 (dd, 3H); 3,38 (d,1H); 3,51 (d,1H; 5,26 (d, 1H); 5,72 (s, 1H); 5,78-5,85 (m, 1H); 5,85 (d, 1H); 6,21 (d, 1H); 7,76-7,83 (q, 2H); 8,12 (s, 1H) ppm.
HPLC-analytisch: Hibar 250-4, RP-8, 10 µm, 254 nm
Laufmittel: 100 ml $CH_3CN$ - 30 ml Essigsäure - 870 ml Wasser
Fluß: 2 ml/min,
Konzentration: 1 mg/ml
Retention: 4,36 (Gehalt: 98,7 %)

Beispiel 18

D-α-t-Butoxycarbonylamino-α-(benzothiazol-6-yl)essigsäure

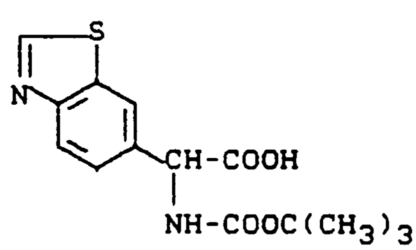

Zu einer Lösung von 20 g (0,0618 mol) D-α-t-Butyloxycarbonylamino-α-(2-aminobenzothiazol-6-yl)-essigsäure in 160 ml Tetrahydrofuran läßt man 12,2 ml (0,102 mol) tert.-Butylnitrit gelöst in 25 ml Tetrahydrofuran innerhalb von 30 Minuten bei +50 °C bis +60 °C zutropfen. Anschließend rührt man 30 Minuten bei +50 °C bis +55 °C nach, destilliert das Lösemittel ab und verteilt den Rückstand in 300 ml Wasser und 300 ml Essigester. Die Mischung wird bei Eiskühlung mit 2 N HCl auf pH 1,5 angesäuert, 5 Minuten gerührt und anschließend mit 40%iger Kaliumcarbonat-Lösung auf pH 8,0 bis 8,5 gestellt. Die wäßrige Phase wird abgetrennt, nochmals mit Essigester gewaschen und danach bei 0 °C mit 2 N HCl auf pH 2,5 angesäuert. Die saure Lösung wird zweimal mit Essigester extrahiert, mit Kochsalzlösung gewaschen und über Natriumsulfat getrocknet. Die Essigesterphase wird bis auf 50 ml eingeengt und in

Petrolether eingerührt.

Ausbeute: 14,4 g (76% der Theorie)

| $C_{14}H_{16}N_2O_4S$ (308,4) | | | | |
|---|---|---|---|---|
| Ber. | C 54,5 | H 5,2 | N 9,1 | S 10,4 |
| Gef. | C 54,1 | H 5,6 | N 8,9 | S 9,7 |

$$[\alpha]^{20}_{589} = -130,3^\circ C \ (c = 1, \text{Methanol})$$

NMR (DMSO): δ = 1,43 (s, 9H); 5,31 (d, 1H); 7,6 (dd, 1H); 7,73 (d, 1H); 8,08 (d, 1H); 8,2 (schwaches d, 1H); 9,4 (s, 1H) ppm.

Beispiel 19

7-Phenylacetamido-3-[(Z)-propen-1-yl]-3-cephem-4-carbonsäure-p-methoxybenzylester

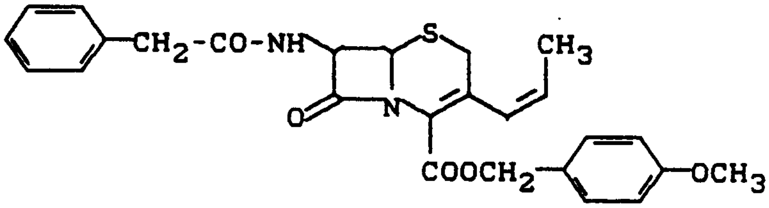

40,0 g (82,1 mmol) 7-Phenylacetamido-3-chlormethyl-3-cephem-4-carbonsäure-p-methoxybenzylester und 22,66 g (86,4 mmol) Triphenylphosphin werden in 300 ml Dimethylformamid gelöst, mit 12,95 g (86,4 mmol) Natriumiodid versetzt und 2 Stunden bei Raumtemperatur gerührt. Die Reaktionslösung wird anschließend im Hochvakuum zur Trockne bis zu einem Öl eingeengt.

Der ölige Rückstand von 4,2 g wird in 130 ml Methylenchlorid aufgenommen (keine vollständige klare Lösung), in einem 500 ml Dreihalskolben mit 69,0 ml (1231,5 mmol) Acetaldehyd versetzt und anschließend mit 1 N Natronlauge unter pH-stat Bedingungen mittels Autotitrator bei pH 8,1 behandelt. Nach 1 Stunde sind 8,7 ml 1 N Natronlauge verbraucht. Anschließend werden bei pH 8,3 innerhalb von 20 Stunden noch 72,5 ml 1 N Natronlauge verbraucht. Die wäßrige Phase wird abgetrennt, die Methylenchlorid- Phase zweimal mit Wasser gewaschen und über Natriumsulfat getrocknet. Anschließend wird die Methylenchlorid-Phase erneut mit 20 ml (358 mmol) Acetaldehyd 2 Stunden bei Raumtemperatur gerührt, Methylenchlorid abdestilliert und das zurückbleibende Öl in Toluol aufgenommen und auf eine Säule mit 1 l Kieselgel (0,04 - 0,063 mm) gegeben.

Die Elution erfolgt zunächst mit Toluol (Fraktion 1 bis 5) und anschließend mit Toluol / Essigester (5:1, Fraktion 6, 7), wobei 600 ml Fraktionen gesammelt werden. Die Fraktionen 3 bis 6 werden vereinigt, bis zur Trockene eingeengt und das erhaltene Öl mit 100 bis 150 ml Ether verrieben. Das ausgefällte weiße Material wird abgesaugt und mit Ether (50 bis 80 ml) gewaschen.

Ausbeute: 12,1 g (31% der Theorie)

$C_{26}H_{26}N_2O_5S$ (478,6)

NMR ($CDCl_3$): δ = 1,52 (d, 3H); 3,23 (d, 1H); 3,41 (d, 1H); 3,61 (q, 2H); 3,78 (s, 3H); 4,95 (d, 1H); 5,13 (s, 2H); 5,59 - 5,69 (dq, 1H); 5,75 - 5,81 (q, 1H); 6,08 (breites d, 1H); 6,45 (d, 1H); 6,87 (d, 2H); 7,25 - 7,36 (m, 7H) ppm.

Die Fraktionen 7 bis 11 werden bis zu einem leicht rötlichen Öl eingeengt, das nicht der gewünschten Verbindung zugeordnet werden kann.

Beispiel 20

7-Amino-3-[(Z)-1-propen-1-yl]-3-cephem-4-carbonsäure-p-methoxybenzylester

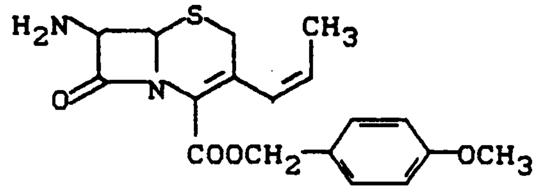

12,1 g (25,28 mmol) 7-Phenylacetamido-3-[(Z)-propen-1-yl]-3-cephem-4-carbonsäure-p-methoxybenzylester (Beispiel 15) werden in 133 ml Methylenchlorid gelöst, auf -50 °C gekühlt und nacheinander 5,11 ml (63,2 mmol) Pyridin und 5,26 g (25,28 mmol) Phosphorpentachlorid zugesetzt. Danach läßt man die Temperatur innerhalb von 25 Minuten auf - 10 °C ansteigen. Nach weiteren 20 Minuten beträgt die Temperatur der Lösung 0 °C; danach rührt man noch 45 Minuten bis +15 °C. Man kühlt den Ansatz jetzt auf -50 °C, gibt in einem Guß 200 ml Methanol (ca. -30 °C) hinzu und rührt ohne Kühlung 30 Minuten. Die Reaktionslösung wird eingeengt, der ölige Rückstand in Methylenchlorid gelöst und auf eine Säule gegeben, die mit 400 ml Kieselgel (0,04 - 0,063 mm) gepackt ist. Man eluiert die Säule zunächst mit Methylenchlorid, danach hintereinander mit den Lösemittelgemischen Methylenchlorid - 5% Methanol und Methylenchlorid - 10% Methanol. Die Fraktionen, die mit dem Gemisch Methylenchlorid - 10% Methanol eluiert werden, engt man zur Trockene ein.

Ausbeute: 10 g

Das Öl wird in 500 ml Ethanol aufgenommen, von einem unlöslichen Schleim durch dekantierendes Filtrieren abgetrennt, das Filtrat zur Trockene eingeengt und das Öl im Vakuum getrocknet.

Ausbeute: 6,8 g (75% der Theorie)
$C_{18}H_{20}N_2O_4S$ (360,4)

NMR ($CDCl_3$): $\delta$ = 1,55 (dd, 3H); 1,95 (schwaches dd, 2H); 3,3 (d, 1H); 3,5 (d, 1H); 3,76 (s, 3H); 4,72 (d, 1H); 4,98 (d, 1H); 5,18 (s, 1H); 5,58 - 5,69 (dq, 1H); 6,1 (breites d, 1H); 6,88 (d, 2H); 7,31 (d, 2H); 8,61 (d, 1H) ppm.

Beispiel 21

D-7-[2-(t-Butyloxycarbonylamino)-2-(benzothiazol-6-yl)-glycylamido]-3-[(Z)-1-propen-1-yl]-3-cephem-4-carbonsäure-p-methoxybenzylester

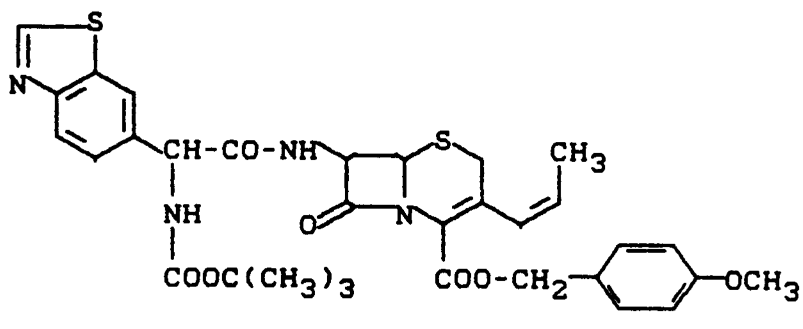

Zu einer Lösung von 5,83 g (18,9 mmol) D-α-t-Butyloxycarbonylamino-α-(benzothiazol-6-yl)essigsäure und 6,8 g (15,1 mmol) 7-Amino-3-[(Z)-1-propen-1-yl]-3-cephem-4-carbonsäure-p-methoxybenzylester (Beispiel 19) in 150 ml Tetrahydrofurn gibt man unter magnetischem Rühren 3,90 g (18,9 mmol) Dicyclohexylcarbodiimid bei Raumtemperatur hinzu und rührt anschließend 2 Stunden. Man saugt den gebildeten Dicyclohexylharnstoff ab, wäscht mit Tetrahydrofuran und engt die Mutterlauge bis zur Trockene ein. Das zurückbleibende Öl wird in 100 ml Methylenchlorid gelöst und an 600 ml Kieselgel (0,04 - 0,063 mm) chromatographiert, wobei man zunächst mit Methylenchlorid (2 x 300 ml) und anschließend mit 5%igem Methanol in

Methylenchlorid unter Kontrolle mittels Dünnschichtchromatographie (Methylenchlorid : Methanol = 100:5) eluiert. Es werden jeweils 300 ml Fraktionen gesammelt. Die gewünschten Fraktionen des Eluates (11 und 12) mit 5%igem Methanol werden vereinigt und zur Trockene eingeengt.

| | |
|---|---|
| Ausbeute: | 8,8 g (89% der Theorie) |
| | $C_{32}H_{34}N_4O_7S_2$ (650,8) |
| NMR ($CDCl_3$): | $\delta$ = 1,4 (s, 9H); 1,5 (dd, 3H); 3,12 (d, 1H); 3,32 (d, 1H); 3,75 (s, 3H); 4,91 (d, 1H); 5,13 (s und d, 3H); 5,58 - 5,68 (m, 1H); 5,73 - 5,79 (q, 1H); 6,03 (breites d, 1H); 6,86 (d, 2H); 7,28 (d, 2H); 7,52 (d, 1H); 8,01 (s, 1H); 8,1 (d, 1H); 9,4 (s, 1H) ppm. |

Beispiel 22

D-7-[(Benzothiazol-6-yl)glycylamido]-3-[(Z)-1-propen-1-yl]-3-cephem-4-carbonsäure

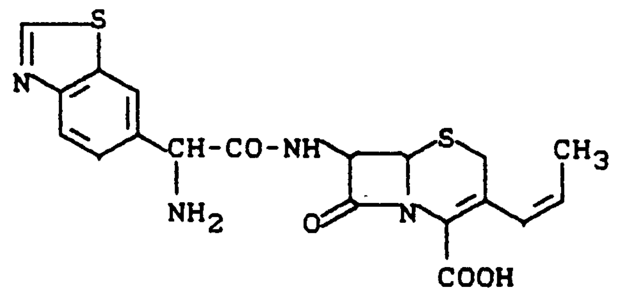

cis

Eine Mischung von 8,8 g (13,5 mmol) D-7-[2-(t-Butyloxycarbonylamino)-2-(benzothiazol-6-yl)glycylamido]-3-[(Z)-1-propen-1-yl]-3-cephem-4-carbonsäure-p-methoxybenzylester (Beispiel 20), 3 ml Anisol und 100 ml Trifluoressigsäure wird 1 Stunde bei Raumtemperatur gerührt. Man engt die Mischung im Vakuum ein, gibt zu dem hellroten, leichtbeweglichen Öl 40 ml Toluol und engt erneut im Vakuum ein. Das zurückbleibende Öl wird mit 400 ml Ether verrieben, der ausgefallene Feststoff abgesaugt, mit Ether gewaschen und im Vakuum getrocknet. Man erhält 6,3 g Trifluoracetat, die in 800 ml Wasser gelöst und von unlöslichen Bestandteilen durch Filtrieren über Kieselgur abgetrennt werden. Die Lösung wird über eine HP-20-Säule (600 ml, Diaion-Adsorberharz, Mitsubishi) geleitet, die mit Wasser und danach mit einem Gemisch von Wasser und Methanol (Gradient bis 50%) eluiert wird. Das die gewünschte Verbindung enthaltende Eluat wird lyophilisiert.

Ausbeute: 1,9 g

Das Lyophilisat wird in 150 ml Wasser unter magnetischem Rühren nahezu vollständig gelöst, 100 mg Aktivkohle zugegeben, 5 Minuten gerührt, dann über Kieselgur abgesaugt und mit 50 ml Wasser nachgewaschen. Das Filtrat wird nochmals mit einer Spritze mittels Membranfilter von Millipore filtriert und dann auf eine präparative Säule (Hibar 250-25, RP-18, Merck, Fluß: 10 - 15 ml $min^{-1}$) gepumpt. Nach der Probenaufgabe wird die Säule mit den folgenden Laufmittelsystemen nacheinander eluiert:

1.) 500 ml Wasser

2.) 500 ml wasser mit 10% Methanol

3.) 1000 ml Wasser mit 10% bis 40% Methanol: Das Eluat wird hier in 50 - 100 ml Fraktionen gesammelt und anschließend mittels analytischer HPLC untersucht, wobei festgestellt wird, daß die Fraktionen 8 bis 10 das cis-isomere Derivat enthalten.

| | |
|---|---|
| Ausbeute: | 468 mg |
| | $C_{19}H_{18}N_4O_4S_2$ (430,5) |
| NMR (DCOOD): | $\delta$ = 1,61 (dd, 3H); 3,31 (d, 1H); 3,48 (d, 1H); 5,25 (d, 1H); 5,75 - 5,9 (m und q, 3H); 6,2 (breites d, 1H); 8,13 (dd, 1H); 8,48 (d, 1H); 8,67 (s, 1H); 10,33 (s, 1H); ppm. |
| HPLC-analytisch: | Hibar 250-4, RP-8, 10 µm, 254 nm |
| Laufmittelsystem: | 790 ml Wasser - 200 ml Methanol - 10 ml Puffer pH 7,0 |
| Fluß: | 2 ml/min, Konzentration: 1 mg/ml |
| Retention: | 7,53 (Gehalt: 92,4%) |

Beispiel 23

D-7-[(2-Aminobenzothiazol-6-yl)glycylamido]-3-vinyl-3-cephem-4-carbonsäure

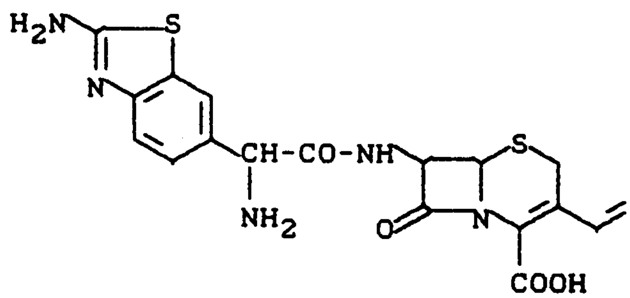

Zu einer auf -50°C gekühlten Lösung von 512,5 mg (1,585 mmol) D-α-t-Butyloxycarbonylamino-α-(2-aminobenzothiazol-6-yl)essigsäure in 5 ml Dimethylformamid werden nacheinander 276,1 μl (1,585 mmol) Ethyldiisopropylamin und 122,7 μl (1,585 mmol) Mesylchlorid langsam zugespritzt. Man rührt 40 Minuten bei -50°C und tropft eine Lösung (-20°C) von 622 mg (1,585 mmol) 7-Amino-3-vinyl-3-cephem-4-carbonsäure-diphenylmethylester und 276,1 μl (1,585 mmol) Ethyldiisopropylamin in 5 ml Tetrahydrofuran and 3 ml Dimethylformamid zu. Es wird 5 Minuten bei -50°C und anschließend noch 50 Minuten ohne Kühlung nachgerührt. Danach wird die Reaktionslösung in 40 ml Wasser und 120 ml Essigester eingerührt, die Essigesterphase abgetrennt, die wäßrige Schicht nochmals mit 60 ml Essigester extrahiert, die organischen Phasen vereinigt, mit 0,1 N Salzsäure, Natriumhydrogencarbonat-Lösung und Kochsalzlösung gewaschen. Nach dem Trocknen und Abdestillieren des Lösemittels wird der Rückstand in 20 ml Methylenchlorid aufgenommen, 20 ml Trifluoressigsäure mit 1 Tropfen Anisol zugesetzt und 45 Minuten bei Raumtemperatur gerührt. Anschließend wird das Trifluoressigsäure / Methylenchlorid- Gemisch im Vakuum abdestilliert und der Rückstand in 15 ml 80%iger Essigsäure gelöst, auf eine RP-18-Säule (Hibar 250-25, Merck) gepumpt und mit 3%iger Essigsäure eluiert. Das Eluat, das die gewünschte Substanz enthält, wird gefriergetrocknet.

Ausbeute: 800 mg

Das Lyophilisat wird nochmals in 10 ml 3%iger Essigsäure gelöst und an einer RP-18-Säule (Hibar 250-25, Merck) rechromatographiert.

Ausbeute: 165 mg

$C_{18}H_{17}N_5O_4S_2 \cdot 3H_2O \cdot 1/2CH_3COOH$ (505,5)

NMR (DCOOD): δ = 3,61 - 3,76 (dd, 2H); 5,28 (d, 1H); 5,52 (d, 1H); 5,69 (d, 1H); 5,75 (s, 1H); 5,91 (d, 1H); 7,18 - 7,28 (q, 1H); 7,84 (m, 2H); 8,18 (s, 1H) ppm.

**Patentansprüche**

**Patentansprüche für folgende Vertragsstaaten : AT, BE, CH, DE, FR, GB, IT, LI, NL, SE**

**1.** β-Lactam-Verbindungen der allgemeinen Formel (I)

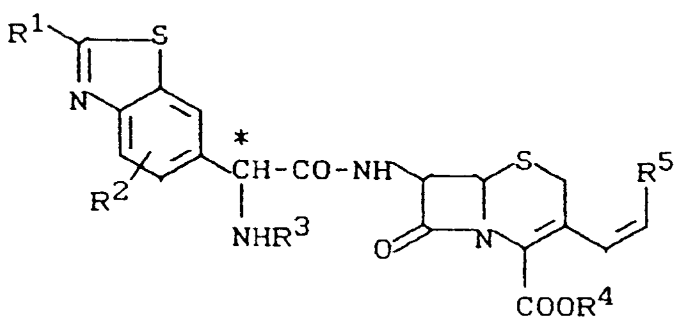

(I),

in welcher

$R^1$

- für Wasserstoff steht, oder
- für ein geradkettiges, verzweigtes oder cyclisches Alkyl mit bis zu 8 Kohlenstoffatomen steht, das durch Halogen, Hydroxy, Alkoxy mit bis zu 4 Kohlenstoffatomen, Carboxy, Phenyl, Sulfo oder durch eine Gruppe der Formel

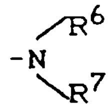

substituiert sein kann,
wobei

$R^6$,$R^7$

- gleich oder verschieden sind und
- Wasserstoff oder Alkyl mit bis zu 6 Kohlenstoffatomen, oder
- für Phenyl, Halogen, Alkoxy, Alkylthio, Alkylsulfonyl mit jeweils bis zu 6 Kohlenstoffatomen, Sulfo, Sulfamoyl, Mercapto, Hydroxy, Phenylthio, Phenyloxy, Guanidino, Amidino, oder
- für eine Gruppe der Formel

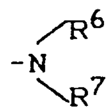

steht,
wobei
$R^6$, $R^7$ die oben angegebene Bedeutung haben,

$R^2$

- für Wasserstoff, Alkyl, Alkoxy, Alkylthio mit jeweils bis zu 6 Kohlenstoffatomen, Trifluormethyl, Trifluormethoxy, Hydroxy, Mercapto, Nitro, Cyano, oder Halogen steht,

$R^3$

- für Wasserstoff oder
- für eine in der $\beta$-Lactamchemie übliche Aminoschutzgruppe steht,

$R^4$

- für Wasserstoff,
- für eine in der $\beta$-Lactamchemie übliche Carboxyschutzgruppe oder
- für einen in vivo spaltbaren Ester steht,

und

$R^5$

- für Wasserstoff steht,
- für geradkettiges oder verzweigtes Alkyl mit bis zu 6 Kohlenstoffatomen steht, das substituiert sein kann durch Halogen, Alkoxy, Alkylthio mit jeweils bis zu 6 Kohlenstoffatomen, Hydroxy, Amino oder durch einen Rest der Formel

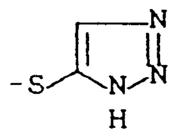 , 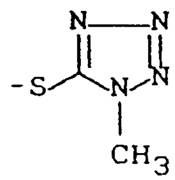 , 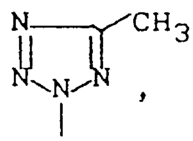 ,

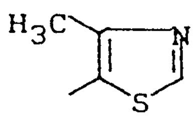 , 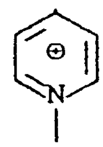 , 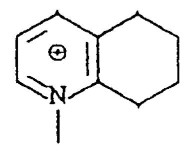

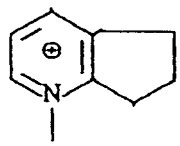 , 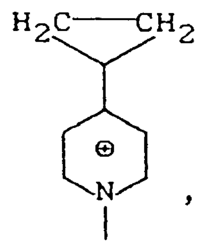 , 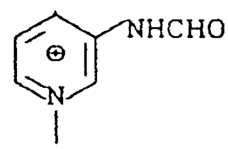

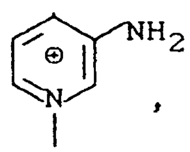 , 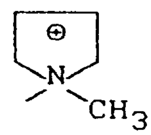 , 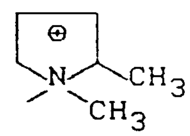 ,

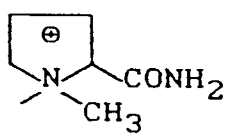 , 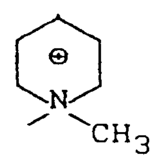 , 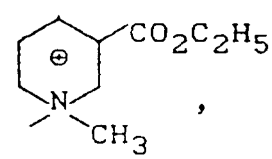 ,

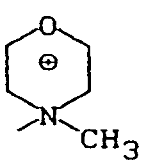 , 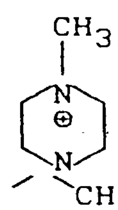 oder 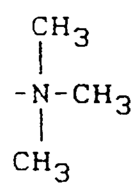

und deren Salze.

**2.** Verbindungen der allgemeinen Formel (I) nach Anspruch 1, in welcher

$R^1$

- für Wasserstoff oder
- für geradkettiges, verzweigtes oder cyclisches Alkyl mit bis zu 6 Kohlenstoffatomen steht, oder
- für Phenyl steht, oder
- für eine Gruppe der Formel $-NHR^6$ steht, worin

$R^6$

- Wasserstoff, Phenyl, Benzyl, Acetyl, Benzoyl oder Alkyl mit bis zu 6 Kohlenstoffatomen bedeutet,

$R^2$

- für Wasserstoff, Methyl, Methoxy, Trifluormethyl, Trifluormethoxy, Nitro, Cyano, Fluor, Chlor, Brom oder Hydroxy steht,

$R^3$

- für Wasserstoff oder
- für eine Aminoschutzgruppe aus der Reihe: Benzyl, tert-Butoxycarbonyl, Benzyloxycarbonyl, 2-Nitrobenzyloxycarbonyl, 4-Nitrobenzyloxycarbonyl, 2,2,2-Trichlorethoxycarbonyl, Fluorenyl-(9)-methoxycarbonyl, N-Diphenyl-methoxycarbonyl, Acetoacetyl, 2-Nitrobenzoyl, 2-Nitrophenylsulfenyl, Phthaloyl, Trityl, Vinyloxycarbonyl, Formyl, Benzoyl, Allyloxycarbonyl, 2,4-Dimethoxybenzyloxycarbonyl, 2-Methylthio-ethoxycarbonyl, 1,3-Dithian-2-ylmethoxycarbonyl (Dmoc), Trimethyl-, Triethyl-, Triphenylsilyl, tert-Butyl-dimethylsilyl, tert-Butyldiphenylsilyl, 1-Methyl-2-benzoyl-vinyl, 1-Methyl-2-ethoxycarbonyl-vinyl, 1-Methyl-2-methoxycarbonylvinyl, 1-Methyl-2-(2,6-dimethoxybenzoyl)-vinyl, 4-Methoxybenzyloxycarbonyl,

$R^4$

- für Wasserstoff steht, oder
- für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxybenzyl, 2,4-Dimethoxybenzyl, 1-Phenoxyethyl, 2-Methyl-2-propenyl, 4-Nitrobenzyl, 2-Nitrobenzyl, Trimethylsilylethyl oder tert.Butyl-dimethylsilylethyl steht,
  oder
- für einen Rest der Formel

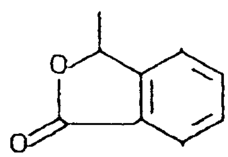 , 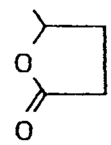 , 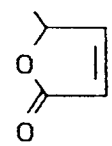 ,

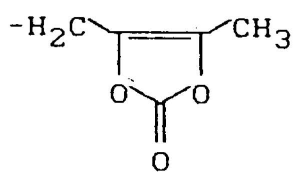 ,

$-CH_2-OCO-C(CH_3)_3$,
$-CH(CH_3)-OCOOC_2H_5$ oder
$-CH_2-OCOCH_3$ steht,

und

$R^5$

- für Wasserstoff steht, oder
- für geradkettiges oder verzweigtes Alkyl mit bis zu 4 Kohlenstoffatomen steht, das substituiert sein kann durch Fluor, Chlor, Brom, Iod, Alkoxy mit bis zu 3 Kohlenstoffatomen, Hydroxy oder Amino oder durch einen Rest der Formel

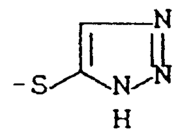 , 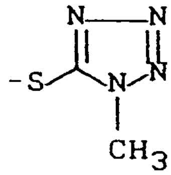 , 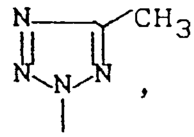 ,

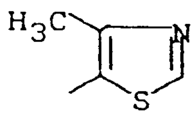 , 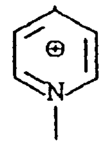 , 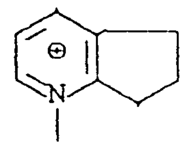 ,

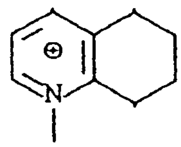 , 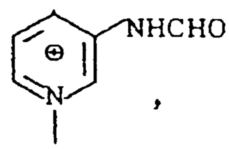 , 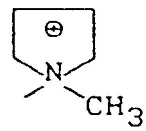 ,

oder 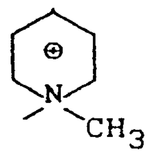

und deren Salze.

3. Verbindungen der allgemeinen Formel (I) nach Anspruch 1,
in welcher

$R^1$

- für Wasserstoff oder
- für geradkettiges oder verzweigtes Alkyl mit bis zu 4 Kohlenstoffatomen steht, oder
- für eine Gruppe der Formel -$NHR^6$ steht,

worin

$R^6$

- Wasserstoff, Methyl, Ethyl, Propyl oder Isopropyl bedeutet,

$R^2$

- für Wasserstoff oder Hydroxy steht,

$R^3$

- für Wasserstoff oder
für tert-Butoxycarbonyl, Benzyloxycarbonyl, Trityl, Allyloxycarbonyl, tert-Butyl-dimethylsilyl, 1-Methyl-2-benzoylvinyl, 1-Methyl-2-methoxycarbonylvinyl, 2-Nitrophenylsulfenyl, oder

$R^4$

- für Wasserstoff steht, oder
- für Methyl, Ethyl, tert.Butyl, Diphenylmethyl, 2,2,2-Trichlorethyl, Allyl, Acetoxymethyl, 4-Nitrobenzyl, 2-Nitrobenzyl, 4-Methoxybenzyl, Benzyl oder Trimethylsilylethyl steht, oder
- für einen Rest der Formel

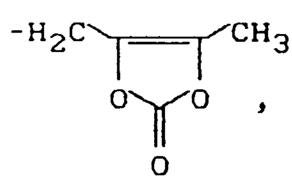 ,

$-CH(CH_3)-OCOOC_2H_5$
oder $-CH_2-OCO-C(CH_3)_3$ steht,

und

$R^5$

- für Wasserstoff, Methyl, Dihydroxyethyl, Chlormethyl, Iodmethyl, oder
- für einen Rest der Formel

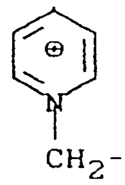 , 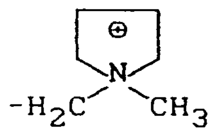 , 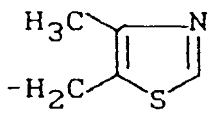 , 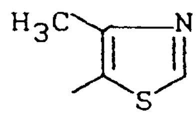

oder 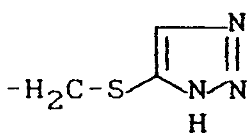

steht,

und deren Salze.

**4.** Verfahren zur Herstellung von β-Lactam-Verbindungen der allgemeinen Formel (I)

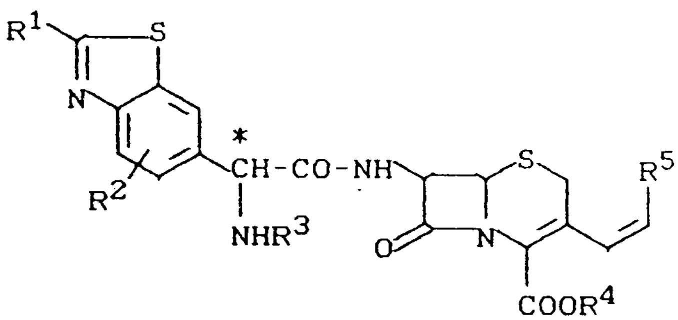 (I),

gemäß Anspruch 1, dadurch gekennzeichnet, daß man

[A] substituierte Cephalosporinverbindungen der allgemeinen Formel (II)

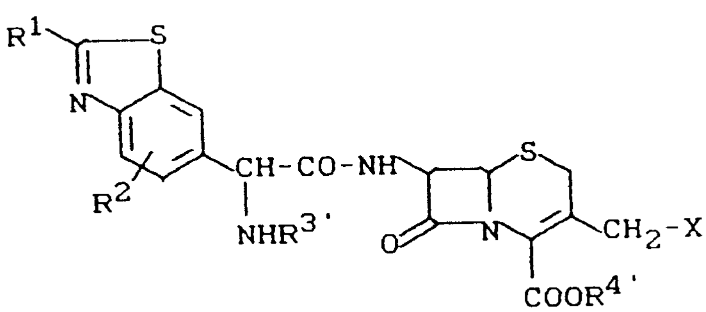 (II)

in welcher

$R^1$ und $R^2$ die oben angegebene Bedeutung hat,

$R^{3'}$

- für eine in der β-Lactamchemie übliche Aminoschutzgruppe steht,

$R^{4'}$

- für eine in der β-Lactamchemie übliche Carboxyschutzgruppe steht,

und

X

- für eine Gruppe der Formel

$-\overset{\oplus}{P}(R^{13})_3Z^{\ominus}$ , 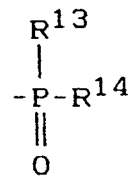 oder 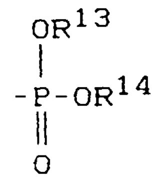

steht, wobei

$R^{13}$ und $R^{14}$ gleich oder verschieden sind und Alkyl, Phenyl oder Tolyl bedeuten

und

$Z^{\ominus}$

- ein Halogenidanion, bevorzugt Chlorid, Bromid oder Iodid bedeutet,

in inerten Lösemitteln in Anwesenheit von Basen, mit Aldehyden der allgemeinen Formel (III)

$R^5$-CHO (III)

in welcher

$R^5$ die oben angegebene Bedeutung hat

umsetzt,

oder daß man

[B] Phosphoniumverbindungen der allgemeinen Formel (IV)

$R^5$-$CH_2$-X (IV)

in welcher

$R^5$ die oben angegebene Bedeutung hat

und

X

- für eine Gruppe der Formel

$-\overset{\oplus}{P}(R^{13})_3Z^{\ominus}$ , 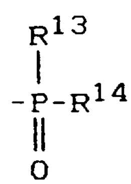 oder 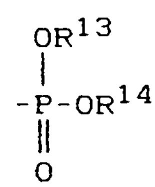

steht,

wobei

$R^{13}$ und $R^{14}$ gleich oder verschieden sind und Alkyl, Phenyl oder Tolyl bedeuten

und

$Z^{\ominus}$

- ein Halogenidanion, bevorzugt Chlorid, Bromid oder Iodid bedeutet,

in inerten Lösemitteln in Anwesenheit von Basen, mit Cephalosporinaldehyden der allgemeinen Formel (V)

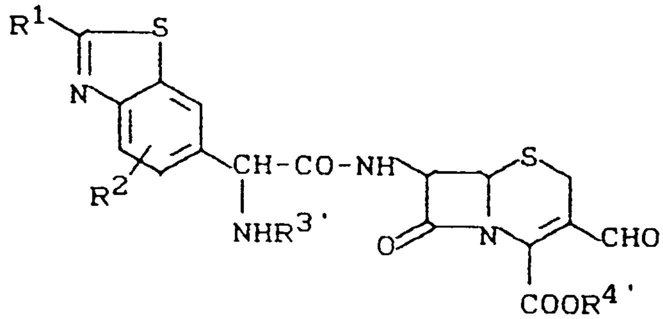

(V)

in welcher
$R^1$ und $R^2$ die oben angegebene Bedeutung haben,
$R^{3'}$ für eine in der $\beta$-Lactamchemie übliche Aminoschutzgruppe steht
und
$R^{4'}$ für eine in der $\beta$-Lactamchemie übliche Carboxyschutzgruppe steht,
umsetzt,
oder, daß man
[C] Carbonsäuren der allgemeinen Formel (VI)

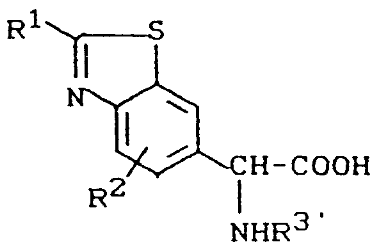

(VI)

in welcher
$R^1$ und $R^2$ die oben angegebene Bedeutung haben,
und
$R^{3'}$ für eine in der $\beta$-Lactamchemie übliche Aminoschutzgruppe steht,
nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester beispielsweise mit N-Hydroxybenztriazol und Dicyclohexylcarbodiimid (DCC),
mit den Vinylcephalosporinaminen der allgemeinen Formel (VII)

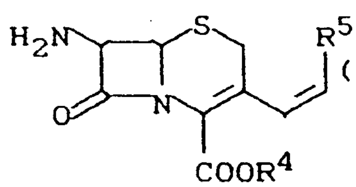

(VII),

in welcher
$R^4$ und $R^5$ die oben angegebene Bedeutung haben,
umsetzt,
dann gegebenenfalls Schutzgruppen abspaltet und die gewünschten Salze oder aus den Salzen die freien Säuren herstellt.

**5.** $\beta$-Lactam-Verbindungen der allgemeinen Formel (I)

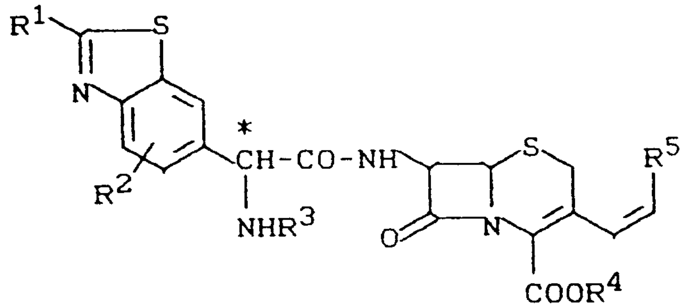

(I),

gemäß Anspruch 1, zur Verwendung in einem Verfahren zur therapeutischen Behandlung des menschlichen oder tierischen Körpers.

**6.** Arzneimittel, enthaltend $\beta$-Lactamverbindungen der allgemeinen Formel (I)

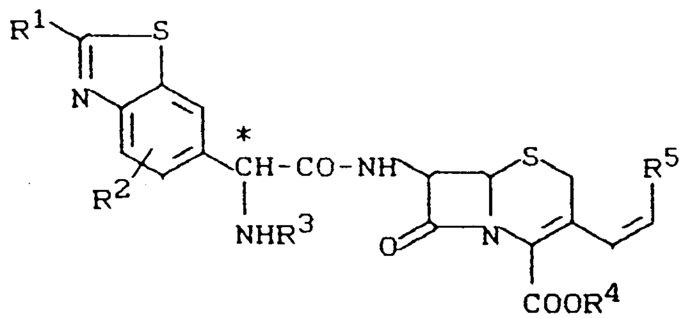

(I),

gemäß Anspruch 1, und deren Salze.

**7.** Verwendung von $\beta$-Lactam-Verbindungen der allgemeinen Formel (I) nach Anspruch 1 zur Herstellung von Arzneimitteln.

**8.** Tierfutter und Tierfutterzusatzmittel, enthaltend $\beta$-Lactam-Verbindungen der allgemeinen Formel (I) nach Anspruch 1.

**Patentansprüche für folgende Vertragsstaaten : ES, GR**

**1.** Verfahren zur Herstellung von $\beta$-Lactam-Verbindungen der allgemeinen Formel (I)

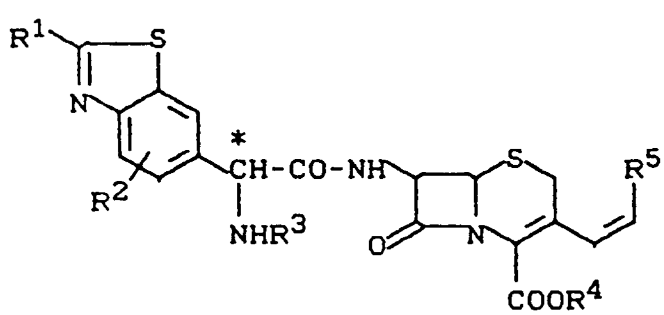

(I),

in welcher

$R^1$

- für Wasserstoff steht, oder
- für ein geradkettiges, verzweigtes oder cyclisches Alkyl mit bis zu 8 Kohlenstoffatomen steht, das durch Halogen, Hydroxy, Alkoxy mit bis zu 4 Kohlenstoffatomen, Carboxy, Phenyl, Sulfo oder durch eine Gruppe der Formel

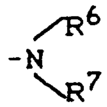

substituiert sein kann,

wobei

$R^6$,$R^7$

- gleich oder verschieden sind und
- Wasserstoff oder Alkyl mit bis zu 6 Kohlenstoffatomen, oder
- für Phenyl, Halogen, Alkoxy, Alkylthio, Alkylsulfonyl mit jeweils bis zu 6 Kohlenstoffatomen, Sulfo, Sulfamoyl, Mercapto, Hydroxy, Phenylthio, Phenyloxy, Guanidino, Amidino, oder
- für eine Gruppe der Formel

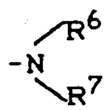

steht,

wobei

$R^6$, $R^7$ die oben angegebene Bedeutung haben,

$R^2$

- für Wasserstoff, Alkyl, Alkoxy, Alkylthio mit jeweils bis zu 6 Kohlenstoffatomen, Trifluormethyl, Trifluormethoxy, Hydroxy, Mercapto, Nitro, Cyano, oder Halogen steht,

$R^3$

- für Wasserstoff oder
- für eine in der $\beta$-Lactamchemie übliche Aminoschutzgruppe steht,

$R^4$

- für Wasserstoff,
- für eine in der $\beta$-Lactamchemie übliche Carboxyschutzgruppe oder
- für einen in vivo spaltbaren Ester steht,

und

$R^5$

- für Wasserstoff steht,
- für geradkettiges oder verzweigtes Alkyl mit bis zu 6 Kohlenstoffatomen steht, das substituiert sein kann durch Halogen, Alkoxy, Alkylthio mit jeweils bis zu 6 Kohlenstoffatomen, Hydroxy, Amino oder durch einen Rest der Formel

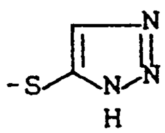, 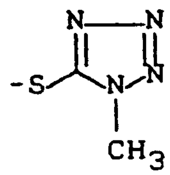, 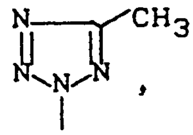,

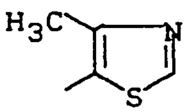, 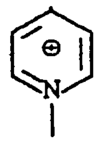, 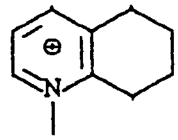

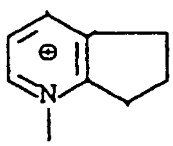, 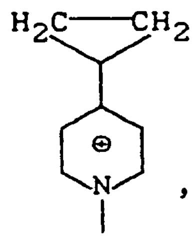, 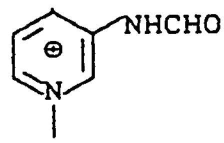

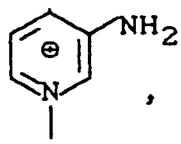, 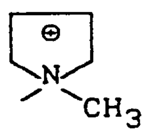, 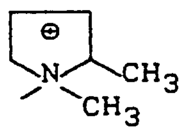,

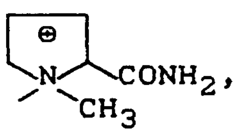, 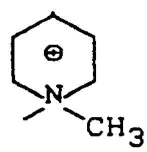, 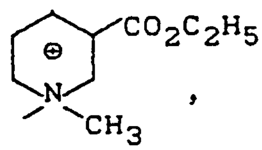,

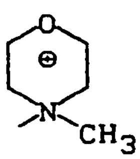, 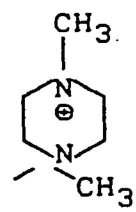 oder 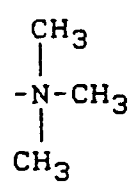

dadurch gekennzeichnet, daß man

[A] substituierte Cephalosporinverbindungen der allgemeinen Formel (II)

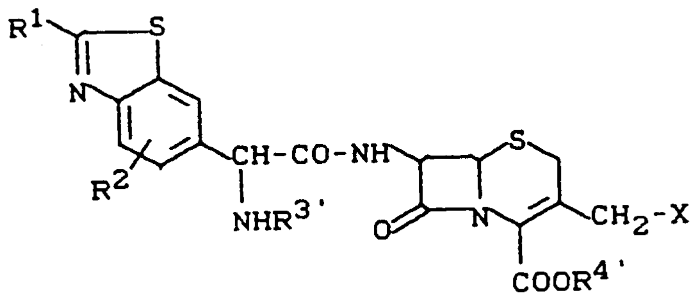 (II)

in welcher

$R^1$ und $R^2$ die oben angegebene Bedeutung hat,

$R^{3'}$

- für eine in der β-Lactamchemie übliche Aminoschutzgruppe steht,

$R^{4'}$

- für eine in der β-Lactamchemie übliche Carboxyschutzgruppe steht,

und

X

- für eine Gruppe der Formel

$-\overset{\ominus}{P}(R^{13})_3Z^{\ominus}$ , 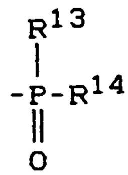 oder 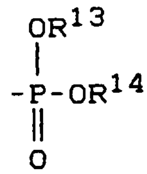

steht, wobei

$R^{13}$ und $R^{14}$ gleich oder verschieden sind und Alkyl, Phenyl oder Tolyl bedeuten

und

$Z^{\ominus}$

- ein Halogenidanion, bevorzugt Chlorid, Bromid oder Iodid bedeutet,

in inerten Lösemitteln in Anwesenheit von Basen, mit Aldehyden der allgemeinen Formel (III)

$R^5$-CHO (III)

in welcher
$R^5$ die oben angegebene Bedeutung hat
umsetzt,
oder daß man
[B] Phosphoniumverbindungen der allgemeinen Formel (IV)

$R^5$-$CH_2$-X (IV)

in welcher
$R^5$ die oben angegebene Bedeutung hat
und

X

- für eine Gruppe der Formel

$-\overset{\ominus}{P}(R^{13})_3Z^{\ominus}$ , 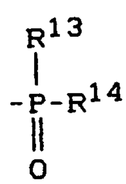 oder 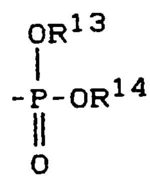

steht,

wobei

$R^{13}$ und $R^{14}$ gleich oder verschieden sind und Alkyl, Phenyl oder Tolyl bedeuten und

$Z^{\ominus}$

- ein Halogenidanion, bevorzugt Chlorid, Bromid oder Iodid bedeutet,

in inerten Lösemitteln in Anwesenheit von Basen, mit Cephalosporinaldehyden der allgemeinen Formel (V)

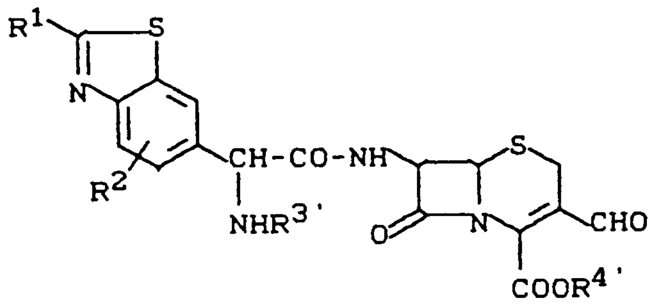

(V)

in welcher
$R^1$ und $R^2$ die oben angegebene Bedeutung haben,
$R^{3'}$ für eine in der $\beta$-Lactamchemie übliche Aminoschutzgruppe steht
und
$R^{4'}$ für eine in der $\beta$-Lactamchemie übliche Carboxyschutzgruppe steht,
umsetzt,
oder, daß man
[C] Carbonsäuren der allgemeinen Formel (VI)

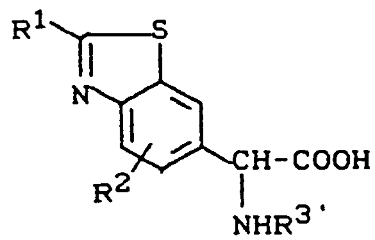

(VI)

in welcher
$R^1$ und $R^2$ die oben angegebene Bedeutung haben,
und
$R^{3'}$ für eine in der $\beta$-Lactamchemie übliche Aminoschutzgruppe steht,
nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester beispielsweise mit N-Hydroxybenztriazol und Dicyclohexylcarbodiimid (DCC),
mit den Vinylcephalosporinaminen der allgemeinen Formel (VII)

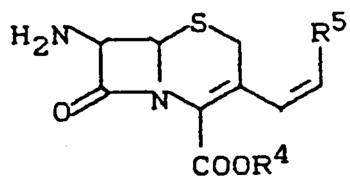

(VII),

in welcher
$R^4$ und $R^5$ die oben angegebene Bedeutung haben,
umsetzt,
dann gegebenenfalls Schutzgruppen abspaltet und die gewünschten Salze oder aus den Salzen die freien Säuren herstellt.

**2.** Verwendung von $\beta$-Lactam-Verbindungen der allgemeinen Formel (I) nach Anspruch 1 zur Herstellung von Arzneimitteln.

**3.** Tierfutter und Tierfutterzusatzmittel, enthaltend β-Lactam-Verbindungen der allgemeinen Formel (I) nach Anspruch 1.

**Claims**

**Claims for the following Contracting States : AT, BE, CH, DE, FR, GB, IT, LI, NL, SE**

**1.** β-Lactam compounds of the general formula (I)

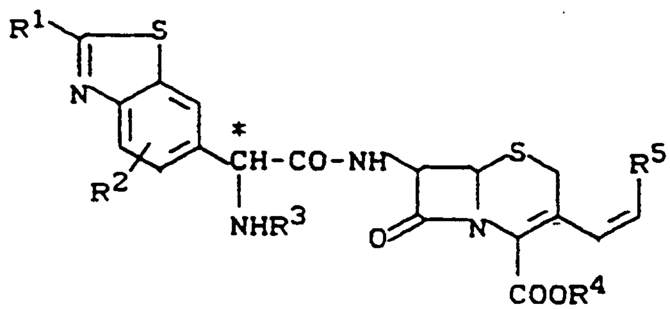

(I),

in which

$R^1$

- represents hydrogen, or
- represents a straight-chain, branched or cyclic alkyl which has up to 8 carbon atoms and can be substituted by halogen, hydroxyl, alkoxy with up to 4 carbon atoms, carboxyl, phenyl or sulpho, or by a group of the formula

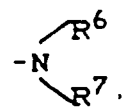

wherein

$R^6$ and $R^7$

- are identical or different and
- denote hydrogen or alkyl with up to 6 carbon atoms,

or

- represent phenyl, halogen, alkoxy, alkylthio or alkylsulphonyl with in each case up to 6 carbon atoms, sulpho, sulphamoyl, mercapto, hydroxyl, phenylthio, phenyloxy, guanidino or amidino, or
- represent a group of the formula

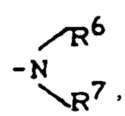

wherein

$R^6$ and $R^7$ have the abovementioned meaning,

$R^2$

- represents hydrogen, alkyl, alkoxy, or alkylthio with in each case up to 6 carbon atoms, trifluoromethyl, trifluoromethoxy, hydroxyl, mercapto, nitro, cyano or halogen,

$R^3$

- represents hydrogen, or

- represents an amino-protective group customary in β-lactam chemistry,

$R^4$

- represents hydrogen,
- represents a carboxyl-protective group customary in β-lactam chemistry, or
- represents an ester which can be split off in vivo,

and

$R^5$

- represents hydrogen, or
- represents straight-chain or branched alkyl which has up to 6 carbon atoms and can be substituted by halogen, alkoxy or alkylthio with in each case up to 6 carbon atoms, hydroxyl or amino, or by a radical of the formula

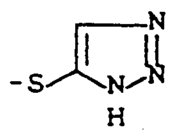 , 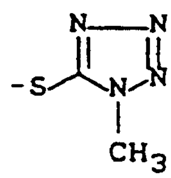 , 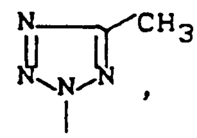 ,

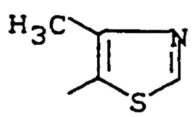 , 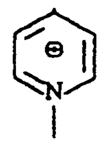 , 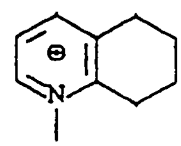

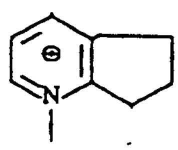 , 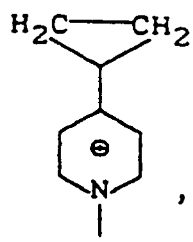 , 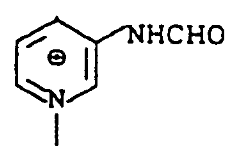

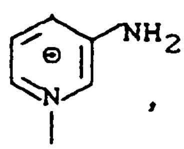 , 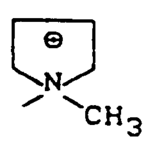 , 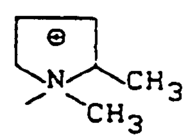 ,

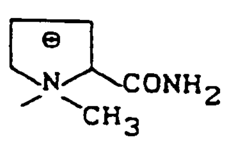 , 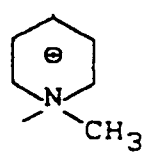 , 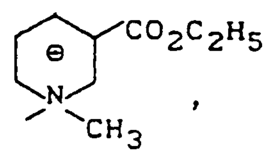 ,

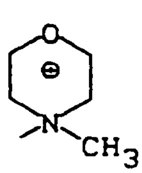 , 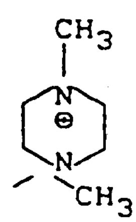 or 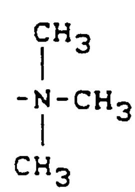

and salts thereof.

2. Compounds of the general formula (I) according to Claim 1, in which

$R^1$

- represents hydrogen, or
- represents straight-chain, branched or cyclic alkyl with up to 6 carbon atoms, or
- represents phenyl, or - represents a group of the formula $-NHR^6$,

wherein

$R^6$

- denotes hydrogen, phenyl, benzyl, acetyl, benzoyl or alkyl with up to 6 carbon atoms,

$R^2$

- represents hydrogen, methyl, methoxy, trifluoromethyl, trifluoromethoxy, nitro, cyano, fluorine, chlorine, bromine or hydroxyl,

$R^3$

- represents hydrogen, or
- represents an amino-protective group from the group consisting of benzyl, tert.-butoxycarbonyl, benzyloxycarbonyl, 2-nitrobenzyloxycarbonyl, 4-nitrobenzyloxycarbonyl, 2,2,2-trichloroethoxycarbonyl, fluorenyl-(9)-methoxycarbonyl, N-diphenyl-methoxycarbonyl, acetoacetyl, 2-nitrobenzoyl, 2-nitrophenylsulphenyl, phthaloyl, trityl, vinyloxycarbonyl, formyl, benzoyl, allyloxycarbonyl, 2,4-dimethoxybenzyloxycarbonyl, 2-methylthio-ethoxycarbonyl, 1,3-dithian-2-ylmethoxycarbonyl (Dmoc), trimethyl-, triethyl-, triphenylsilyl, tert.-butyldimethylsilyl, tert.-butyldiphenylsilyl, 1-methyl-2-benzoyl-vinyl, 1-methyl-2-ethoxycarbonyl-vinyl, 1-methyl-2-methoxycarbonyl-vinyl, 1-methyl-2-(2,6-dimethoxybenzoyl)-vinyl, 4-methoxybenzyloxycarbonyl,

$R^4$

- represents hydrogen, or
- represents methyl, ethyl, tert.-butyl, 2-chloroethyl, 2,2,2-trichloroethyl, cyanoethyl, diphenylmethyl, triphenylmethyl, acetoxymethyl, allyl, benzyl, 4-methoxybenzyl, 2,4-dimethoxybenzyl, 1-phenoxyethyl, 2-methyl-2-propenyl, 4-nitrobenzyl, 2-nitrobenzyl, trimethylsilylethyl or tert.-butyl-dimethylsilylethyl, or
- represents a radical of the formula

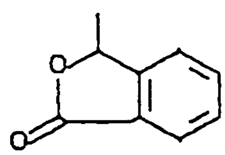 , 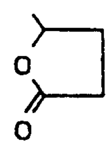 , 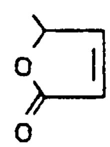 ,

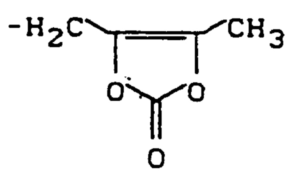 ,

$-CH_2-OCO-C(CH_3)_3$,
$-CH(CH_3)-OCOOC_2H_5$ or
$-CH_2-OCOCH_3$,

and

$R^5$

- represents hydrogen, or
- represents straight-chain or branched alkyl which has up to 4 carbon atoms and can be substituted by fluorine, chlorine, bromine, iodine, alkoxy with up to 3 carbon atoms, hydroxyl or amino, or by a radical of the formula

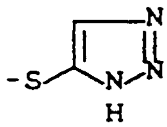 , 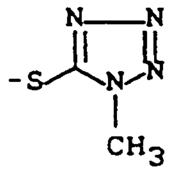 , 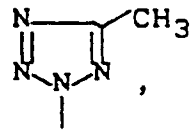 ,

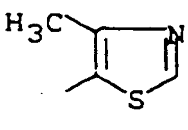 , 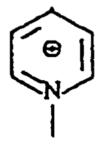 , 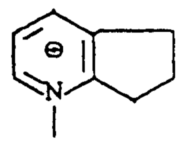 ,

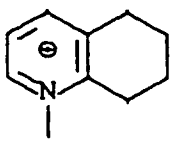 , 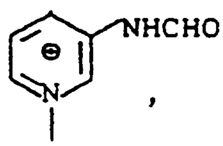 , 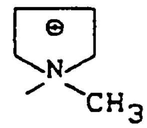 ,

or 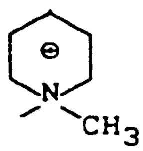

and salts thereof.

**3.** Compounds of the general formula (I) according to Claim 1, in which

$R^1$
- represents hydrogen, or
- represents straight-chain or branched alkyl with up to 4 carbon atoms, or
- represents a group of the formula $-NHR^6$

  wherein

$R^6$
- denotes hydrogen, methyl, ethyl, propyl or isopropyl,

$R^2$
- represents hydrogen or hydroxyl,

$R^3$
- represents hydrogen, or
- represents tert.-butoxycarbonyl, benzyloxycarbonyl, trityl, allyloxycarbonyl, tert.-butyldimethylsilyl, 1-methyl-2-benzoylvinyl, 1-methyl-2-methoxycarbonylvinyl, 2-nitrophenylsulphenyl, or

$R^4$
- represents hydrogen, or
- represents methyl, ethyl, tert.-butyl, diphenylmethyl, 2,2,2-trichloroethyl, allyl, acetoxymethyl, 4-nitrobenzyl, 2-nitrobenzyl, 4-methoxybenzyl, benzyl or trimethylsilylethyl, or

- represents a radical of the formula

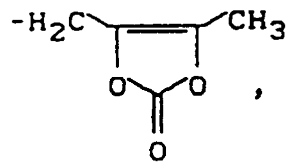

,

-CH($CH_3$)-$OCOOC_2H_5$
or -$CH_2$-OCO-C($CH_3$)$_3$,

and
$R^5$

- represents hydrogen, methyl, dihydroxyethyl, chloromethyl or iodomethyl, or
- represents a radical of the formula

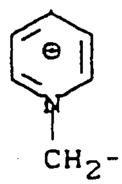 , 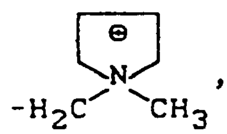 , 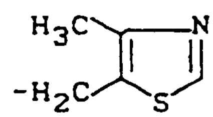 , 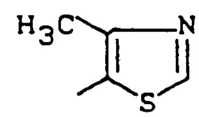

or

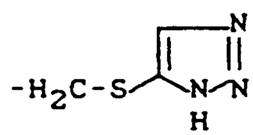

,

and salts thereof.

**4.** Process for the preparation of $\beta$-lactam compounds of the general formula (I)

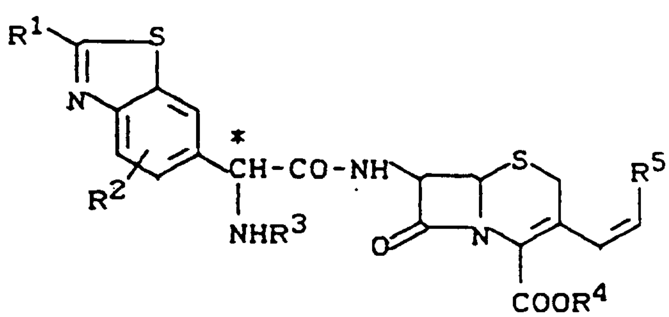

(I),

according to Claim 1, characterized in that

[A] substituted cephalosporin compounds of the general formula (II)

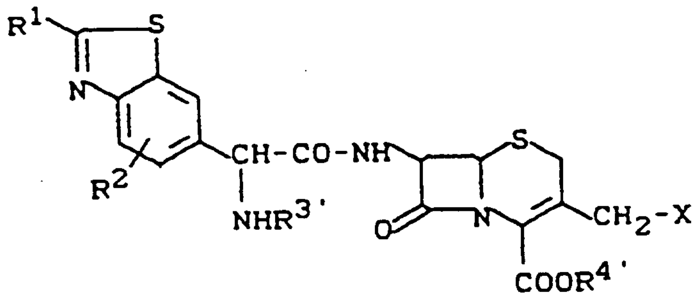

(II)

in which
$R^1$ and $R^2$ have the abovementioned meaning,

$R^{3'}$
- represents an amino-protective group customary in $\beta$-lactam chemistry,

$R^{4'}$
- represents a carboxyl-protective group customary in $\beta$-lactam chemistry,

and

X
- represents a group of the formula

$-\overset{\ominus}{P}(R^{13})_3Z^{\ominus}$ , 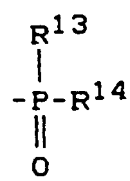 or 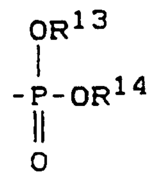 ,

wherein

$R^{13}$ and $R^{14}$ are identical or different and denote alkyl, phenyl or tolyl

and

$Z^{\ominus}$
- denotes a halide anion, preferably chloride, bromide or iodide,

are reacted with aldehydes of the general formula (III)

$R^5$-CHO (III)

in which
$R^5$ has the abovementioned meaning,
in inert solvents in the presence of bases,
or in that

[B] phosphonium compounds of the general formula (IV)

$R^5$-$CH_2$-X (IV)

in which
$R^5$ has the abovementioned meaning
and
X

- represents a group of the formula

$-\overset{\ominus}{P}(R^{13})_3Z^{\ominus}$ , 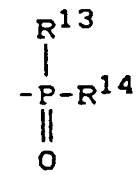 or 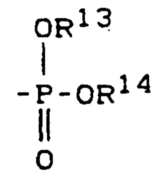 ,

wherein

$R^{13}$ and $R^{14}$ are identical or different and denote alkyl, phenyl or tolyl

and

$Z^{\ominus}$

- denotes a halide anion, preferably chloride, bromide or iodide,

are reacted with cephalosporinaldehydes of the general formula (V)

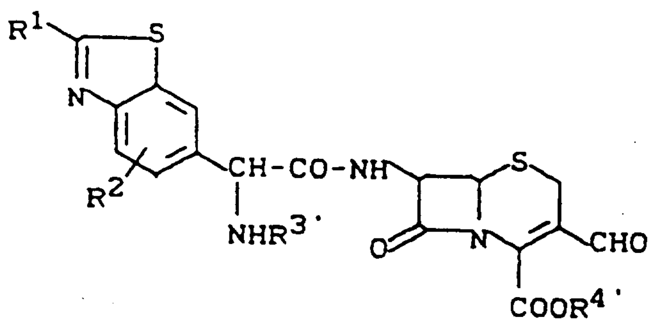

(V)

,

in which

$R^1$ and $R^2$ have the abovementioned meaning,

$R^{3'}$ represents an amino-protective group customary in $\beta$-lactam chemistry

and

$R^{4'}$ represents a carboxyl-protective group customary in $\beta$-lactam chemistry,

in inert solvents in the presence of bases, or in that

[C] carboxylic acids of the general formula (VI)

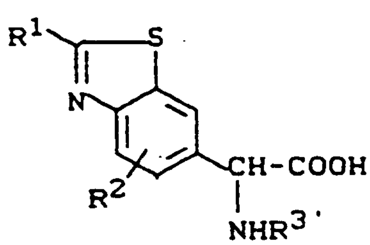

(VI)

in which

$R^1$ and $R^2$ have the abovementioned meaning, and

$R^{3'}$ represents an amino-protective group customary in $\beta$-lactam chemistry,

after activation of the carboxyl group by conversion into a mixed anhydride, for example with ethyl chloroformate, isobutyl chloroformate or methanesulphonyl chloride, or by conversion into the acid halide, or by conversion into an activated ester, for example with N-hydroxybenzotriazole and dicyclohexylcarbodiimide (DCC),

are reacted with the vinylcephalosporinamines of the general formula (VII)

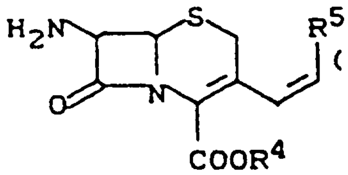

(VII),

in which
$R^4$ and $R^5$ have the abovementioned meaning,
and, if appropriate, protective groups are then split off and the desired salts are prepared or the free acids are prepared from the salts.

**5.** β-Lactam compounds of the general formula (I)

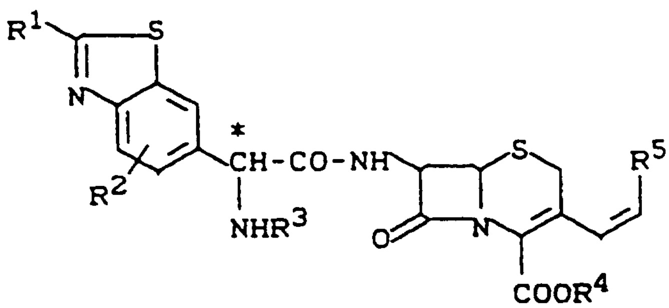

(I),

according to Claim 1, for use in a process for the therapeutic treatment of the human or animal body.

**6.** Medicaments containing β-lactam compounds of the general formula (I)

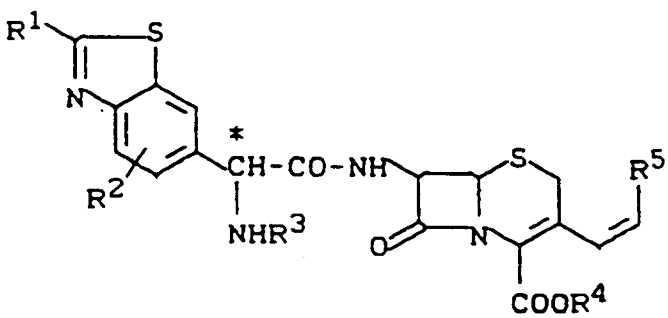

(I),

according to Claim 1, and salts thereof.

**7.** Use of β-lactam compounds of the general formula (I) according to Claim 1 for the preparation of medicaments.

**8.** Animal feeds and animal feed additives containing β-lactam compounds of the general formula (I) according to Claim 1.

**Claims for the following Contracting States : ES, GR**

**1.** Process for the preparation of β-lactam compounds of the general formula (I)

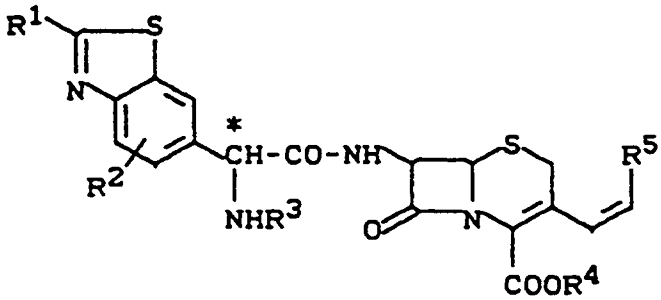

(I),

in which

$R^1$

- represents hydrogen, or
- represents a straight-chain, branched or cyclic alkyl which has up to 8 carbon atoms and can be substituted by halogen, hydroxyl, alkoxy with up to 4 carbon atoms, carboxyl, phenyl or sulpho, or by a group of the formula

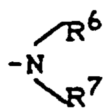

,

wherein

$R^6$ and $R^7$

- are identical or different and
- denote hydrogen or alkyl with up to 6 carbon atoms,

  or
- represent phenyl, halogen, alkoxy, alkylthio or alkylsulphonyl with in each case up to 6 carbon atoms, sulpho, sulphamoyl, mercapto, hydroxyl, phenylthio, phenyloxy, guanidino or amidino, or
- represent a group of the formula

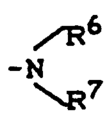

,

wherein

$R^6$ and $R^7$ have the abovementioned meaning,

$R^2$

- represents hydrogen, alkyl, alkoxy or alkylthio with in each case up to 6 carbon atoms, trifluoromethyl, trifluoromethoxy, hydroxyl, mercapto, nitro, cyano or halogen,

$R^3$

- represents hydrogen, or
- represents an amino-protective group customary in β-lactam chemistry,

$R^4$

- represents hydrogen,
- represents a carboxyl-protective group customary in β-lactam chemistry, or

- represents an ester which can be split off in vivo

and

$R^5$

- represents hydrogen, or
- represents straight-chain or branched alkyl which has up to 6 carbon atoms and can be substituted by halogen, alkoxy or alkylthio with in each case up to 6 carbon atoms, hydroxyl or amino, or by a radical of the formula

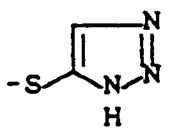 , 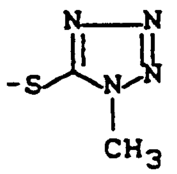 , 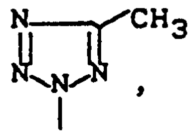 ,

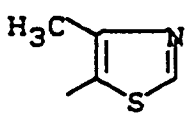 , 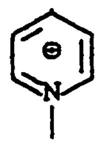 , 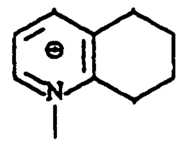

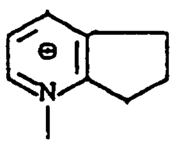 , 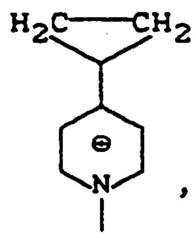 , 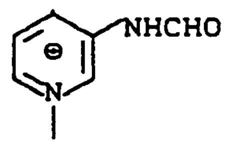

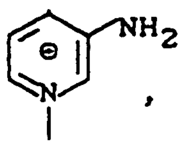 , 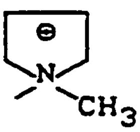 , 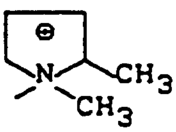 ,

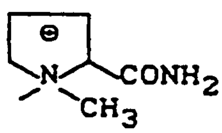 , 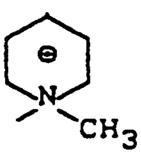 , 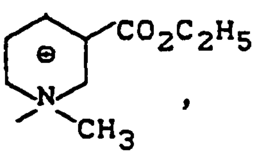 ,

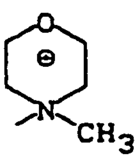 , 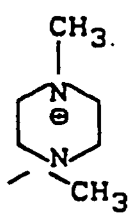 or 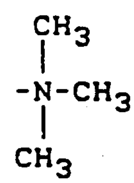

characterized in that

[A] substituted cephalosporin compounds of the general formula (II)

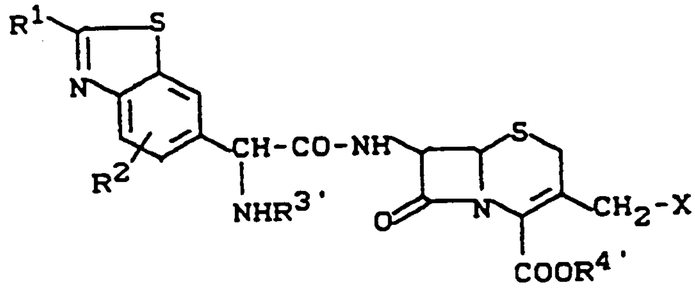

(II)

in which

$R^1$ and $R^2$ have the abovementioned meaning,

$R^{3'}$ - represents an amino-protective group customary in $\beta$-lactam chemistry,

$R^{4'}$ - represents a carboxyl-protective group customary in $\beta$-lactam chemistry,

and

X - represents a group of the formula

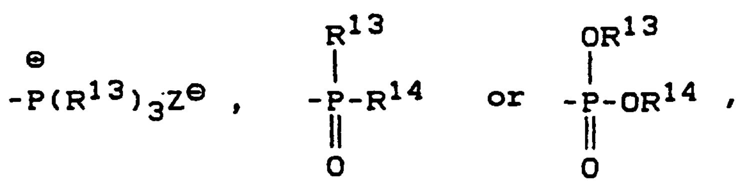

, or ,

wherein

$R^{13}$ and $R^{14}$ are identical or different and denote alkyl, phenyl or tolyl

and

$Z^{\ominus}$ - denotes a halide anion, preferably chloride, bromide or iodide,

are reacted with aldehydes of the general formula (III)

$R^5$-CHO (III)

in which

$R^5$ has the abovementioned meaning,

in inert solvents in the presence of bases, or in that

[B] phosphonium compounds of the general formula (IV)

$R^5$-$CH_2$-X (IV)

in which

$R^5$ has the abovementioned meaning

and

X

- represents a group of the formula

$-\overset{\ominus}{P}(R^{13})_3Z^{\ominus}$ , 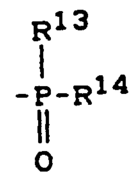 or 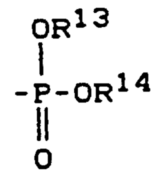 ,

wherein

$R^{13}$ and $R^{14}$ are identical or different and denote alkyl, phenyl or tolyl and

$Z^{\ominus}$ - denotes a halide anion, preferably chloride, bromide or iodide,

are reacted with cephalosporinaldehydes of the general formula (V)

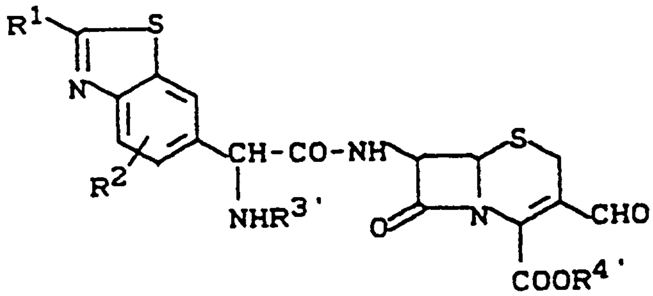

(V)

in which

$R^1$ and $R^2$ have the abovementioned meaning,

$R^{3'}$ represents an amino-protective group customary in $\beta$-lactam chemistry

and

$R^{4'}$ represents a carboxyl-protective group customary in $\beta$-lactam chemistry,

in inert solvents in the presence of bases,

or in that

[C] carboxylic acids of the general formula (VI)

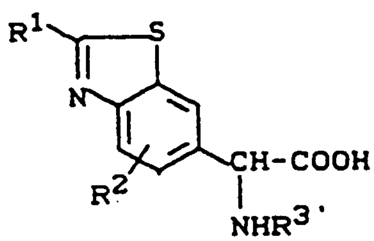

(VI)

in which

$R^1$ and $R^2$ have the abovementioned meaning,

and

$R^{3'}$ represents an amino-protective group customary in $\beta$-lactam chemistry,

after activation of the carboxyl group by conversion into a mixed anhydride, for example with ethyl chloroformate, isobutyl chloroformate or methanesulphonyl chloride, or by conversion into the acid halide, or by conversion into an activated ester, for example with N-hydroxybenzotriazole and dicyclohexylcarbodiimide (DCC),

are reacted with the vinylcephalosporinamines of the general formula (VII)

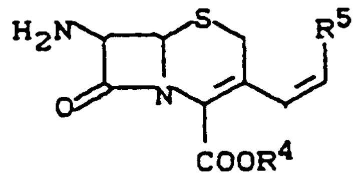

(VII),

in which

$R^4$ and $R^5$ have the abovementioned meaning,

and, if appropriate, protective groups are then split off and the desired salts are prepared or the free acids are prepared from the salts.

**2.** Use of $\beta$-lactam compounds of the general formula (I) according to Claim 1 for the preparation of medicaments.

**3.** Animal feeds and animal feed additives containing $\beta$-lactam compounds of the general formula (I) according to Claim 1.

## Revendications

**Revendications pour les Etats contractants suivants : AT, BE, CH, DE, FR, GB, IT, LI, NL, SE**

**1.** Composés $\beta$-lactames répondant à la formule générale (I)

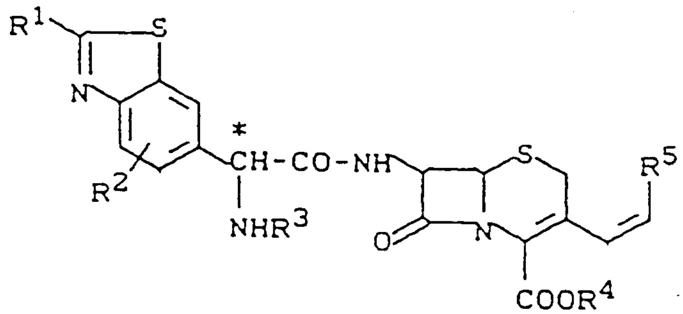

(I),

dans laquelle

$R^1$ représente un atome d'hydrogène, ou

représente un groupe alkyle à chaîne droite, à chaîne ramifiée ou encore cyclique, contenant jusqu'à 8 atomes de carbone, qui peut être substitué par un atome d'halogène, par un groupe hydroxyle, par un groupe alcoxy contenant jusqu'à 4 atomes de carbone, par un groupe carboxyle, par un groupe phényle, par un groupe sulfo ou encore par un groupe de formule

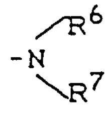

dans laquelle

$R^6$, $R^7$ sont identiques ou différents et

représentent un atome d'hydrogène ou un groupe alkyle contenant jusqu'à 6 atomes de carbone,

ou représentent un groupe phényle, un atome d'halogène, un groupe alcoxy, un groupe alkylthio, un groupe alkylsulfonyle contenant chacun jusqu'à 6 atomes de carbone, un groupe sulfo, un groupe sulfamoyle, un groupe mercapto, un groupe hydroxyle, un groupe phénylthio, un groupe phényloxy, un groupe guanidino ou un groupe amidino, ou représentent un groupe de formule

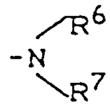

où

$R^6$, $R^7$ ont la signification indiquée ci-dessus,

$R^2$ représente un atome d'hydrogène, un groupe alkyle, un groupe alcoxy, un groupe alkylthio contenant chacun jusqu'à 6 atomes de carbone, un groupe trifluorométhyle, un groupe trifluorométhoxy, un groupe hydroxyle, un groupe mercapto, un groupe nitro, un groupe cyano ou un atome d'halogène,

$R^3$ représente un atome d'hydrogène ou
représente un groupe protecteur du groupe amino habituel dans la chimie des β-lactames,

$R^4$ représente un atome d'hydrogène,
représente un groupe protecteur du groupe carboxyle habituel dans la chimie des β-lactames, ou
représente un ester séparable in vivo,
et

$R^5$ représente un atome d'hydrogène,
un groupe alkyle à chaîne droite ou ramifiée contenant jusqu'à 6 atomes de carbone, qui peut être substitué par un atome d'halogène, par un groupe alcoxy, par un groupe alkylthio, contenant chacun jusqu'à 6 atomes de carbone, par un groupe hydroxyle, par un groupe amino, ou encore par un radical de formule

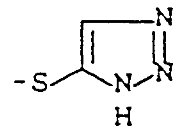, 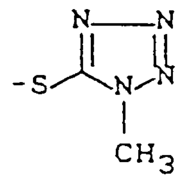, 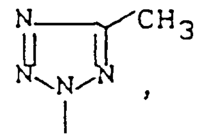,

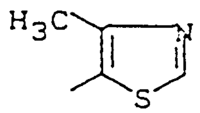, 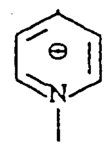, 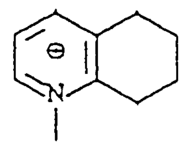·

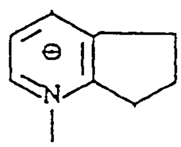, 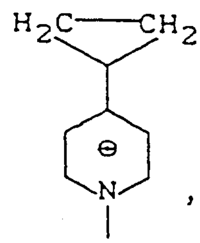, 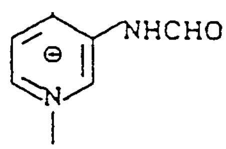

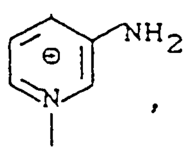, 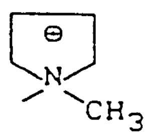, 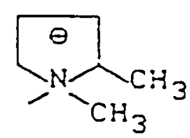,

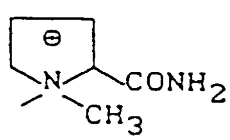 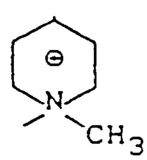, 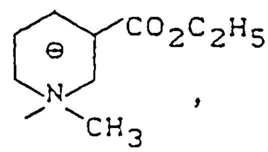,

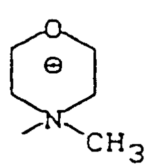, 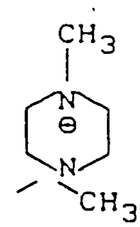 ou 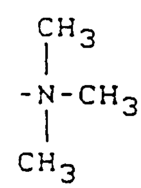

ainsi que leurs sels.

**2.** Composés répondant à la formule générale (I) selon la revendication 1, dans lesquels

$R^1$ représente un atome d'hydrogène ou un groupe alkyle à chaîne droite, à chaîne ramifiée ou cyclique contenant jusqu'à 6 atomes de carbone,
ou
représente un groupe phényle, ou
représente un groupe de formule $-NHR^6$,
dans laquelle

$R^6$ représente un atome d'hydrogène, un groupe phényle, un groupe benzyle, un groupe acétyle, un groupe benzoyle ou un groupe alkyle contenant jusqu'à 6 atomes de carbone,

$R^2$ représente un atome d'hydrogène, un groupe méthyle, un groupe méthoxy, un groupe trifluorométhyle, un groupe trifluorométhoxy, un groupe nitro, un groupe cyano, un atome de fluor, un atome de chlore, un atome de brome ou un groupe hydroxyle,

$R^3$ représente un atome d'hydrogène ou

représente un groupe protecteur du groupe amino de la série de groupes ci-après : un groupe benzyle, un groupe tert.-butoxycarbonyle, un groupe benzyloxycarbonyle, un groupe 2-nitrobenzyloxycarbonyle, un groupe 4-nitrobenzyloxycarbonyle, un groupe 2,2,2-trichloréthoxycarbonyle, un groupe fluorényl-(9)-méthoxycarbonyle, un groupe N-diphénylméthoxycarbonyle, un groupe acétoacétyle, un groupe 2-nitrobenzoyle, un groupe 2-nitrophénylsulfényle, un groupe phtaloyle, un groupe trityle, un groupe vinyloxycarbonyle, un groupe formyle, un groupe benzoyle, un groupe allyloxycarbonyle, un groupe 2,4-diméthoxybenzyloxycarbonyle, un groupe 2-méthylthioéthoxycarbonyle, un groupe 1,3-dithian-2-ylméthoxycarbonyle (Dmoc), un groupe triméthylsilyle, un groupe triéthylsilyle, un groupe triphénylsilyle, un groupe tert.-butyldiméthylsilyle, un groupe tert.-butyldiphényl-silyle, un groupe 1-méthyl-2-benzoylvinyle,un groupe 1-méthyl-2-éthoxycarbonylvinyle, un groupe 1-méthyl-2-méthoxycarbonylvinyle, un groupe 1-méthyl-2-(2,6-diméthoxybenzoyl)-vinyle, un groupe 4-méthoxybenzyloxy-carbonyle,

$R^4$ représente un atome d'hydrogène ou

représente un groupe méthyle, un groupe éthyle, un groupe tert.-butyle, un groupe 2-chloréthyle, un groupe 2,2,2-trichloréthyle, un groupe cyanoéthyle, un groupe diphénylméthyle, un groupe triphénylméthyle, un groupe acétoxyméthyle, un groupe allyle, un groupe benzyle, un groupe 4-méthoxybenzyle, un groupe 2,4-diméthoxybenzyle, un groupe 1-phénoxyéthyle, un groupe 2-méthyl-2-propényle, un groupe 4-nitrobenzyle, un groupe 2-nitrobenzyle, un groupe triméthylsilyléthyle ou un groupe tert.-butyldiméthylsilyléthyle, ou représente un radical de formule

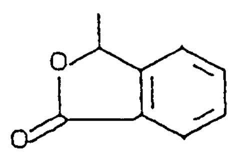 , 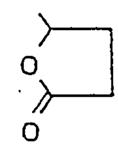 , 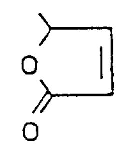

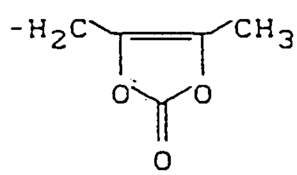 ,

$-CH_2-OCO-C(CH_3)_3$,
$-CH(CH_3)-OCOOC_2H_5$ ou
$-CH_2-OCOCH_3$
et

$R^5$ représente un atome d'hydrogène, ou

un groupe alkyle à chaîne droite ou ramifiée contenant jusqu'à 4 atomes de carbone, qui peut être substitué par un atome de fluor, par un atome de chlore, par un atome de brome, par un atome d'iode, par un groupe alcoxy contenant jusqu'à 3 atomes de carbone, par un groupe hydroxyle ou par un groupe amino, ou encore par un radical de formule

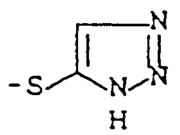 , 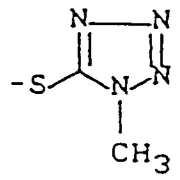 , 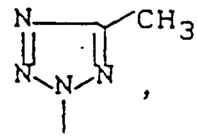 ,

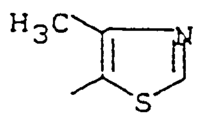 , 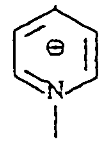 , 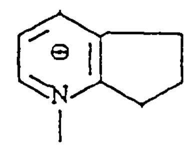 ,

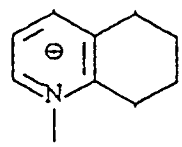 , 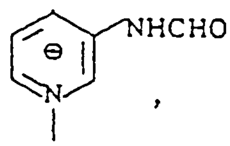 , 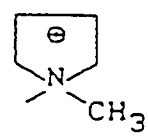 ,

ou 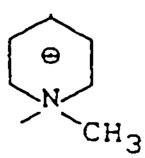 .

ainsi que leurs sels.

3. Composés répondant à la formule générale (I) selon la revendication 1, dans lesquels

$R^1$ représente un atome d'hydrogène ou représente un groupe alkyle à chaîne droite ou ramifiée contenant jusqu'à 4 atomes de carbone, ou un groupe de formule $-NHR^6$, dans laquelle

$R^6$ représente un atome d'hydrogène, un groupe méthyle, un groupe éthyle, un groupe propyle ou un groupe isopropyle,

$R^2$ représente un atome d'hydrogène ou un groupe hydroxyle,

$R^3$ représente un atome d'hydrogène ou un groupe tert.-butoxycarbonyle, un groupe benzyloxycarbonyle, un groupe trityle, un groupe allyloxycarbonyle, un groupe tert.-butyldiméthylsilyle, un groupe 1-méthyl-2-benzoylvinyle, un groupe 1-méthyl-2-méthoxycarbonylvinyle, un groupe 2-nitrophénylsulfényle, ou

$R^4$ représente un atome d'hydrogène ou représente un groupe méthyle, un groupe éthyle, un groupe tert.-butyle, un groupe diphénylméthyle, un groupe 2,2,2-trichloréthyle, un groupe allyle, un groupe acétoxyméthyle, un groupe 4-nitrobenzyle, un groupe 2-nitrobenzyle, un groupe 4-méthoxybenzyle, un groupe benzyle ou un groupe triméthylsilyléthyle, ou représente un radical de formule

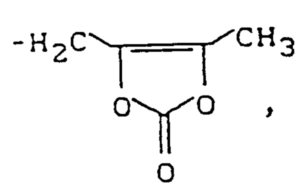 ,

$-CH(CH_3)-OCOOC_2H_5$
ou $-CH_2-OCO-C(CH_3)_3$
et

$R^5$ représente un atome d'hydrogène, un groupe méthyle, un groupe dihydroxyéthyle, un groupe chlorométhyle, un groupe iodométhyle, ou
représente un radical de formule

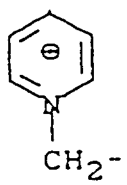 , 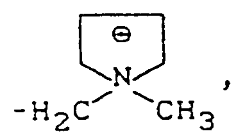 , 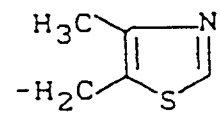 , 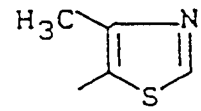

ou 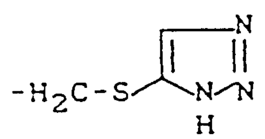

ainsi que leurs sels.

**4.** Procédé pour la préparation de composés $\beta$-lactames répondant à la formule générale (I)

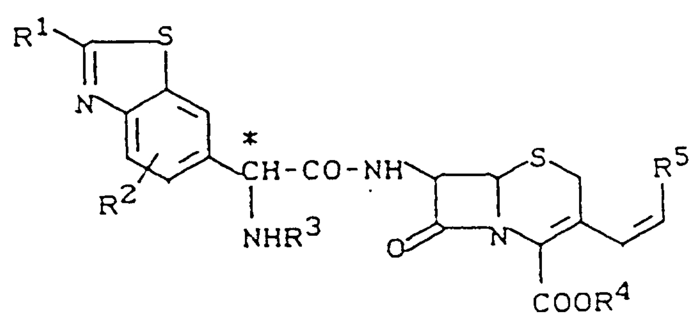
(I),

selon la revendication 1, caractérisé en ce que

[A] on fait réagir des composes substitués de céphalosporines répondant à la formule générale (II)

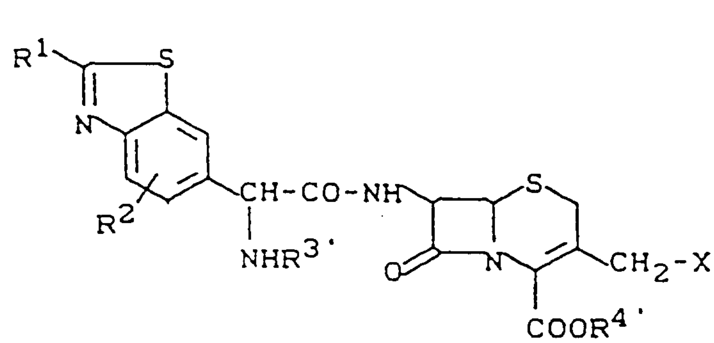
(II)

dans laquelle

$R^1$ et $R^2$ ont la signification indiquée ci-dessus,

$R^{3'}$ représente un groupe de protection du groupe amino habituel dans la chimie des $\beta$-lactames,

$R^{4'}$ représente un groupe protecteur du groupe carboxyle habituel dans la chimie des $\beta$-lactames,

et

X représente un groupe de formules

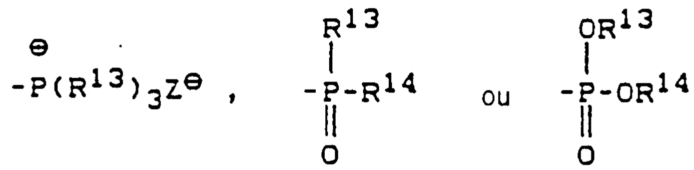

dans lesquelles

$R^{13}$ et $R^{14}$ sont identiques ou différents et représentent un groupe alkyle, un groupe phényle ou un groupe tolyle et

$Z^{\ominus}$ représente un anion halogénure, de préférence un anion chlorure, un anion bromure ou un anion iodure,

dans des solvants inertes en présence de bases,
avec des aldéhydes répondant à la formule générale (III)

$R^5$-CHO (III)

dans laquelle

$R^5$ a la signification indiquée ci-dessus,

ou bien en ce que
[B] on fait réagir des composés de phosphonium répondant à la formule générale (IV)

$R^5$-$CH_2$-X (IV)

dans laquelle

$R^5$ a la signification indiquée ci-dessus, et

X représente un groupe de formules

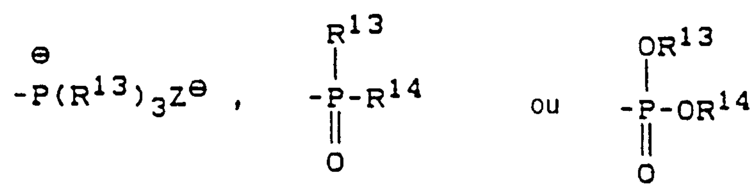

dans lesquelles

$R^{13}$ et $R^{14}$ sont identiques ou différents et représentent un groupe alkyle, un groupe phényle ou un groupe tolyle et

$Z^{\ominus}$ représente un anion halogénure, de préférence un anion chlorure, un anion bromure ou un anion iodure,

dans des solvants inertes en présence de bases, avec des aldéhydes de céphalosporines, répondant à la formule générale (V)

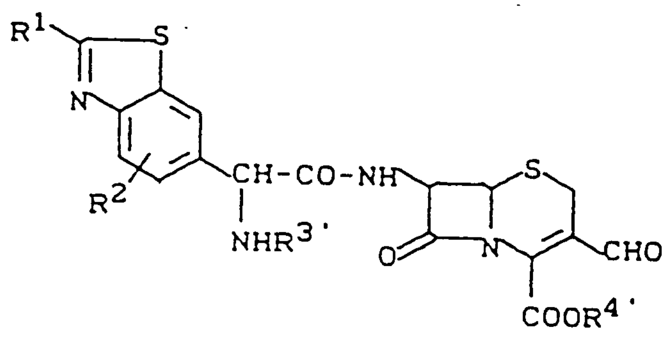

(V)

dans laquelle

$R^1$ et $R^2$ ont la signification indiquées ci-dessus,

$R^{3'}$ représente un groupe de protection du groupe amino habituel dans la chimie des $\beta$-lactames,

et

$R^{4'}$ représente un groupe protecteur du groupe carboxyle habituel dans la chimie des $\beta$-lactames,

ou bien en ce que

[C] on fait réagir des acides carboxyliques répondant à la formule générale (VI)

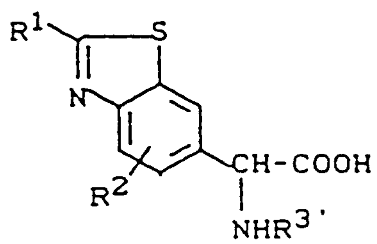

(VI)

dans laquelle

$R^1$ et $R^2$ ont la signification indiquée ci-dessus, et

$R^{3'}$ représente un groupe protecteur du groupe amino habituel dans la chimie des $\beta$-lactames, après activation du groupe carboxyle par transformation en un anhydride mixte, par exemple avec de l'ester éthylique de l'acide chloroformique ou l'ester butylique de l'acide chloroformique, ou encore le chlorure de l'acide méthanesulfonique, ou bien par transformation en halogénure d'acide ou encore par transformation en un ester activé par exemple avec du N-hydroxybenzotriazole et du dicyclohexylcarbodiimide (DCC),

avec les vinylcéphalosporinamines répondant à la formule générale (VII)

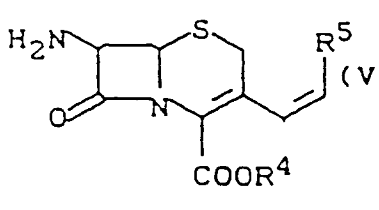

(VII),

dans laquelle

$R^4$ et $R^5$ ont la signification indiquée ci-dessus,

puis on élimine éventuellement les groupes de protection et on prépare les sels désirés ou, à partir des sels, les acides libres.

**5.** Composés $\beta$-lactames répondant à la formule générale (I)

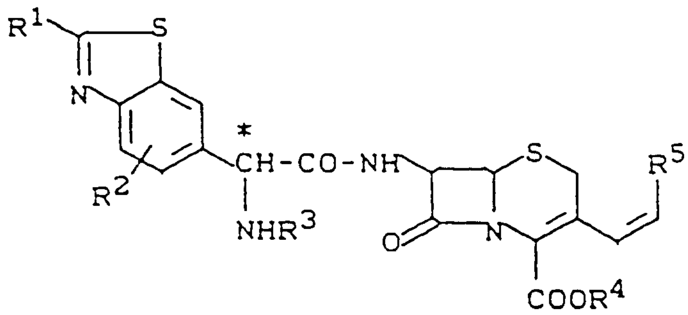

(I),

selon la revendication 1, pour les utiliser dans un procédé destiné au traitement thérapeutique du corps humain ou animal.

**6.** Médicament contenant des composés $\beta$-lactames répondant à la formule générale (I)

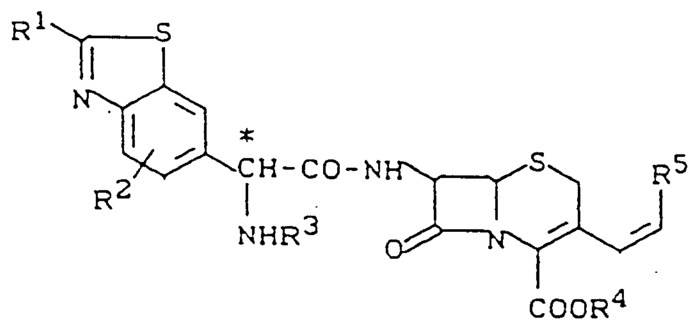

(I),

selon la revendication 1, ainsi que leurs sels.

**7.** Utilisation de composés $\beta$-lactames répondant à la formule générale (I) selon la revendication 1 pour la préparation de médicaments.

**8.** Aliments pour animaux et additifs d'aliments pour animaux contenant des composés $\beta$-lactames répondant à la formule générale (I) selon la revendication 1.

**Revendications pour les Etats contractants suivants : ES, GR**

**1.** Procédé pour la préparation de composés $\beta$-lactames répondant à la formule générale (I)

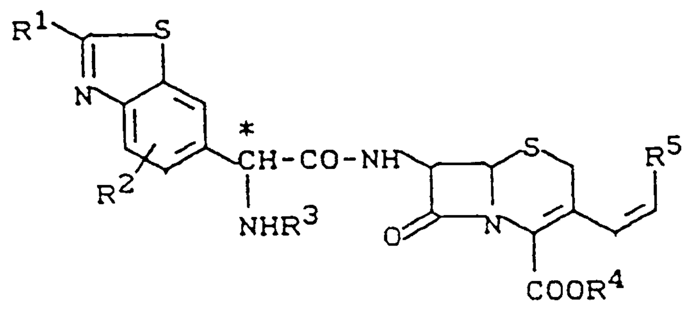

(I),

dans laquelle

$R^1$ représente un atome d'hydrogène, ou
représente un groupe alkyle à chaîne droite, à chaîne ramifiée, ou encore cyclique, contenant

jusqu'à 8 atomes de carbone, qui peut être substitué par un atome d'halogène, par un groupe hydroxyle, par un groupe alcoxy contenant jusqu'à 4 atomes de carbone, par un groupe carboxyle, par un groupe phényle, par un groupe sulfo ou encore par un groupe de formule

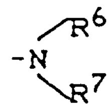

dans laquelle
$R^6$ , $R^7$ sont identiques ou différents et
représentent un atome d'hydrogène ou un groupe alkyle contenant jusqu'à 6 atomes de carbone,
ou
représentent un groupe phényle, un atome d'halogène, un groupe alcoxy, un groupe alkylthio, un groupe alkylsulfonyle contenant chacun jusqu'à 6 atomes de carbone, un groupe sulfo, un groupe sulfamoyle, un groupe mercapto, un groupe hydroxyle, un groupe phénylthio, un groupe phényloxy, un groupe guanidino ou un groupe amidino, ou représentent un groupe de formule

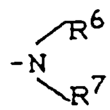

où
$R^6$, $R^7$ ont la signification indiquée ci-dessus,

$R^2$ représente un atome d'hydrogène, un groupe alkyle, un groupe alcoxy, un groupe alkylthio contenant chacun jusqu'à 6 atomes de carbone, un groupe trifluorométhyle, un groupe trifluorométhoxy, un groupe hydroxyle, un groupe mercapto, un groupe nitro, un groupe cyano ou un atome d'halogène,

$R^3$ représente un atome d'hydrogène ou
représente un groupe protecteur du groupe amino habituel dans la chimie des $\beta$-lactames,

$R^4$ représente un atome d'hydrogène,
représente un groupe protecteur du groupe carboxyle habituel dans la chimie des $\beta$-lactames, ou
représente un ester séparable in vivo,
et

$R^5$ représente un atome d'hydrogène,
un groupe alkyle à chaîne droite ou ramifiée contenant jusqu'à 6 atomes de carbone, qui peut être substitué par un atome d'halogène, par un groupe alcoxy, par un groupe alkylthio, contenant chacun jusqu'à 6 atomes de carbone, par un groupe hydroxyle, par un groupe amino, ou encore par un radical de formule

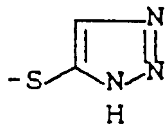 , 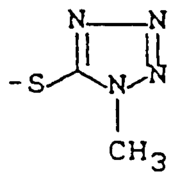 , 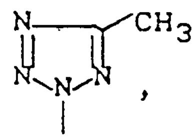 ,

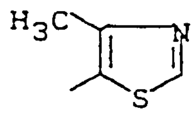 , 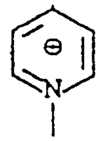 , 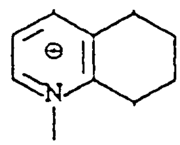

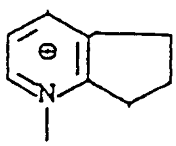 , 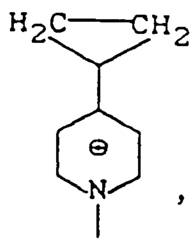 , 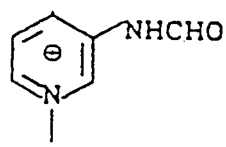

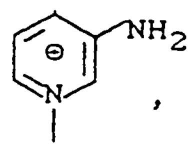 , 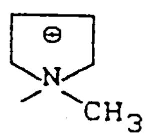 , 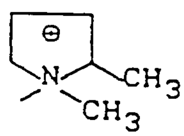 ,

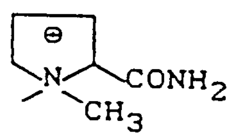 , 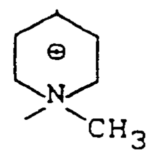 , 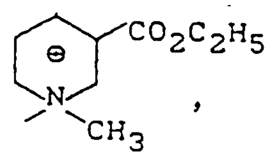 ,

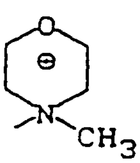 , 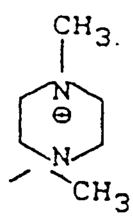 ou 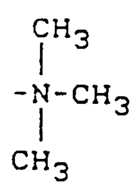

caractérisé en ce que

[A] on fait réagir des composés substitués de céphalosporines répondant à la formule générale (II)

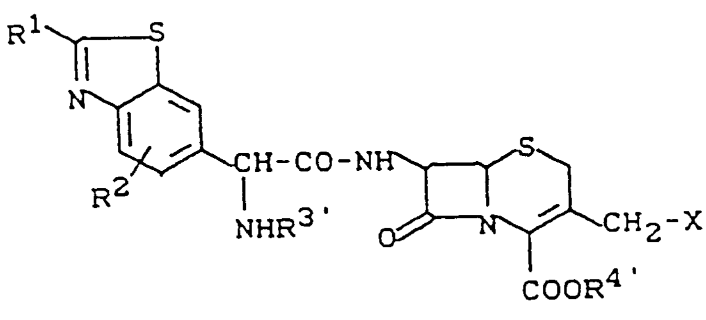 (II)

dans laquelle

$R^1$ et $R^2$ ont la signification indiquées ci-dessus,

$R^{3'}$ représente un groupe de protection du groupe amino habituel dans la chimie des $\beta$-lactames,

$R^{4'}$ représente un groupe protecteur du groupe carboxyle habituel dans la chimie des $\beta$-lactames,

et

X représente un groupe de formules

$\overset{\ominus}{-P}(R^{13})_3Z^{\ominus}$ , 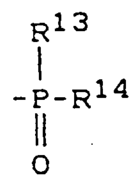 ou 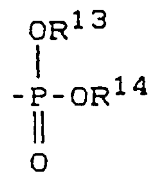

dans lesquelles

$R^{13}$ et $R^{14}$ sont identiques ou différents et représentent un groupe alkyle, un groupe phényle ou un groupe tolyle

et

$Z^{\ominus}$ représente un anion halogénure, de préférence un anion chlorure, un anion bromure ou un anion iodure,

dans des solvants inertes en présence de bases,

avec des aldéhydes répondant à la formule générale (III)

$R^5$-CHO (III)

dans laquelle

$R^5$ a la signification indiquée ci-dessus,

ou bien en ce que

[B] on fait réagir des composés de phosphonium répondant à la formule générale (IV)

$R^5$-$CH_2$-X (IV)

dans laquelle

$R^5$ a la signification indiquée ci-dessus,

et

X représente un groupe de formules

$\overset{\ominus}{-P}(R^{13})_3Z^{\ominus}$ , 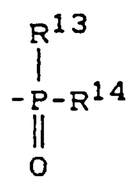 ou 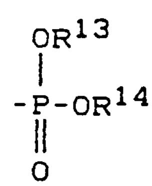

dans lesquelles

$R^{13}$ et $R^{14}$ sont identiques ou différents et représentent un groupe alkyle, un groupe phényle ou un groupe tolyle

et

$Z^{\ominus}$ représente un anion halogénure, de préférence un anion chlorure, un anion bromure ou un anion iodure,

dans des solvants inertes en présence de bases,

avec des aldéhydes de céphalosporines, répondant à la formule générale (V)

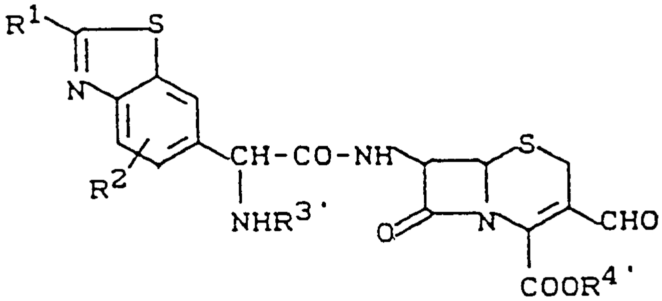

(V)

dans laquelle

$R^1$ et $R^2$ ont la signification indiquées ci-dessus,

$R^{3'}$ représente un groupe de protection du groupe amino habituel dans la chimie des $\beta$-lactames,

et

$R^{4'}$ représente un groupe protecteur du groupe carboxyle habituel dans la chimie des $\beta$-lactames,

ou bien en ce que

[C] on fait réagir des acides carboxyliques répondant à la formule générale (VI)

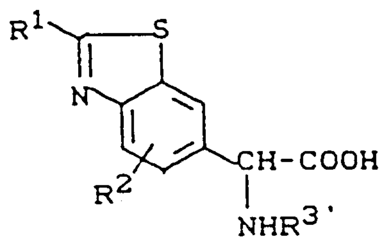

(VI)

dans laquelle

$R^1$ et $R^2$ ont la signification indiquée ci-dessus, et

$R^{3'}$ représente un groupe protecteur du groupe amino habituel dans la chimie des $\beta$-lactames, après activation du groupe carboxyle par transformation en un anhydride mixte, par exemple avec de l'ester éthylique de l'acide chloroformique ou l'ester butylique de l'acide chloroformique, ou encore le chlorure de l'acide méthanesulfonique, ou bien par transformation en halogénure d'acide ou encore par transformation en un ester activé par exemple avec du N-hydroxybenzotriazole et du dicyclohexylcarbodiimide (DCC),

avec les vinylcéphalosporinamines répondant à la formule générale (VII)

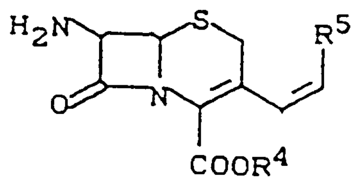

(VII),

dans laquelle

$R^4$ et $R^5$ ont la signification indiquée ci-dessus,

puis on élimine éventuellement les groupes de protection et on prépare les sels désirés ou, à partir des sels, les acides libres.

**2.** Utilisation de composés $\beta$-lactames répondant à la formule générale (I) selon la revendication 1 pour la préparation de médicaments.

3. Aliments pour animaux et additifs d'aliments pour animaux contenant des composés $\beta$-lactames répondant à la formule générale (I) selon la revendication 1.